# EUROPEAN PATENT APPLICATION

(11) **EP 2 151 433 A1**
(43) Date of publication of application: **10.02.2010**
(21) Application number: 08305449.4
(22) Date of filing: 05.08.2008
(51) Int. Cl.: C07D 231/20, A61K 31/415, A61P 3/10, A61P 29/00

(54) **Alkoxypyrazoles and the process for their preparation**

(71) Applicant: Institut Pasteur, 75015 Paris (FR); Centre National de la Recherche Scientifique (CNRS), 75794 Paris Cedex 16 (FR)
(72) Inventor: Janin, Yves, 75005, PARIS (FR); Guillou, Sandrine, 94700, MAISON ALFORT (FR)
(74) Representative: Warcoin, Jacques

(57) **Abstract**

The present invention relates to a alkoxypyrazoles preparation process, and new alkoxypyrazole compounds.

## Description

The present invention relates to synthesis of 3 or 5-alkoxy pyrazoles as well as pyrazol-3-ones.

Heterocyclic compounds particularly five membered heterocycles have attracted the attention of pharmaceutical community over the years due to their therapeutic value.

Remarkably few 3/5-alkoxypyrazoles have been reported for their biological properties but in recent years, there has been a growing interest in discovering new pyrazolones having advantageous biological properties.

Found among these compounds, in particular, are pyrazol-3-ones having applications in the field of the treatment or prevention of diseases such as diabetes, inflammatory diseases, prion diseases, and of the treatment of pain.

Nineteen pyrazolone derivatives have been shown to inhibit PrP-res (resistant prion protein) accumulation. The most effective compound was 2-phenyl-5-(4-nitrophenyl)-pyrazol-3-one^{1,2}.

1,2-dihydro-4-[[4-(methylthio)phenyl]methyl]-5-(trifluoromethyl)-3H-pyrazol-3-one has been shown to be a potent antihyperglycemic agent³.

Propyphenazone (1,2-dihydro-1,5-dimethyl-4-(1-methylethyl)-2-phenyl-3H-pyrazol-3-one) is a nonsteroidal anti-inflammatory drug frequently used as mild analgesic medicament⁴.

5-alkyl-4-(arylmethyl)pyrazol-3-ones have been shown to be the most promising new class of potential antidiabetic agent⁵.

The new immunosuppressant 2-(4'-chlorophenyl)benzothiopyrano-(4,3-c)pyrazol-3-one has been characterized⁶.

Moreover, few pyrazole-featuring compounds, including some 3-ethoxypyrazoles,⁷ have been reported for their antimicrobial activity.^{8, 9}

However, the known synthesis processes of pyrazol-3-ones, for example N1-aryl pyrazol-3-ones, are circuitous.

In fact, very few reports describe the preparation of N1-arylpyrazol-3-ones isomers. The first work describes a condensation between acylhydrazines and acetoacetates in the presence of phosphorus trichloride.⁵⁴⁻⁵⁷ A second type of preparation requires the oxidation⁵⁸⁻⁶⁰ of pyrazolidin-3-ones which would, for instance, result from the addition of arylhydrazines on acrylic esters. A third involves a reaction between arylhydrazines and diketene.^{61, 62} The fourth approach requires reactions between either 2,3-dichloropropionic acid amide⁶³ or β-ethoxyacryloylchloride^{64, 65} and phenylhydrazines as well as α-acetylenic esters with phenylhydrazine in the presence of alkoxides.⁶⁶ and the most recent method takes place via a rearrangement of the reaction product between either biacetyldimer⁶⁷ or the intermediate furanone^{68, 69} and aryldiazonium salts.

The present invention proposes a simple and general access to N1-aryl or N1-alkyl C4-aryl pyrazol-3-ones.

The well known Knorr pyrazol-3/5-ones synthesis consists in a double condensation reaction between, for example ethylacetoacetate **(1),** and various hydrazines. ^{70,71} The reaction proceeds via intermediates **2a-c** followed by an elimination reaction of the alcohol moiety to give pyrazol-5-ones **3a-c** bearing, if relevant, a substituent on nitrogen 2. However, very early (1895),⁷²⁻⁷⁴ the reaction was noted to partially proceed otherwise, leading to 5-ethoxypyrazoles **4a-c** *via* a water elimination instead of the ethanol (see scheme 1).

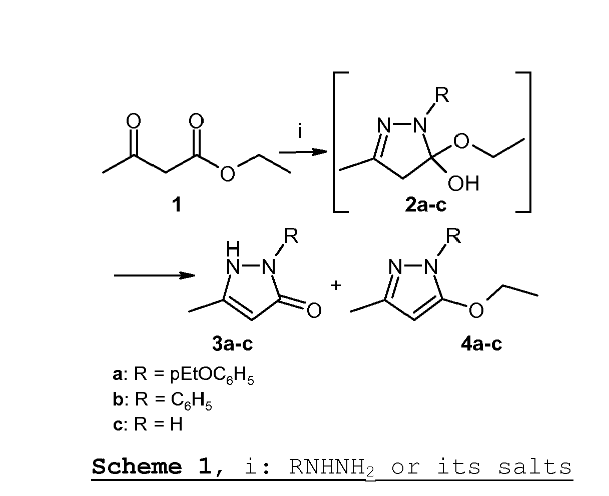

Further studies were undertaken in 1926 in the course of which intermediate **2c** was postulated and compound **4c** was isolated in a 30% yield along with **3c.** This result was obtained by reacting hydrazine with **1** in the presence of two equivalents of hydrochloride acid in boiling alcohol⁷⁵ and remarkably few reports followed these early studies.⁷⁶⁻⁸⁵ This is quite surprising as O-protected pyrazoles such as compound **4** could be starting material for many syntheses aiming at selective reactions either on nitrogen 1 or carbon 4.

Scheme 2 provides an illustration of the selectivity problem encountered with the alkylation of pyrazol-3-ones. The benzylation of 5-methylpyrazol-3-one **(3c)** under basic conditions led to the O-benzyl derivative **5** as well as, at least, the fully characterized compounds **6-8.**

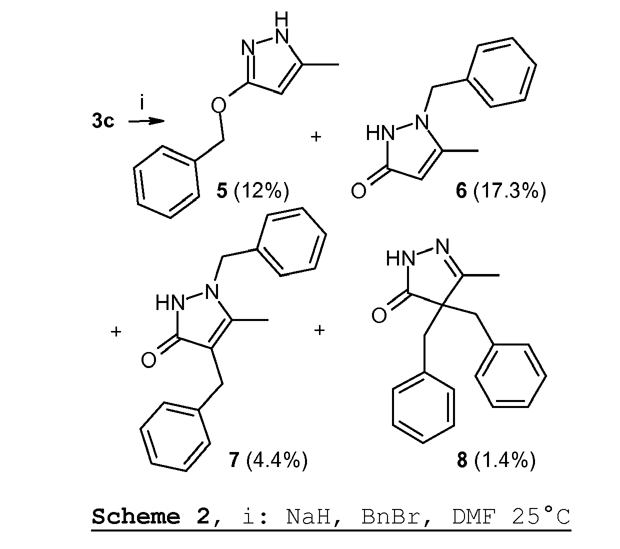

Thus, 3-alkoxypyrazoles and N1-substituted pyrazol-3-ones were previously tedious to prepare. A brute statistical survey of the all the pyrazol-3-one-bearing substances ever reported up to 2007 led to the following facts. As much as 156278 compounds featuring this ring system were reported. However, most of these compounds (113469) are N1-aryl-pyrazol-5-ones and only 9997 are the isomeric N1-aryl-pyrazol-3-ones. This last number actually includes compounds with a substituent on the other nitrogen (4189) as well as ring-fused 1-arylindazol-3-ones (138). This bias is very much due to the regiochemistry of any Knorr reactions between the vast array of available arylhydrazines and acetoacetates.

There exist important needs for a new process for a simple access to N1-aryl or N1-alkyl C4-aryl pyrazol-3-ones.

The present inventors have shown that the reaction of Scheme 1 in the presence of hydrazine monohydrochloride or methyl-, benzyl-, phenyl-, or 4-chlorophenylhydrazine monohydrochloride can lead to a better yield of 3-alkoxypyrazole-bearing compounds when compared to previously reported cases which used the sulfate or, more often,⁶ the dihydrochloride salts. This process has the advantage to provide a very simple access to many alkoxypyrazole-bearing compounds as, quite often, an extraction is the sole work up required.

From this, the use of further chemical transformations insured the design of accesses to fully original chemical entities as the presence of an oxygen protecting group substantially altered the pyrazole nitrogens reactivity. Depending on the substituents of the alkoxypyrazole ring system, a preference for the otherwise not so easy to attain N1-arylation or alkylation products was observed. Thus, from the many 3-alkoxylpyrazoles prepared in the two steps process according to the invention, the inventors prepared N1-aryl or N1-alkyl pyrazol-3-ones.

Moreover, the presence of an oxygen protecting group substantially altered the pyrazole carbon-4 reactivity, toward, if this center is bearing a halogen function, carbon-carbon bond forming reactions such as the Suzuki or the Negishi reaction. Thus, from the many 3-alkoxypyrazoles prepared in another three or four steps process according to the invention, the inventors prepared C-4 arylpyrazol-3-ones.

Moreover the combination of these steps led the inventors to prepare N1-aryl or N1-alkyl pyrazol-3-ones featuring aryl subtituents on carbon 4.

Therefore, the first object of the present invention relates to a process for the preparation of 5-alkoxypyrazoles of formula (I) or, when R₇ = H, another representation is the tautomeric 3-alkoxypyrazoles of formula (I') 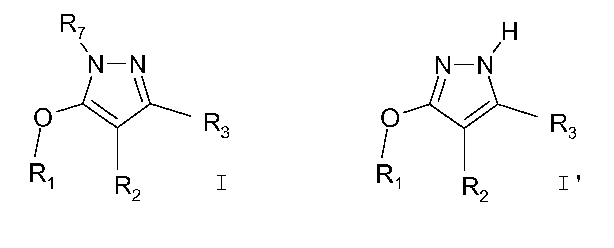 in which
R₁ stands for a radical selected from the group consisting of C₁-C₆ alkyl, C₂-C₆ alkenyl, C₂-C₆ alkynyl and (C₆-C₁₀)aryl(C₁-C₆)alkyl, each optionally substituted by R₄;
R₂ stands for a radical selected from the group consisting of hydrogen, halogen, C₁-C₆ alkyl, C₂-C₆ alkenyl, C₂-C₆ alkynyl, C₃-C₈ cycloalkyl, C₃-C₈ heterocycloalkyl, C₆-C₁₀ aryl, C₆-C₁₀ heteroaryl, C₆-C₁₀ aryloxy, (C₆-C₁₀) aryl (C₁-C₆) alkyl, (C₆-C₁₀)heteroaryl (C₁-C₆) alkyl, pyridyl, NR_{α}R_{β}, each optionally substituted by R₄;
R_{α} and R_{β}, identical or different, stand for a radical selected from the group consisting of H, C₁-C₆ alkyl or C₆-C₁₀ aryl;
R₃ stands for a radical selected from the group consisting of C₁-C₆ alkyl, C₂-C₆ alkenyl, C₂-C₆ alkynyl, C₁-C₆ polyhalogenoalkyl, C₃-C₈ cycloalkyl, C₃-C₈ heterocycloalkyl, C₆-C₁₀ aryl, C₆-C₁₀ heteroaryl, (C₆-C₁₀) aryl (C₁-C₆) alkyl, (C₆-C₁₀)heteroaryl(C₁-C₆)alkyl, -COOR₅, each optionally substituted by R₄;
or R₂ and R₃ form together an C₄-C₆ alkylene radical, an C₄-C₆ alkenylene, an C₄-C₆ heteroalkylene radical or an C₄-C₆ heteroalkenylene radical, each optionally substituted by R₆;
R₄ is a radical selected from the group consisting of halogen, hydroxyl, C₁-C₆ alkyl, C₁-C₆ alkylthio, C₁-C₆ alkoxy, and trialkylsilyl;
R₅ is a radical selected from the group consisting of hydrogen, C₁-C₆ alkyl, C₂-C₆ alkenyl and C₂-C₆ alkynyl;
R₆ is a radical selected from the group consisting of C₁-C₆ alkyl, C₂-C₆ alkenyl, C₂-C₆ alkynyl, C₆-C₁₀ aryl and (C₆-C₁₀ )aryl(C₁-C₆)alkyl;
R₇ stands for H, C₁-C₆ alkyl, C₆-C₁₀ aryl, (C₆-C₁₀)aryl(C₁-C₆)alkyl, C₆-C₁₀ heteroaryl or (C₆-C₁₀) heteroaryl (C₁-C₆)alkyl, each optionally substituted by a halogen;
comprising the successive following steps:
(a) reacting an alkylacetoacetate of formula (II) or one of its salts 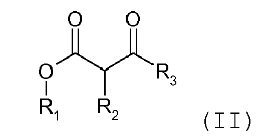 in which R₁, R₂ and R₃ are as defined for compound of formula (I)
   with 1 to 1.5 molar equivalents of R₇-NH-NH₂, xHX, in which R₇ is as defined for compound of formula (I), x is 1 or 2 and X is Cl or Br;
   in an alkylalcohol R₈OH as a solvent, in which R₈ is a radical selected from the group consisting of C₁-C₆ alkyl, C₂-C₆ alkenyl, C₂-C₆ alkynyl and (C₆-C₁₀)aryl(C₁-C₆)alkyl, (C₆-C₁₀)heteroaryl(C₁-C₆)alkyl;
(b) recovering the 3-alkoxypyrazole of formula (I). Advantageously, the molar ratio of said alkylacetoacetate of formula (II) to said R₇-NH-NH₂, xHX, x is 1 or 2, X is Cl or Br, is close to 1, preferably comprised between 0.95 and 1.1, more preferably between 1 and 1.05.

In a preferred embodiment, said alkylacetoacetate of formula (II) is reacted with 1.05 molar equivalents of R₇-NH-NH₂, xHX, in which R₇, x and X are as defined above.

In a preferred embodiment, step (a) is run with dihydrochloride hydrazine when a free basic group or a salt of the alkylacetoacetate of formula (II), preferably a sodium salt, has to be neutralized. For example of this embodiment, in step (a), a salt of the alkylacetoacetate of formula (II), preferably a sodium salt, is reacted with R₇-NH-NH₂, 2HCl.

Advantageously, R₇ is hydrogen or a methyl group.

When R₇ is hydrogen, the two tautomeric forms of formula (I) and (I') are obtained.

Advantageously, X is Cl.

Preferably, said R₇-NH-NH₂, xHCl is R₇-NH-NH₂, HCl (x is 1), more preferably said R₇-NH-NH₂, xHCl is NH₂-NH₂, HCl.

Preferably R₈ is a C₁-C₆ alkyl, more preferably R₈ is ethyl or isopropyl. The choice of R₈ is driven by the nature of R₁, in the case of high boiling R₁OH, the reaction is preferably run in R₈OH in which R₈ is a C₁-C₆ alkyl.

Preferably, R₈ is R₁.

In one embodiment, the reaction in step (a) is run at the boiling point of the solvent, preferably for between eight and 24 hours, more preferably for eight hours.

In another embodiment, the reaction in step (a) is run at room temperature, preferably for between eight and 24 hours.

In a further embodiment, when R₁ is different from R₈, the reaction in step (a) is run at room temperature, preferably for between eight and 24 hours. For example, the reaction in step (a) with allylacetoacetate is run overnight at room temperature because a transesterification was observed in boiling isopropanol.

In a preferred embodiment, 1.05 equivalents of hydrazine monohydrochloride are used in boiling ethanol or boiling isopropanol.

In a preferred embodiment, said alkylacetoacetate of formula (II) is isopropylacetoacetate.

In a preferred embodiment, said alkylacetoacetate of formula (II) is ethylacetoacetate.

In a preferred embodiment, said alkylacetoacetate of formula (II) is ethyl 2-benzyl-3-oxobutanoate.

In a preferred embodiment, said alkylacetoacetate of formula (II) is ethyl 2- (methyl (phenyl) amino)-3-oxobutanoate.

In a preferred embodiment, said alkylacetoacetate of formula (II) is ethyl 3-oxo-2-phenoxybutanoate.

In a preferred embodiment, R₁ stands for a radical selected from the group consisting of C₁-C₆ alkyl optionally substituted by a C₁-C₆ alkoxy or trialkylsilyl group, (C₆-C₁₀)aryl(C₁-C₆)alkyl and C₂-C₆ alkenyl, preferably stands for methyl, ethyl, benzyl, propene, more preferably 1-propene or 2-propyl.

In a preferred embodiment, R₂ stands for a radical selected from the group consisting of hydrogen, F, Cl, C₁-C₆ alkyl, C₆-C₁₀ aryloxy, and (C₆-C₁₀)aryl(C₁-C₆) alkyl, NR_{α}R_{β} wherein R_{α}=Me and R_{β}=Phenyl, preferably stands for hydrogen, F, Cl, methyl, ethyl, benzyl, phenoxy optionally substituted by one or more methyl, pyridyl, more preferably 2-pyridyl.

In a preferred embodiment, R₃ stands for a radical selected from the group consisting of C₁-C₆ alkyl, -CF₃, C₁-C₆ oxaalkyl, C₆-C₁₀ aryl optionally substituted by a halogen atom, -COOR₅, in which R₅ is a hydrogen or C₁-C₆ alkyl, preferably stands for methyl, -CF₃, benzyl, C₆-C₁₀ aryl, -COOR₅, in which R₅ is a hydrogen or ethyl.

In a preferred embodiment, R₂ and R₃ form together a C₄-C₆ alkylene radical or a C₄-C₆ heteroalkenylene radical, each eventually substituted by a benzyl group, preferably form a butylene group wherein a carbon atom is optionally substituted by a nitrogen atom, said nitrogen atom being optionally substituted by a benzyl group, more preferably form a butylene, a -CH₂-CH₂-NBn-CH₂- group, a -CH₂-NBn-CH₂-CH₂- group or a -CH₂-NH-CH₂-CH₂- group.

Preferably, R₇ is a hydrogen atom or a methyl group.

Another object of the present invention relates to alkoxypyrazoles of formula I or I' obtainable by the process according to the invention, except compounds of formula (I') wherein simultaneously
R₂ is H, R₃ is methyl, R₁ is ethyl, methyl or benzyl;
R₁ is ethyl, R₂ is H, R₃ is phenyl, COOH or CF₃;
R₁ is ethyl, R₂ is ethyl, R₃ is methyl;
R₁ is ethyl, R₂ is phenyl, R₃ is phenyl;
R₁ is benzyl, R₂ is H, R₃ is methyl.

Another object of the present invention relates to alkoxypyrazoles of formula (III) or (IV) 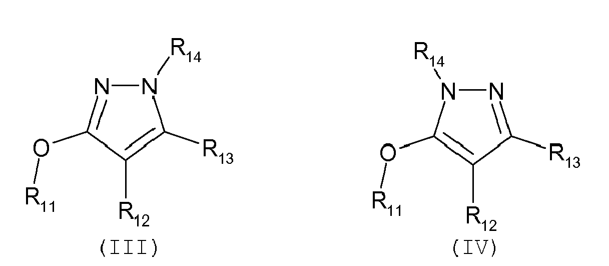 in which
R₁₁ stands for a radical selected from the group consisting of methyl, ethyl, 2-propyl, propene, benzyl, methoxyethyl, 2-(1-trimethylsilyl)ethyl,
R₁₂ stands for a radical selected from the group consisting of a hydrogen, halogen, a methyl, ethyl, benzyl, phenyl optionally substituted by Cl or methoxy, pyridyl, pyridyl N-oxide, phenoxy optionally substituted by one or more methyl, N,N-methylphenylamino, 2-phenethyl and is preferably an halogen and more preferably an iodine or a bromine atom,
R₁₃ stands for a radical selected from the group consisting of methyl, CF₃, phenyl, 4-chlorobenzyl, -COOR₅, in which R₅ is a radical selected from the group consisting of C₁-C₆ alkyl, C₂-C₆ alkenyl, and C₂-C₆ alkynyl and is preferably ethyl,
or R₁₂ and R₁₃ form together an C₄-C₆ alkylene radical, an C₄-C₆ alkenylene, an C₄-C₆ heteroalkylene radical or an C₄-C₆ heteroalkenylene radical, each optionally substituted by R₆ as defined above and is preferably selected among butylene, a -CH₂-NBn-CH₂-CH₂- group, a -CH₂-CH₂-NBn-CH₂- group,
R₁₄ stands for a radical selected from the group consisting of hydrogen, methyl, benzyl, mesyl, -CH₃SO₂, tosyl, phenyl optionally substituted by Cl or a methoxy group, pyridyl, except compounds of formula (III) wherein R₁₄ is H and simultaneously
R₁₂ is H, R₁₃ is methyl, R₁₁ is ethyl, methyl or benzyl;
R₁₁ is ethyl, R₁₂ is H, R₁₃ is phenyl, COOH or CF₃;
R₁₁ is ethyl, R₁₂ is ethyl, R₁₃ is methyl;
R₁₁ is ethyl, R₁₂ is phenyl, R₁₃ is phenyl;
R₁₁ is benzyl, R₁₂ is H, R₁₃ is methyl.

Preferably, the alkoxypyrazoles according to the invention are of formula (III) wherein R₁₄ is H or of formula (IV) wherein R₁₄ stands for a radical selected from the group consisting of hydrogen, methyl, benzyl, phenyl optionally substituted by Cl, and
R₁₁ stands for a radical selected from the group consisting of methyl, ethyl, 2-propyl, propene, benzyl, 2-(1-trimethylsilyl)ethyl,
R₁₂ stands for a radical selected from the group consisting of a hydrogen, F, Cl, a methyl, ethyl, benzyl,
R₁₃ stands for a radical selected from the group consisting of methyl, CF₃, phenyl, -COOR₅. in which R₅ is ethyl,
or R₁₂ and R₁₃ form together an C₄-C₆ alkylene radical, an C₄-C₆ heteroalkylene radical each optionally substituted by R₆ as defined in claim 1 and is preferably selected among butylene, a -CH₂-NBn-CH₂-CH₂- group, a -CH₂-CH₂-NBn-CH₂- group,
except compounds of formula (III) wherein R₁₄ is H and simultaneously
R₁₂ is H, R₁₃ is methyl, R₁₁ is ethyl, methyl or benzyl;
R₁₁ is ethyl, R₁₂ is H, R₁₃ is phenyl, COOH or CF₃;
R₁₁ is ethyl, R₁₂ is ethyl, R₁₃ is methyl;
R₁₁ is ethyl, R₁₂ is phenyl, R₁₃ is phenyl;
R₁₁ is benzyl, R₁₂ is H, R₁₃ is methyl.

Preferably, the alkoxypyrazoles according to the invention are of formula (III) in which,
R₁₁ is ethyl, R₁₂ is benzyl, R₁₃ is methyl, R₁₄ is phenyl or 4-ClC₆H₄;
R₁₁ is 2-propyl, R₁₂ is H, R₁₃ is methyl, R₁₄ is benzyl, phenyl, 4-ClC₆H₄, 2-or 3- MeOC₆H₄;
R₁₁ is 2-propyl, R₁₂ is I, R₁₃ is methyl, R₁₄ is phenyl, 4-ClC₆H₄, 2- or -3- MeOC₆ H₄;
R₁₁ is 2-propyl, R₁₂ is Br, R₁₃ is methyl, R₁₄ is H, phenyl, 4-ClC₆H₄, 2- or 3-MeOC₆ H₄;
R₁₁ is 2-propyl, R₁₃ is methyl, R₁₄ is methyl, and R₁₂ is phenyl, 4-ClC₆ H₄, or 2- or 3-MeOC₆ H₄
R₁₁ is 2-propyl, R₁₃ is methyl, R₁₄ is phenyl, R₁₂ is phenyl, 4-ClC₆ H₄, 2- or 3-MeOC₆H₄, 2-pyridyl or 2-pyridyl N-Oxide,
R₁₁ is 2-propyl, R₁₃ is methyl, R₁₄ is H, R₁₂ is H, I, C₆H₅, 4 ClC₆H₄, 2- or 3- MeOC₆H₄, 3- or 4-pyridyl, or CH₂-C₆H₅,
R₁₁ is 2-propyl, R₁₃ is methyl, R₁₄ is methyl, and R₁₂ is H, I, or 2-pyridyl N-Oxide,
R₁₁ is 2-propyl, R₁₂ is H, R₁₃ is methyl, R₁₄ is 2- 3-or-4-pyridyl,;
R₁₁ is 2-propyl, R₁₃ is methyl, R₁₄ is phenyl, R₁₂ is CH₂C₆H₅ or 3-or 4-pyridyl,
R₁₁ is ethyl, R₁₃ is methyl, R₁₄ is H, R₁₂ is F, Cl, benzyl, C₆H₅, 3,5(CH₃)C₆H₅ O,
R₁₁ is ethyl, R₁₂ is H, R₁₃ is CF₃, R₁₄ is phenyl, 2-, 3- or 4-pyridyl,
R₁₁ is ethyl, R₁₂ is methyl or H, R₁₃ is -COOEt, R₁₄ is H;
R₁₁ is 1-propene, R₁₂ is H, R₁₃ is methyl, R₁₄ is H, 4-ClC₆ H₄, R₁₁ is ethyl, R₁₂ is H, R₁₃ is phenyl, R₁₄ is 4-ClC₆ H₄;
R₁₁ is ethyl, R₁₂ is H, R₁₃ is CH₂-4-ClC₆ H₄, R₁₄ is H;
R₁₁ is methoxyethyl, R₁₂ is H, R₁₃ is CH₃, R₁₄ is H;
R₁₁ is ethyl, R₁₂ is benzyl, R₁₃ is methyl, R₁₄ is 4-MeOC₆H₄;
R₁₁ is 2- (1-trimethylsilyl)ethyl, R₁₂ is H, R₁₃ is methyl, R₁₄ is H;
R₁₁ is ethyl, R₁₂ is N, N-methylphenylamino, R₁₃ is methyl, R₁₄ is H;
R₁₁ is ethyl, R₁₂ is I, R₁₃ is -CF₃ or phenyl, R₁₄ is H;
R₁₁ is 2-propyl, R₁₂ is I, R₁₃ is methyl, R₁₄ is -CH₃SO₂;
R₁₁ is ethyl, R₁₂ is I, R₁₃ is -CF₃, R₁₄ is -CH₃SO₂;
R₁₁ is ethyl, R₁₂ is phenyl or benzyl, R₁₃ is -CF₃, R₁₄ is H; R₁₁ is 2-propyl, R₁₂ is 2-phenethyl, R₁₃ is methyl, R₁₄ is H;
R₁₁ is ethyl, R₁₂ and R₁₃ form together a -CH₂-CH₂-NBn-CH₂-group, R₁₄ is H, 2-, 3- or 4-pyridyl or phenyl,
R₁₁ is ethyl, R₁₂ and R₁₃ form together a -CH₂-NBn-CH₂-CH₂-group, R₁₄ is 2-, 3 or 4-pyridyl, 4-ClC₆H₅ or H,
R₁₁ is ethyl; R₁₂ and R₁₃ form together a -CH₂-NH-CH₂-CH₂-group, R₁₄ is H, phenyl, 2-, 3- or 4- pyridyl.

Preferably, the alkoxypyrazoles according to the invention are of formula (IV) in which
R₁₁ is 2-propyl, R₁₂ is H, R₁₃ is methyl, R₁₄ is methyl, benzyl, phenyl, 4-ClC₆H₄, 2- or 3-MeOC₆H₄, 2-, 3- or 4- pyridyl, R₁₁ is 2-propyl, R₁₂ is I, R₁₃ is methyl, R₁₄ is methyl, phenyl, 4-ClC₆H₄, 3-MeOC₆H₄,
R₁₁ is 2-propyl, R₁₂ is Br, R₁₃ is methyl, R₁₄ is phenyl or 4-ClC₆H₄,
R₁₁ is 2-propyl, R₁₂ is phenyl, R₁₃ is methyl, R₁₄ is phenyl, R₁₁ is ethyl, R₁₂ is H, R₁₃ is phenyl, R₁₄ is 4-ClC₆H₄,
R₁₁ is ethyl, R₁₂ and R₁₃ form together a -CH₂-NBn-CH₂-CH₂-group, R₁₄ is 4-ClC₆H₅,
R₁₁ is 1-propene, R₁₂ is H, R₁₃ is methyl, R₁₄ is 4-ClC₆H₄;
R₁₁ is ethyl, R₁₂ is benzyl, R₁₃ is methyl, R₁₄ is phenyl or 4-ClC₆H₄;
R₁₁ is 2-propyl, R₁₂ is I, R₁₃ is methyl, R₁₄ is -CH₃SO₂;
R₁₁ is ethyl, R₁₂ is I, R₁₃ is -CF₃, R₁₄ is -CH₃SO₂.

### Definitions

The abbreviations encountered are defined as follows:
Me is methyl,
H is hydrogen,
I is iodide, Br is bromide, Cl is chloride, F is fluorine MeO is methoxy, Bn is benzyl.

Within the meaning of this invention, "halogen" is understood to mean an atom of fluorine, bromine, chlorine or iodine.

Within the meaning of this invention, "C₁-C₆ alkyl" group is understood to mean a saturated, linear or branched hydrocarbon chain comprising from 1 to 6 carbon atoms, in particular the methyl, ethyl, n-propyl, isopropyl, n-butyl, iso-butyl, sec-butyl, tert-butyl, n-pentyl, n-hexyl groups.

Within the meaning of this invention, "C₂-C₆ alkenyl" group is understood to mean a linear or branched hydrocarbon chain comprising at least one double bond and comprising from 2 to 6 carbon atoms, e.g., such as an ethenyl (vinyl) or propenyl group.

Within the meaning of the invention, "C₂-C₆ alkynyl" group is understood to mean a linear or branched hydrocarbon chain comprising at least one triple bond and comprising from 2 to 6 carbon atoms, e.g., such as an ethynyl or propynyl group.

Within the meaning of this invention, "C₃-C₇ cycloalkyl" group is understood to mean a saturated hydrocarbon ring comprising from 3 to 7.

Within the meaning of this invention, "heterocycloalkyl" group is understood to mean a saturated hydrocarbon ring having 5 to 7 members and containing one or more, advantageously one or two, heteroatoms, e.g., such as sulphur, nitrogen or oxygen atoms, e.g., such as the tetrahydrofuranyl, piperidinyl, pyrrolidinyl, tetrahydropyranyl, 1,3-dioxolanyl group.

Within the meaning of this invention, "aryl" group is understood to mean an aromatic group preferably comprising from 5 to 10 carbon atoms and including one or more fused rings, e.g., such as a phenyl or naphtyl group. This is advantageously phenyl.

Within the meaning of the invention, "heteroaryl" group is understood to mean any aryl group as defined above wherein one or more carbon atoms have been replaced by one or more heteroatoms, advantageously 1 to 4, and even more advantageously 1 to 2, e.g., such as sulphur, nitrogen or oxygen atoms. Examples of heteroaryl groups are the furyl, thiophenyl, pyrrolyl, pyridyl, pyrimidyl, pyrazolyl, imidazolyl, tetrazolyl or else indyl groups.

Within the meaning of this invention, "aryl-(C₁-C₆)-alkyl" group is understood to mean any aryl group as defined above, which is bound to the molecule by means of a (C₁-C₆)-alkyl group as defined above. In particular, a group such as this can be a benzyl group.

Within the meaning of this invention, "C₄-C₆ alkylene" group is understood to mean a saturated, linear or branched hydrocarbon chain comprising from 4 to 6 carbon atoms which forms a saturated hydrocarbon ring having 6 to 8 members, preferably 6 members, with the two carbon atoms of the pyrazole cycle to which it is linked, in particular a butylene group.

Within the meaning of this invention, "C₄-C₆ alkenylene" group is understood to mean a linear or branched hydrocarbon chain comprising at least one double bond and comprising from 4 to 6 carbon atoms which forms a saturated hydrocarbon ring having 6 to 8 members, preferably 6 members, with the two carbon atoms of the pyrazole cycle to which it is linked,, e.g., a butenylene group.

Within the meaning of this invention, "C₄-C₆ heteroalkylene" group is understood to mean any alkylene group as defined above wherein one or more carbon atoms have been replaced by one or more heteroatoms, advantageously 1 to 2, and even more advantageously 1, e.g., such as sulphur, nitrogen or oxygen atoms, advantageously nitrogen.

Within the meaning of this invention, "C₄-C₆ heteroalkenylene" group is understood to mean any alkenylene group as defined above wherein one or more carbon atoms have been replaced by one or more heteroatoms, advantageously 1 to 2, and even more advantageously 1, e.g., such as sulphur, nitrogen or oxygen atoms, advantageously nitrogen.

The invention will be better understood upon reading the following examples, these examples serving solely to illustrate the invention.

### Examples

### Example 1 Preparation of 3-alkoxypyrazoles

The features of the devices used to conduct analyses of all of the compounds described in Example 1 are indicated hereinbelow:

Melting points were measured on a Kofler bench and are uncorrected. ¹H NMR and ¹³C NMR spectra were recorded on a Bruker spectrometer at 400 MHz and 100 MHz, respectively. Shifts (δ) are given in ppm with respect to the TMS signal and coupling constants (J) are given in Hertz. Column chromatography were performed over Merck silica gel 60 (0.035 - 0.070 mm), using a solvent pump operating at pressure between 2 and 7 bar (25-50 mL/mn) and an automated collecting system driven by a UV detector set to 254 nm unless stated otherwise. Mass spectra were obtained on an Agilent 1100 serie LC/MSD system using the atmospheric electrospray ionisation system. (HRMS) were obtained using a Waters Micromass Q-Tof with an electrospray ion source.

### General procedure for the preparation of alkoxypyrazoles.

The relevant acetoacetate-bearing compound (0.01 mmol) and hydrazine hydrochloride (0.0105 mmol) were refluxed in the relevant solvent (60 mL; i.e. ethanol for ethyl esters of acetoacetates, isopropanol for isopropyl esters of acetoacetate, tertbutanol for tertbutyl esters of acetoacetate) for usually 8 hours or more as specified below. In the case of allyl or 2-methoxyethyl acetoacetates, the reaction was run at room temperature overnight in isopropanol. In the case of benzyl acetoacetates, the reaction was run in isopropanol. The solvent were removed under reduced pressure, the residue was dispersed in dichloromethane and an excess of a 1N solution of sodium hydrogencarbonate. The organic phase was washed with 1N solution of sodium hydrogencarbonate three times, dried over sodium sulfate and concentrated to dryness. The resulting residue was either pure enough for their analytical characterization (> 95% pure as seen by ¹H NMR and LC/MS) or further purified as described below. 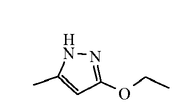 **3-Ethoxy-5-methyl-1H-pyrazole (4c):**¹¹ Obtained as an oil that solidified. M.p. = 62 °C, lit.¹¹ = 67-68 °C. ¹H (CDCl₃): 1.39 (t, 3H, J = 7.2); 2.25 (s, 3H); 4.17 (q, 2H, J = 7.2); 5.48 (s, 1H) ¹³C (CDCl₃): 12.0; 15.2; 65.0; 89.9; 141.2; 164.2. 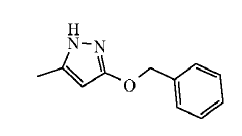 **3-Benzyloxy-5-methyl-1H-pyrazole (5):** This compound was further purified first using a high vacuum pump to remove most of the benzyl alcohol present and then a chromatography over silica gel eluting with a mixture of dichloromethane-ethanol 98/2. The fraction obtained crystallizes. M.p. = 91 °C. 1H (CDCl3): 2.24 (s, 3H); 5.21 (s, 2H); 5.55 (s, 1H); 7.38 (m, 5H). 13C (CDCl3): 12.0; 71.0; 90.4; 128.1; 128.3; 128.9; 137.6; 141.3; 164.2. The HRMS could not be obtained as the compound readily looses it benzyl moiety; m/z (electronic impact): 91 (100%) and 188 (4%). Note: the ¹H NMR data previously reported¹² for this compound fit much better with the data we obtained for the isomeric 1-benzyl-5-methyl-1,2-dihydro-3H-pyrazol-3-one we prepared by alkylation. 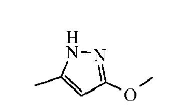 **3-Methoxy-5-methyl-1H-pyrazole (10b):**^{13,14} Obtained as an oil that solidified. M.p. = 46 °C, lit.¹³ = 49-50 °C. ¹H (CDCl₃): 2.25 (s, 3H); 3.88 (s, 3H); 5.50 (s, 1H) ¹³C (CDCl₃): 12.0; 56.5; 89.9; 141.3; 165.0. 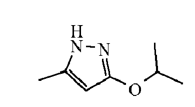 **3-Isopropoxy-5-methyl-1H-pyrazole (10c):** Obtained as an oil. ¹H (CDCl₃): 1.34 (d, 6H, J = 6.5); 2.25 (s, 3H); 4.65 (sept, 1H, J = 6.5); 5.45 (s, 1H) ¹³C (CDCl₃): 11.9; 22.6; 72.1; 90.3; 141.2; 163.2. The HRMS could not be obtained as the compound readily looses it isopropyl moiety; m/z (electronic impact): 98 (100%) and 140 (5%). 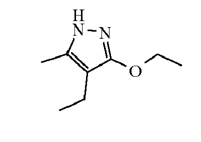 **3-Ethoxy-4-ethyl-5-methyl-1H-pyrazole (10e):** Obtained as a white solid. M.p. = 85 °C, lit.¹⁰ = 86°C. ¹H (CDCl₃): 1.11 (t, 3H, J = 7.5); 1.40 (t, 3H, J = 7.0); 2.18 (s, 3H); 2.33 (q, 2H, J = 7.5); 4.24 (q, 2H, J = 7.0); 8.5 (s (br.), 1H) ¹³C (CDCl₃): 10.5; 15.0; 15.4; 15.43; 64.4; 104.8; 137.4; 162.7. HRMS: Calcd. for C₈H₁₄N₂O + H : 155.1184. Found: m/z, 155.1188. 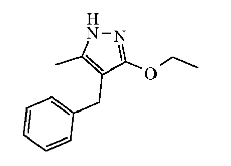 **4-Benzyl-3-ethoxy-5-methyl-1H-pyrazole (10f):** Obtained as a white solid. M.p. = 107 °C. ¹H (CDCl₃): 1.38 (t, 3H, J = 6.7); 2.11 (s, 3H); 3.69 (s, 2H); 4.25 (q, 2H, J = 6.7); 7.20 (m, 5H) ¹³C (CDCl₃): 10.7; 15.4; 28.0; 64.5; 102.4; 127.1; 128.6; 138.3; 141.6; 162.7. HRMS: Calcd. for C₁₃H₁₆N₂O + H : 217.1341. Found: m/z, 217.1387. 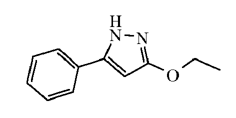 **3-Ethoxy-5-phenyl-1H-pyrazole (10g):**¹¹ This compound was further purified using a chromatography over silica gel eluting with a mixture of dichloromethane/ethanol 98-2 and was obtained as a white solid. On a bigger scale, this chromatography was avoided as the residue could be recristallized in cyclohexane (500 mL for 11 g of crude compound; long boiling time required). M.p. = 130 °C, lit.¹¹ = 126-128 °C. ¹H (CDCl₃): 1.40 (t, 3H, J = 7.0); 4.22 (q, 2H, J = 7.0); 5.96 (s, 1H); 7.40 (m, 3H); 7.57 (m, 2H); 9.9 (s (br), 1H) ¹³C (CDCl₃): 15.2; 65.4; 88.1; 125.7; 128.0; 129.3; 130.3; 145.3; 164.3. HRMS: Calcd. for C₁₁H₁₂N₂O + H : 189.1028. Found: m/z, 189.1036. 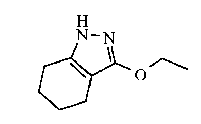 **3-Ethoxy-4,5,6,7-tetrahydro-1H-indazole (10h):** M.p. = 113°C. ¹H (CDCl₃): 1.40 (t, 3H, J = 7.0); 1.76 (m, 4H); 2.38 (m, 2H); 2.55 (m, 2H); 4.26 (q, 2H, J = 7.0). ¹³C (CDCl₃): 15.1; 19.2; 22.1; 22.9; 23.3; 64.4; 101.3; 141.2; 161.4. HRMS: Calcd. for C₉H₁₄N₂O + H : 167.1184. Found: m/z, 167.1184. 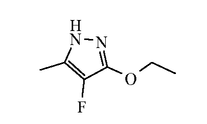 **3-Ethoxy-4-fluoro-5-methyl-1H-pyrazole (10i)**: Obtained as yellow crystals after recrystallisation in cyclohexane. M.p. = 91 °C. ¹H (CDCl₃): 1.42 (t, 3H, J = 7.0); 2.22 (s, 3H); 4.25 (q, 2H, J = 7.0) ¹³C (CDCl₃): 8.9; 15.1; 65.4; 125.9 (d, J = 23.5); 134.1 (d, J = 237.8); 150.8 (d, J = 9.1). HRMS: Calcd. for C₆H₉N₂FO + H : 145.0777. Found: m/z, 145.0783. 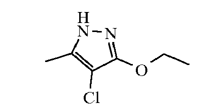 **3-Ethoxy-4-chloro-5-methyl-1H-pyrazole (10i'):** Obtained as pale yellow crystals after recrystallisation in cyclohexane. ¹H (CDCl₃): 1.43 (t, 3H, J = 7.1); 2.24 (s, 3H); 4.28 (q, 2H, J = 7.1) ¹³C (CDCl₃): 9.8; 14.8; 65.1; 94.3; 137.4; 158.7. m/z (LC/MS) = 161/163. 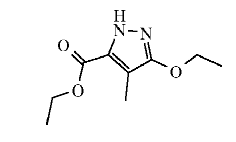 **Ethyl 3-ethoxy-4-methyl-1H-pyrazole-5-carboxylate (10j):** Obtained as white crystals after a chromatography over silica gel using dichloromethane. M.p. = 95 °C. ¹H (CDCl₃): 1.38 (m, 6H); 2.14 (s, 3H) ; 4.30 (q, 2H, J = 7.0); 4.37 (q, 2H, J = 7.0); 10.7 (s (br), 1H). ¹³C (CDCl₃): 7.0; 14.2; 14.8; 61.0; 64.8; 105.5; 130.9; 160.3; 162.3. HRMS: Calcd. for C₉H₁₄N₂O₃ + H : 199.1083. Found: m/z, 199.1116. 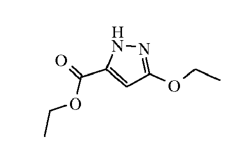 Ethyl **3-ethoxy-1H-pyrazole-5-carboxylate (10k):** This compound was prepared from the sodium salt of diethyloxalacetate **(9k)** as follow: diethyloxalacetate sodium salt (27.8 g, 0.132 mol; 95 % pure) was dissolved in ethanol (300 mL) and water (20 mL). To this was added hydrazine dihydrochloride (14.3 g, 0.136 mol) and the suspension was stirred overnight at room temperature before heating to reflux for an additional 12 hours. The solvent was removed under reduced pressure; the residue was dispersed in water and made basic with solid sodium hydrogencarbonate. The aqueous phase was extracted with ethyl acetate five times, dried over sodium sulfate and concentrated to dryness. The residue was further purified by a chromatography over silica gel (dichloromethane / ethanol 97-3) to yield compound **10k** (2.19 g, 9 %) as a low melting solid. ¹H (CDCl₃): 1.41 (m, 6H); 4.26 (q, 2H, J = 7.0); 4.38 (q, 2H, J = 7.0); 6.22 (s, 1H); 10.6 (s (br), 1H). ¹³C (CDCl₃): 14.2; 14.7; 61.4; 65.3; 93.2; 134.3; 159.6; 163.4.. HRMS: Calcd. for C₈H₁₂N₂O+ H : 185.0926. Found: m/z, 185.0980. 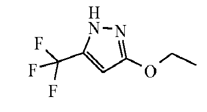 **3-Ethoxy-5-(trifluoromethyl)-1H-pyrazole (101)**¹⁵: Obtained as an oil that slowly cristallizes. ¹H (CDCl₃): 1.43 (t, 3H, J = 7.1); 4.18 (q, 2H, J = 7.1); 5.86 (d, 1H); 11.3 (s (br), 1H). ¹³C (CDCl₃): 14.4; 67.4; 85.2; 120.0 (q, J = 267); 141.1 (m); 158.3. 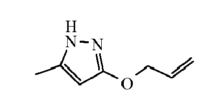 **3-Allyloxy-5-methyl-1H-pyrazole (10m):** This compound was obtained as an oil. ¹H (CDCl₃): 2.25 (s, 3H) ; 4.66 (m, 2H); 5.25 (m, 1H); 5.39 (dd, 1H, J = 1.6 and 17.2); 6.07 (m, 1H); 8.9 (s(1), 1H). ¹³C (CDCl₃): 11.9; 70.1; 90.1; 117.8; 133.8; 141.3; 163.9. HRMS: Calcd. for C₇H₁₀N₂O + H : 139.0871. Found: m/z, 139.0900. 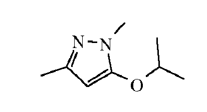 **5-isopropoxy-1,3-dimethyl-1H-pyrazole (11):** This compound was obtained as an oil when reacting 1.05 equivalents of methylhydrazine monohydrochloride and isopropylacetoacetate as described above. ¹H (CDCl₃): 1.32 (d, 6H, J = 6.1); 2.16 (s, 3H); 3.53 (s, 3H); 4.31 (sept, 1H, J = 6.1); 5.26 (s, 1H) ¹³C (CDCl₃): 14.7; 22.3; 33.5; 75.5; 85.5; 147.1; 154.3. Anal. Calcd. for C₈H₁₄N₂O: C, 14.55; H, 8.55; N, 33.94. Found: C, 14.61; H, 8.51; N, 33.97. 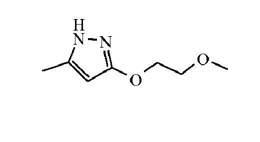 **3-(2-methoxyethoxy)-5-methyl-1H-pyrazole (13a)** This compound was obtained as an oil when reacting the hydrazine monohydrochloride and 2-methoxyethyl acetoacetate at room temperature in isopropanol overnight. ¹H (CDCl₃): 2.26 (s, 3H); 3.40 (s, 3H); 3.73 (m, 2H); 4.30 (m, 2H); 5.53 (s, 1H); 7.2 (s (br), 1H) ¹³C (CDCl₃): 11.6; 59.1; 67.9; 71.1; 90.1; 140.9; 163.6. m/z (LC/MS) = 157. 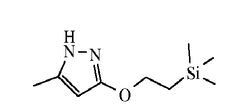 **5-methyl-3-(2-(trimethylsilyl)ethoxy)-1H-pyrazole (13b)** This compound was obtained as an oil when reacting the hydrazine monohydrochloride and 2-(trimethylsilyl)ethyl 3-oxobutanoate¹⁶ at room temperature in isopropanol overnight. ¹H (CDCl₃): 0.09 (s, 9H); 1.13 (m, 2H); 2.27 (s, 3H); 4.22 (m, 2H); 5.49 (s, 1H). 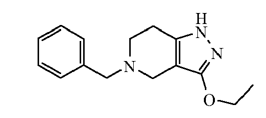 **5-Benzyl-3-ethoxy-4,5,6,7-tetrahydro-1H-pyrazolo[4,3-c]pyridine (16):** This compound was obtained from ethyl 1-benzyl-4-oxopiperidine-3-carboxylate hydrochloride. Nota, prior to the concentration of the ethanol as described above, a white precipitate (usually containing a mixture of compounds) was filtered off. M.p. = 101 °C. ¹H (CDCl₃): 1.37 (t, 3H, J = 7.3); 2.66 (m, 2H); 2.76 (m, 2H); 3.42 (s, 2H); 3.73 (s, 2H); 4.23 (q, 2H, J = 7.3); 7.34 (m, 5H) ¹³C (CDCl₃): 15.3; 22.6; 48.2; 49.8; 62.5; ;64.6; 99.9; 127.5; 128.7; 129.5; 138.7; 139.4; 159.7. Calcd. for C₁₅H₁₉N₃O + Na : 280.1426. Found: m/z, 280.1442. 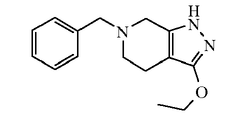 **6-Benzyl-3-ethoxy-4,5,6,7-tetrahydro-1H-pyrazolo[3,4-c]pyridine (17):** This compound was obtained from ethyl 1-benzyl-3-oxopiperidine-4-carboxylate hydrochloride. It was further purified by a chromatography over silica eluting with a mixture of dichloromethane-ethanol 96/4. M.p. = 111 °C. ¹H (CDCl₃): 1.38 (t, 3H, J = 7.0); 2.52 (t, 2H, J = 5.7); 2.77 (t, 2H, J = 5.7); 3.45 (s, 2H); 3.7 (s, 2H); 4.23 (q, 2H, J = 7.0); 7.34 (m, 5H) ¹³C (CDCl₃): 15.3; 19.7; 49.2; 50.9; 62.0; ;64.7; 99.2; 127.7; 128.7; 129.4; 138.5; 139.6; 160.8. Calcd. for C₁₅H₁₉N₃O + H : 258.1606. Found: m/z, 258.1620. 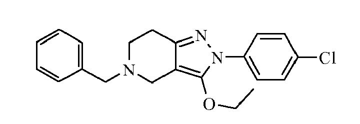 **5-benzyl-2-(4-chlorophenyl)-3-ethoxy-4,5,6,7-tetrahydro-2H-pyrazolo[4,3-c]pyridine (18):** This compound was obtained in a 5 % yield when reacting ethyl 1-benzyl-4-oxopiperidine-3-carboxylate hydrochloride and 4-chlorophenylhydrazine hydrochloride in boiling ethanol overnight and a chromatography over silica gel (dichloromethane - ethanol 98/2) followed by a second one over over neutral alumina containing 1.5 % of water (ethyl acetate / cyclohexane 99/1). ¹H (CDCl₃): 1.31 (t, 3H, J = 7.1); 2.81 (m, 4H); 3.62 (s, 2H); 3.75 (s, 2H); 4.12 (q, 2H, J = 7.1);7.36 (m, 7H); 7.84 (m, 2H). ¹³C (CDCl₃): 15.3; 24.3; 48.9; 50.1; 62.0; 68.6; 98.9; 123.2; 127.2; 128.4; 128.8; 128.9; 131.3; 137.7; 138.5; 148.1; 148.3. LC/MS: m/z = 368/370. 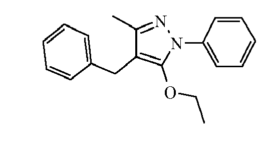 **4-benzyl-5-ethoxy-1-phenyl-3-methyl-1H-pyrazole (19):** Ethyl-2-benzylacetoacetate (1 g, 4.54 mmol) and phenylhydrazine hydrochloride (0.65 g, 4.54 mmol) were refluxed in ethanol (50 mL) for 4 hours. This was then concentrated to dryness and the residue dispersed in water made basic with solid sodium hydrogenocarbonate, and extracted with dichloromethane. The organic phase was washed with 1N sodium hydrogenocarbonate, dried over sodium sulfate and concentrated to dryness. The resulting residue was further purified by a chromatography over silica gel (cyclohexane - ethylacetate 4/1) to yield compound **19** as a white solid (0.5g; 37 %). ¹H (CDCl₃): 1.22 (t, 3H, J = 7.0); 2.20 (s, 3H); 3.84 (s, 2H); 3.88 (q, 2H, J = 7.0); 7.23-7.28 (m, 6H); 7.30-7.47 (m, 2H); 7.76-7.78 (d, 2H). ¹³C (CDCl₃): 13.5; 15.6; 28.9; 70.9; 105.0; 122.3; 126.4; 128.7; 128.8; 129.3; 139.5; 140.8; 148.8; 151.7. ¹⁵N (CDCl₃): -96 (CH₃); -169 (Ar). HRMS: Calcd. for C₁₉H₂₀N₂O + H : 293.1654. Found: m/z, 293.1684. 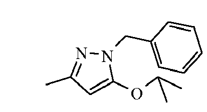 **1-benzyl-5-isopropoxy-3-methyl-1H-pyrazole (20):** Isopropyl acetoacetate (0.23 g, 1.59 mmol) and benzylhydrazine dihydrochloride (0.32 g, 1.64 mmol) were refluxed in isopropanol (50 mL) for 2 hours. This was then concentrated to dryness and the residue dispersed in water, made basic with solid sodium hydrogenocarbonate, and extracted with dichloromethane. The organic phase was washed with 1N sodium hydrogenocarbonate, dried over sodium sulfate and concentrated to dryness. The resulting residue was further purified by a chromatography over silica gel (cyclohexane - ethylacetate 1/1) to yield compound **20** as an oil (0.23 g; 62 %; weakly visible in UV). ^{1H} (CDCl₃): 1.30 (d, 6H, J = 6.2); 2.21 (s, 3H); 4.34 (sept, 1H, J = 6.2); 5.08 (s, 2H); 5.33 (s, 1H); 7.24 (m, 3H); 7.31 (m, 2H). ¹³C (CDCl₃): 14.6; 21.9; 50.2; 75.0; 85.4; 127.2; 127.4; 128.4; 137.7; 147.3; 153.9. ¹⁵N (CDCl₃): -98 (CH₃, CH₂); -190 (CH₂, H-4). HRMS: Calcd. for C₁₄H₁₈N₂O + H : 231.1497. Found: m/z, 231.1525. 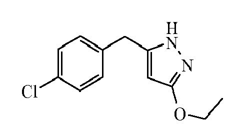 **5-(4-chlorobenzyl)-3-ethoxy-1H-pyrazole (22):** Obtained after 24 hours of reflux as a white crystals after two chromatographies, the first one over silica gel (dichloromethane / ethanol 99/1) the second over neutral alumina containing 1.5 % of water (dichloromethane / ethanol 99/1). ¹H (CDCl₃): 1.40 (t, 3H, J = 7.0); 3.94 (s, 2H); 4.17 (q, 2H, J = 7.0); 5.51 (s, 1H); 7.18 (m, 2H); 7.31 (m, 2H). ¹³C (CDCl₃): 14.8; 32.2; 65.0; 89.6; 128.9; 130.0; 132.8; 135.8; 144.0; 163.1. LC/MS: m/z = 237/239. 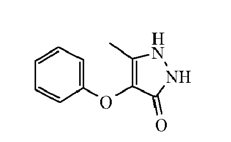 **5-methyl-4-phenoxy-1H-pyrazol-3(2H)-one (24a):** Obtained after 18 hours of reflux by a filtration as a beige solid before the purification of the filtrate as described below to obtain the 4-phenoxy-3-ethoxy-5-methyl-1H-pyrazole **(25a).** ¹H (DMSOd6): 1.97 (s, 3H); 6.85 (m, 2H); 6.99 (m, 1H); 7.28 (m, 2H) ; 9.6 (s(1) 1H); 11.3 (s(1), 1H). ¹³C (DMSO*d6*): 8.8; 114.7; 120.1; 121.4; 129.4; 130.2; 152.4; 158.7. Calcd. for C₁₀H₁₀N₂O₂ + H : 191.0821. Found: m/z, 191.0763. 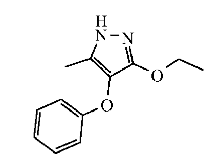 **4-phenoxy-3-ethoxy-5-methyl-1H-pyrazole (25a):** Obtained as pale yellow crystals after after 18 hours of reflux followed by filtration of 5-methyl-4-phenoxy-1H-pyrazol-3(2H)-one as described above and a chromatography over silica gel (dichloromethane - ethanol 99/1). ¹H (CDCl₃): 1.34 (t, 3H, J = 7.1); 2.14 (s, 3H); 4.26 (q, 1H, J = 7.1); 6.96 (m, 2H); 7.00 (m, 1H); 7.30 (m, 2H). ¹³C (CDCl₃): 9.1; 14.9; 64.9; 115.0; 121.8; 121.9; 129.4; 132.0; 155.1; 158.7. Calcd. for C₁₂H₁₄N₂O₂ + H : 219.1134. Found: m/z, 219.1054. 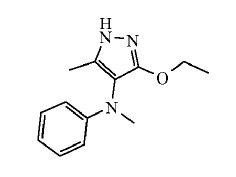 **3-ethoxy-N,5-dimethyl-N-phenyl-1H-pyrazol-4-amine (25b):** Obtained as a red oil after a chromatography over silica gel (cyclohexane / ethyl acetate). ¹H (CDCl₃): 1.34 (t, 3H, J = 7.1); 2.14 (s, 3H); 3.20 (s, 3H); 4.30 (q, 2H, J = 7.1); 6.66 (m, 2H); 6.77 (m, 1H); 7.23 (m, 2H). ¹³C (CDCl₃): 9.9; 15.0; 39.1; 64.6; 112.4; 112.5; 116.8; 128.9; 137.5; 149.3; 159.2.m/z (LC/MS) = 232. 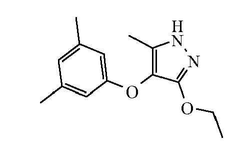 **4-(3,5-dimethylphenoxy)-3-ethoxy-5-methyl-1H-pyrazole (27):** Obtained as white crystals after 18 hours of reflux and a chromatography over silica gel (dichloromethane - ethanol 99/1) followed by a recrystallization in cyclohexane. ¹H (CDCl₃): 1.34 (t, 3H, J = 7.1); 2.16 (s, 3H); 2.28 (s, 6H); 4.28 (q, 2H, J = 7.1); 6.58 (s, 2H); 6.66 (s, 1H). ¹³C (CDCl₃): 9.4; 15.0; 21.5; 65.2; 112.8; 122.0; 123.9; 133.5; 139.4; 155.8; 158.8. Calcd. for C₁₄H₁₈N₂O₂ + H : 247.1447. Found: m/z, 247.1371.

### Results

The use of 1.05 equivalents of hydrazine monohydrochloride in boiling ethanol led to 55% yield of 3-ethoxypyrazole **(4c).** Increasing the proportion to two equivalents of hydrazine monohydrochloride led to a lower yield. A trial run with 0.5 equivalent of hydrazine and 0.5 equivalent of hydrazine monohydrochloride also led to a lower yield of **4c.** Replacing the hydrazine monohydrochloride with hydrazine sulfate in refluxing ethanol gave compound **4c,** although still in lower yield, whereas hydrazine acetate only led to compound **3c.** Other salts were tried (hydrobromide, tetrafluoroboride or monophosphate) without any improvement. The reaction could also be run with hydrazine monohydrochloride overnight at room temperature and gave a similar yield of **4c.** The absence of ethanol or the use of a different solvent (THF, toluene, DMF, water) at room temperature gave lower yields of **4c.** Compounds **10b-k** were obtained by using the optimal conditions found so far i.e.: refluxing alkyl acetoacetates **9a-k** and 1.05 equivalents of hydrazine monohydrochloride, in either the corresponding alkylalcohol or isopropanol in the case of compound **9a,** for eight hours. From the allyl acetoacetate **9m,** a transesterification was observed in boiling isopropanol and thus the reaction was undertaken at room temperature overnight instead.

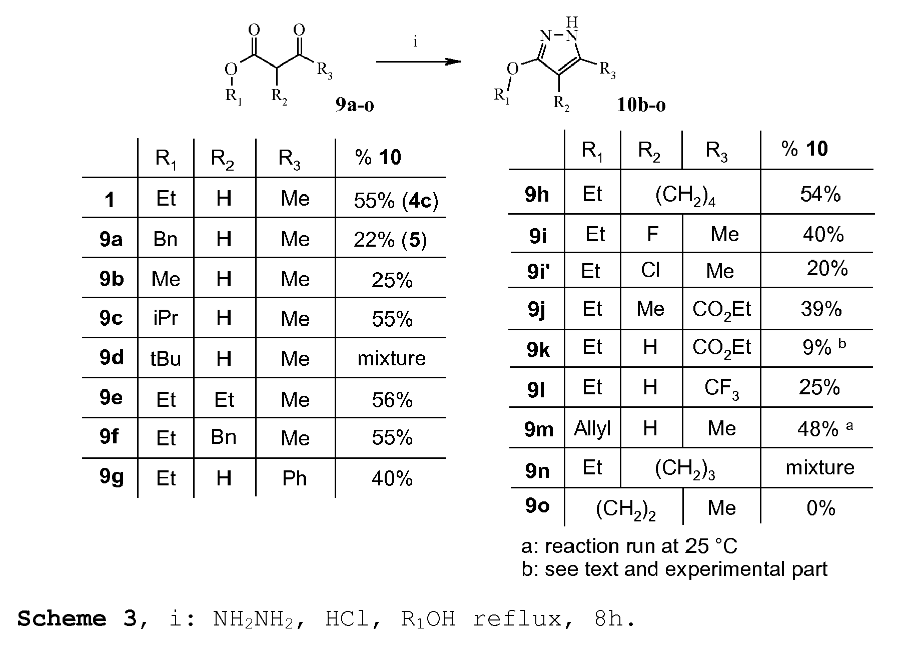

The results reported in scheme 3 point out that starting from ethyl or isopropyl acetoacetates **1** or **9c** the reaction led mainly to the corresponding O-alkoxypyrazoles **4c** and **10c** (55% yield). On the other hand, the methyl or benzyl acetoacetates **9a-b** gave lower yield of alkoxypyrazoles. The acidic lability of a benzyl and a methyl groups may explain the lower yield observed and the volatility and water solubility of **10b** may also provide an explanation in this case. However, concerning an eventual acidic lability, trials at room temperature or with shorter reaction time provided compound **5** or **10b** in the same yield range. In the case of the tertbutyl acetoacetate, a mixture of compounds that could not be properly purified was obtained. If the ethyl or the benzyl group of ethyl acetoacetates **9e** and **9f** do not seem to hinder this reaction, the phenyl of compound **9g** leads to a lower yield. Interestingly, a fluorine or a chlorine atoms are compatible with the reaction conditions and we obtained 40 % of the 4-fluoropyrazole **10i** or 20 % of the 4-chloropyrazole **10i'** from the corresponding 4-halogenoacetates. Moreover, an ester function did not hinder the preparation of the carboxyl-bearing compound **10j** in a 39 % yield from diethyl 2-methyl-3-oxosuccinate **(9j).** From the commercially available sodium salt of diethyl 2-oxosuccinate **(9k)** a different procedure was used which led to the expected compound **10k** although in an unoptimized 9 % yield (see experimental part). Finally, the comparison of the trials starting with the cyclic compounds **9h** and **9n-o** illustrates some of the structural constraints which limit this method. Thus the six-membered cyclic acetoacetate **9h** gives a 54% yield of the corresponding 3-ethoxytetrahydroindazole **(10h)** but trials with the five-membered cyclic acetoacetate **9n** leads to an inseparable mixture which probably contains the target compound. In the case of **9o,** the water elimination seems to be even more disfavoured and no O-alkylated substances could be detected in ¹H NMR spectra of the reaction mixture.

As shown in scheme 4, a similar yield increase was also observed when reacting 1.05 equivalents of methylhydrazine monohydrochloride with isopropylacetoacetate **9c** as the corresponding 2,5-dimethyl-3-isopropyloxypyrazole **(11)** was obtained in a 69% yield instead of the previously reported 30%.¹³ The regioselectivity of this reaction, which corresponds to the reported examples,^{17, 18} was determined by ¹³C-¹H long distance correlations.

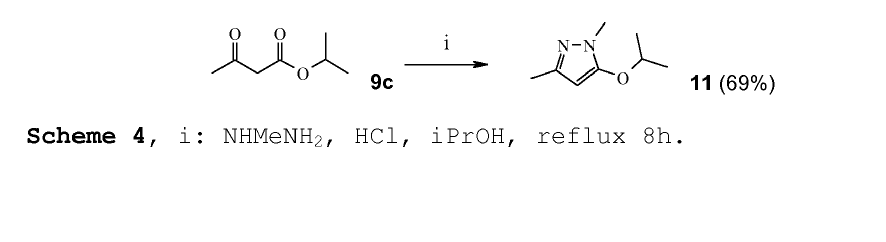

As shown in scheme 5, further trials with various acetoacetate-bearing starting materials illustrate the scope of this reaction. From 2-methoxyethyl acetoacetate **(12a)** and hydrazine hydrochloride, the corresponding 3-alkoxypyrazole **13a** was obtained when the reaction was run at room temperature in isopropanol. Moreover, from compound **12b,**¹⁶ the corresponding trimethylsilyl-bearing 3-alkoxypyrazole **(13b)** was obtained. From the isomeric tertiary amine-containing oxopiperidine-carboxylate **14** and **15,** the corresponding tetrahydropyrazolopyridines **16** and **17** were also easily obtained. From compound **14,** its reaction with 4-chlorophenylhydrazine hydrochloride unambiguously led to small amount of the N-arylated isomer **18** which was usefull as a control reaction to ascertain the regio chemistry of further transformations described below. Such control reactions were also made from compound **9f** to give the N-phenyl isomer **19** and from **9c** to give the N-benzyl isomer **20.**

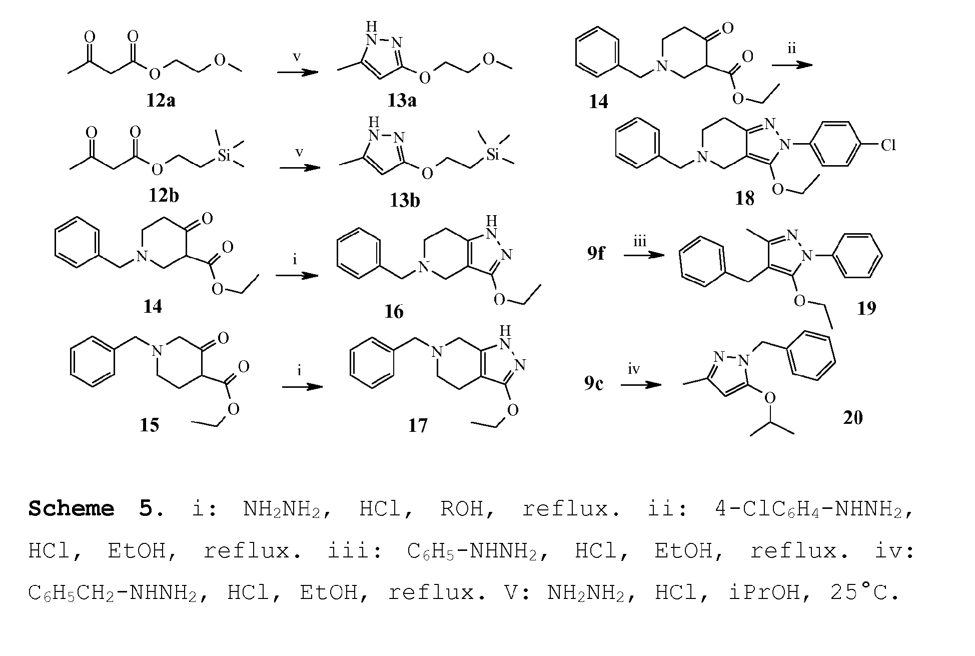

Moreover, as depicted in scheme 6, the reaction of the benzyl-bearing compound **21** led to the pyrazole **22.** From the readily available¹⁹ phenoxy-bearing acetoacetate **23a,** the corresponding pyrazolone **24** as well as the 3-ethoxypyrazole **25a** were obtained. This was also achieved from compound **26** which led top compound **27.** Moreover, from ethyl 2-(methyl (phenyl) amino)-3-oxobutanoate **(23b)**²⁰,. the N-methylaniline-bearing 3-alkoxypyrazole **25b** was obtained.

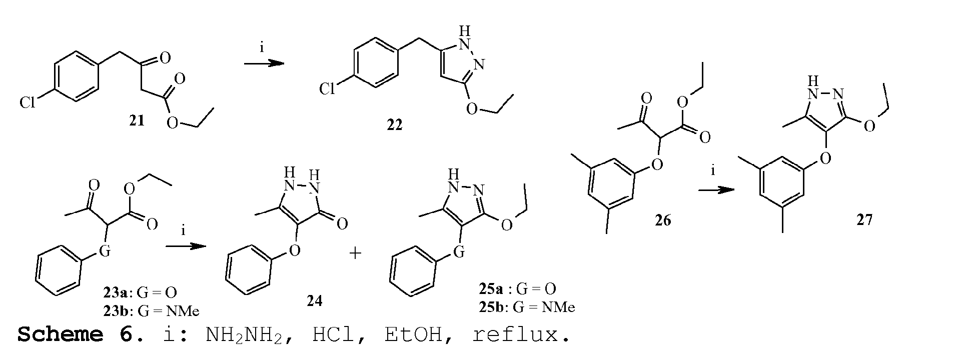

### Example 2: reactivity of the N1 centre of 3-alkoxypyrazoles

The features of the devices used to conduct analyses of all of the compounds described in Example 2 are indicated hereinbelow:

A Biotage initiator 2 microwave oven was used for of the reactions requiring microwaves irradiations. ¹H NMR and ¹³C NMR spectra were recorded on a Bruker Avance 400 spectrometers at 400 MHz and 100 MHz, respectively. Unless otherwise noted, CDCl₃ was the solvent used. Column chromatography were performed either Merck silica gel 60 (0.035-0.070 mm) or neutral alumina containing a suitable proportion of water, using a solvent pump operating at pressure between 2 and 7 bar (25-50 mL/mn) and an automated collecting system driven by a UV detector set to 254 nm unless stated otherwise (i.e. if ethylacetate was used then it would be set to 280 nm). Sample deposition was always carried out by absorption of the mixture to be purified on a small amount of the solid phase followed by its deposition of the top of the column. The low resolution mass spectra were obtained on an Agilent 1100 serie LC/MSD system using an atmospheric electrospray ionisation system and the high resolution mass spectroscopy spectra (HRMS) were obtained using a Waters Micromass Q-Tof with an electrospray ion source.

### A-4-Halogenation of some 3-alkoxypyrazoles

Prior to the study of the 3-alkoxypyrazoles nitrogens reactivity, the halogenation of the carbon 4 of 3-alkoxypyrazoles **10c, 10g** and **10l** led to compounds **28-31** as described below. 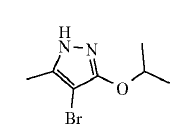 **4-bromo-3-isopropoxy-5-methyl-1H-pyrazole (28):** Compound **10c** (3.94 g, 28.1 mmol), sodium hydrogenocarbonate (2.80 g, 35.2 mmol) and bromine (1.73 mL, 33.7 mmol) were mixed in 100 mL of ethanol. The solution was stirred at room temperature for 1 hour. After concentration to dryness, the residue was washed with sodium hydrogenocarbonate 1N and extracted with dichloromethane. The organic phase was dried over anhydrous sodium sulfate and concentrated to dryness. The residue was purified by flash chromatography on silica gel (dichloromethane-ethanol 99/1) to provide compound **28** (3.97 g, 64 %) as a white solid. ¹H (CDCl₃): 1.38 (d, 6H, J = 6.1); 2.24 (s, 3H); 4.85 (sept, 1H, J = 6.1); 8.1 (s(l), 1H). ¹³C (CDCl₃): 11.1; 22.54; 73.2; 81.2; 139.6; 159.8. HRMS: Calcd. for C₇H₁₁⁷⁹BrN₂O + H : 219.0133. Found: m/z, 219.0169. 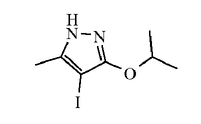 **4-Iodo-3-isopropoxy-5-methyl-1H-pyrazole (29):** Compound **10c** (6.52 g, 0.046 mol), sodium iodide (7.68 g, 0.051 mol) and potassium carbonate (16 g, 0.116 mol) were dissolved in water (250 mL) and ethanol (60 mL). To this was added iodine (15.3 g, 0.06 mol). The resulting suspension was stirred for 90 minutes, decolorized (if necessary) with sodium bisulfite and diluted with brine (1 L). The resulting precipitate was filtered, washed with water and dried under vacuum while heating at 80 °C in a large Petri dish in order to sublimate the iodoform also occurring in this reaction to yield compound **29** as an yellow solid (10.2 g; 82 %). ¹H (CDCl₃): 1.38 (d, 6H, J = 6.3); 2.25 (s, 3H); 4.84 (sept, 1H, J = 6.3). ¹³C (CDCl₃): 12.6; 22.5; 48.3; 73.8; 142.9; 162.9. HRMS: Calcd. for C₇H₁₁IN₂O + H : 266.9994. Found: m/z, 267.0023. 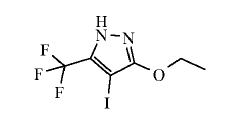 **3-ethoxy-4-iodo-5-(trifluoromethyl)-1H-pyrazole (30):** The 3-ethoxy-5-(trifluoromethyl)-1H-pyrazole (101) (1.05 g, 5.83 mmol), sodium iodide (1.31 g, 8.75 mmol) were stirred in a 1N solution of potassium carbonate (70 mL) and ethanol (7 mL). To this was added iodine (2.22 g, 8.75 mmol). The resulting solution was stirred for 2 hours, decolorized (if necessary) with sodium bisulfite and diluted with brine (40 mL). The resulting precipitate was filtered, washed with water and dried under vacuum to yield compound **30** as an white solid (0.82 g; 45 %). Nota: further product could be obtained by an extraction of the filtrate. ¹H (CDCl₃): 1.45 (d, 3H, J = 7.0); 4.34 (q, 2H, J = 7.0). ¹³C (CDCl₃): 14.6; 44.6; 66.4; 119.2 (q, J = 260 Hz); 136.3 (1); 162.4. m/z (LC/MS) = 307. 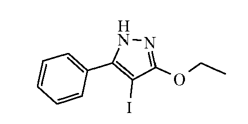 **3-ethoxy-4-iodo-5-phenyl-1H-pyrazole (31):** Compound **10g** (1.6 g, 8.51 mmol), potassium carbonate (3.52 g, 25.53 mmol), sodium iodide (1.27 g, 8.51 mmol) were dissolved in 80 mL of a water ethanol mixture 4-6. To this was added iodine (3.2 g, 12.76 mmol). The solution was stirred for one hour dispersed in water (300 mL) and the resulting precipitate was filtered, washed with water and dried under high vacuum to yield compound **31** as a slightly yellow powder (2.02 g, 75 %). ¹H (CDCl₃): 1.38 (t, 3H, J = 7.0); 4.21 (q, 2H, J = 7.0); 7.48 (m, 3H); 7.66 (m, 2H); 10.53 (s(1), 1H). ¹³C (CDCl₃): 14.8; 45.1; 65.2; 126.6 (two signals); 128.8; 129.3; 144.5; 163.8. HRMS: Calcd. for C₁₁H₁₁IN₂O + H : 314.9994. Found: m/z, 315.0020.

### B-General procedure for the N-arylation of 3-alkoxypyrazoles using arylboronates.

The corresponding alkoxypyrazole (1.45 mmol), arylboronic acid, such as compound **32a-d** (1.60 mmol), pyridine (3 mmol, dried over 4Å molecular sieves), 4Å molecular sieves (0.6 g) and copper (II) acetate hydrate (2.2 mmol) were dispersed in dichloromethane (20 mL). The reaction was stirred in open air for 48 hours. After concentration to dryness, the residue was absorbed on a small amount of silica gel and the resulting arylated isomers purified as described below. An alternative method was to run the reaction in boiling acetonitrile (20 mL, dried over 4Å molecular sieves) for 12 hours using the same reagents, as long as the reaction media was protected from moisture by a calcium chloride guard.
**Isomers 33a and 34a:** The reaction was run dichloromethane; a chromatography over silica gel (cyclohexane - ethyl acetate 9/1) led to 39 % of compound **33a** followed by 53% of compound **34a.** 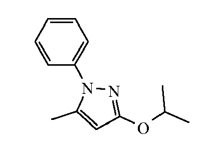 **3-isopropoxy-5-methyl-1-phenyl-1H-pyrazole (33a):** Obtained as an oil. ¹H (CDCl₃): 1.39 (d, 6H, J = 6.1); 2.31 (s, 3H); 4.85 (sept, 1H, J = 6.1); 5.65 (s, 1H); 7.28-7.46 (m, 5H). ¹³C (CDCl₃): 13.2; 22.6; 71.7; 94.1; 124.8; 127.0; 129.3; 140.4 (two signals ?); 163.0. ¹⁵N (CDCl₃): -184 (CH₃, H-4, Ar). HRMS: Calcd. for C₁₃H₁₆N₂O + H : 217.1341. Found: m/z, 217.1372. 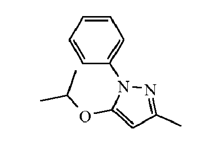 **5-isopropoxy-3-methyl-1-phenyl-1H-pyrazole (34a):** Obtained as an oil. ¹H (CDCl₃): 1.40 (d, 6H, J = 6.1); 2.31 (s, 3H); 4.45 (sept, 1H, J = 6.1); 5.48 (s, 1H); 7.21-7.73 (m, 5H). ¹³C (CDCl₃): 14.9; 22.3; 76.0; 87.4; 122.3; 126.0; 129.0; 139.4; 149.1; 154.4. ¹⁵N (CDCl₃): -99 (CH₃); -185 (H-4, Ar). HRMS: Calcd. for C₁₃H₁₆N₂O + H : 217.1341. Found: m/z, 217.1381.

Isomers **33b** and **34b:** A chromatography over silica gel (cyclohexane - ethyl acetate 98/2) led to 20 % of compound **33b** followed by 37% of compound **34b.** 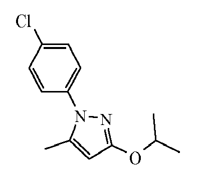 **1-(4-chlorophenyl)-3-isopropoxy-5-methyl-1H-pyrazole (33b):** Obtained as an oil. ¹H (CDCl₃): 1.38 (d, 6H, J = 6.1); 2.29 (s, 3H); 4.83 (sept, 1H, J = 6.1); 5.65 (s, 1H); 7.35-7.40 (m, 4H). ¹³C (CDCl₃): 12.9; 22.1; 71.4; 94.3; 125.3; 129.0; 132.1; 138.6; 140.0; 162.8. ¹⁵N (CDCl₃): -185 (CH₃; H-4; Ar). HRMS: Calcd. for C₁₃H₁₅N₂O³⁵Cl + H : 251.0951. Found: m/z, 251.0967. 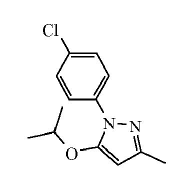 **1-(4-chlorophenyl)-5-isopropoxy-3-methyl-1H-pyrazole (34b):** Obtained as an oil. ¹H (CDCl₃): 1.38 (d, 6H, J = 6.1); 2.27 (s, 3H); 4.42 (sept, 1H, J = 6.1); 5.45 (s, 1H); 7.28-7.37 (m, 2H); 7.67-7.71 (m, 2H). ¹³C (CDCl₃): 14.3; 21.9; 75.9; 87.2; 123.0; 128.8; 131.2; 137.3; 149.1; 154.1. ¹⁵N (CDCl₃): - 100 (CH₃); -184 (H-4 and Ar). HRMS: Calcd. for C₁₃H₁₅N₂O³⁵Cl + H : 251.0903. Found: m/z, 251.0951.

**Isomers 33c and 34c:** The reaction was run in boiling acetonitrile. A chromatography over silica gel (cyclohexane - ethyl acetate 4/1) led to 37 % of compound **33c** and a fraction containing compound **34c** and unidentified material. This second fraction was further purified by a second chromatography (cyclohexane - ethyl acetate 4/1) to give 40 % of compound **34c.** 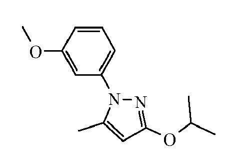 **3-isopropoxy-1-(3-methoxyphenyl)-5-methyl-1H-pyrazole (33c):** Obtained as an oil. ¹H (CDCl₃): 1.39 (d, 6H, J = 6.1); 2.31 (s, 3H); 3.85 (s, 3H); 4.85 (sept, 1H, J = 6.1); 5.65 (s, 1H); 6.85-7.35 (m, 4H). ¹³C (CDCl₃): 13.0; 22.2; 55.4; 71.4; 93.8; 110.2; 112.5; 116.5; 129.5; 140.0; 141.1; 160.1; 162.6. ¹⁵N (CDCl₃) : -182 (CH₃; H-4 HRMS: Calcd. for C₁₄H₁₈N₂O₂ + H : 247.1447. Found: m/z, 247.1468. 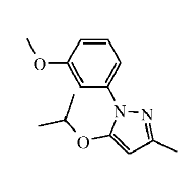 **5-isopropoxy-1-(3-methoxyphenyl)-3-methyl-1H-pyrazole (34c):** Obtained as an oil. ¹H (CDCl₃): 1.41 (d, 6H, J = 6.1); 2.29 (s, 3H); 3.86 (s, 3H); 4.45 (sept, 1H, J = 6.1); 5.48 (s, 1H); 6.80 (m, 1H); 7.32 (m, 3H). ¹³C (CDCl₃) : 14.5; 21.9; 55.3; 75.6; 87.1; 107.4; 111.8; 114.1; 129.3; 140.1; 148.7; 154.1; 159.9. ¹⁵N (CDCl₃): -100 (CH₃); -182 (H-4, ArH). HRMS: Calcd. for C₁₄H₁₈N₂O₂ + H : 247.1447. Found: m/z, 247.1465.

**Isomers 33d and 34d:** The reaction was run in boiling acetonitrile. A chromatography over silica gel (cyclohexane - ethyl acetate 4/1) led to 15 % of compound **33d** and a fraction containing compound **34d** and some starting material. This second fraction was further purified by a second chromatography (cyclohexane - ethyl acetate 4/1) to give 11 % of compound **34d**. 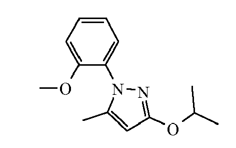 **3-isopropoxy-1-(2-methoxyphenyl)-5-methyl-1H-pyrazole (33d):** Obtained as an oil. ¹H (CDCl₃): 1.36 (d, 6H, J = 6.1); 2.07 (s, 3H); 3.80 (s, 3H); 4.84 (sept, 1H, J = 6.1); 5.61 (s, 1H); 7.05 (m, 2H); 7.35 (m, 2H). ¹³C (CDCl₃): 12.0; 22.6; 56.2; 71.4; 92.2; 112.6; 121.3; 129.2; 129.9; 130.0; 142.5; 155.3; 163.0. ¹⁵N (CDCl₃): -192 (CH₃; H-4). HRMS: Calcd. for C₁₄H₁₈N₂O₂ + H : 247.1447. Found: m/z, 247.1459. 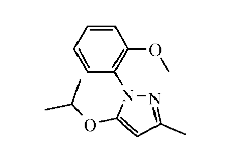 **5-isopropoxy-1-(2-methoxyphenyl)-5-methyl-1H-pyrazole (34d):** Obtained as a solid. ¹H (CDCl₃): 1.28 (d, 6H, J = 6.1); 2.28 (s, 3H); 3.78 (s, 3H); 4.37 (sept, 1H, J = 6.1); 5.44 (s, 1H); 6.99 (m, 2H); 7.32 (m, 2H). ¹³C (CDCl₃): 14.7; 21.9; 55.6; 75.0; 85.6; 111.9; 120.4; 127.2; 128.9; 129.5; 148.7; 154.8; 154.9. ¹⁵N (CDCl₃): ¹⁵N (CDCl₃): -95 (CH₃); -191 (H-4, ArH). HRMS: Calcd. for C₁₄H₁₈N₂O₂ + H : 247.1447. Found: m/z, 247.1469.

**Isomers 35b and 36b:** A chromatography over silica gel (cyclohexane - ethyl acetate 99/1) led to 31 % of compound **35b** followed by 42 % of compound **36b** when the reaction was run in acetonitrile and to 39 % of compound **35b** followed by 28 % of compound **36b** when it was run in dichloromethane. 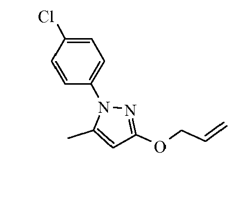 40
**3-(allyloxy)-1-(4-chlorophenyl)-5-methyl-1H-pyrazole (35b):** Obtained as an oil. ¹H (CDCl₃): 2.30 (s, 3H); 4.73 (m, 2H); 5.27 (d, 1H, J = 9.0); 5.41 (d, 1H, J = 15.0); 5.70 (s, 1H); 6.09 (m, 1H); 7.36-7.42 (m, 4H). ¹³C (CDCl₃): 12.9; 69.4; 93.7; 117.6; 125.4; 129.1; 132.3; 133.4; 138.5; 140.4; 163.2. ¹⁵N (CDCl₃) : -180 (CH₃ and H-4). HRMS: Calcd. for C₁₃H₁₃N₂O³⁵Cl + H : 249.0795. Found: m/z, 249.0815. 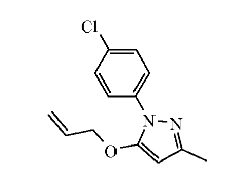 **5-(allyloxy)-1-(4-chlorophenyl)-3-methyl-1H-pyrazole (36b):** Obtained as an oil. ¹H (CDCl₃): 2.29 (s, 3H); 4.65 (m, 2H); 5.33-5.48 (m, 2H); 5.51 (s, 1H); 6.04 (m, 1H); ); 7.28 (m, 2H); 7.68 (m, 2H). ¹³C (CDCl₃): 14.5; 69.4; 87.1; 118.6; 122.7; 128.8; 131.0; 131.7; 137.4; 149.0; 154.5. ¹⁵N (CDCl₃): -100 (CH₃); -185 (H-4 and ArCl). HRMS: Calcd. for C₁₃H₁₃N₂O³⁵Cl + H : 249.0795. Found: m/z, 249.0821.

Isomers **37a** and **19**: The reaction was run in dichloromethane and a chromatography over silica gel (cyclohexane - dichloromethane 85/15) led to 75 % of compound **37a** followed by only traces of compound **19**. 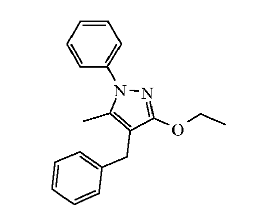 **4-benzyl-3-ethoxy-1-phenyl-5-methyl-1H-pyrazole** (**37a):** Obtained as a white solid. ¹H (CDCl₃): 1.22 (t, 3H, J = 7.0); 2.17 (s, 3H); 3.81 (s, 2H); 3.86 (q, 2H, J = 7.0); 7.21-7.74 (m, 10H). ¹³C (CDCl₃): 13.5; 15.6; 28.8; 70.9; 105.0; 122.3; 126.4; 125.5; 128.5; 128.8; 129.3; 151.7. ¹⁵N (CDCl₃): -185 (CH₃). HRMS: Calcd. for C₁₉H₂₀N₂O + H : 293.1654. Found: m/z, 293.1684. 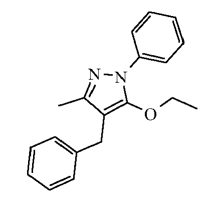 **4-benzyl-5-ethoxy-1-phenyl-3-methyl-1H-pyrazole (19):** As this compound could not be isolated in sizable quantity in the course of the arylation of compound **10f,** it was prepared in 37% yield by using the method of Example 1 as described above.

Isomers **37b** and **38b**: The reaction was run in dichloromethane and a chromatography over silica gel (cyclohexane - ethylacetate 98/2) led to 66 % of compound **37b** followed by less than 10 % of compound **38b** as it still contained traces of unidentified compounds and had to be purified again by another chromatography over silica gel. 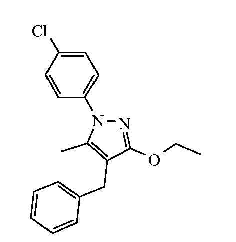 **4-benzyl-1-(4-chlorophenyl)-3-ethoxy-5-methyl-1H-pyrazole (37b):** Obtained as white solid. ¹H (CDCl₃) : 1.42 (t, 3H, J = 7.0); 2.21 (s, 3H); 3.76 (s, 2H); 4.34 (q, 2H, J = 7.0); 7.21-7.42 (m, 9H). ¹³C (CDCl₃) : 11.4; 14.9; 28.1; 64.3; 105.2; 125.4; 125.8; 128.2; 128.3; 129.0; 131.9; 137.4; 138.8; 141.0; 162.2. ¹⁵N (CDCl₃): -187 (CH₃ and ArCl). HRMS: Calcd. for C₁₉H₁₉³⁵ClN₂O + H : 327.1264. Found: m/z, 327.1244. 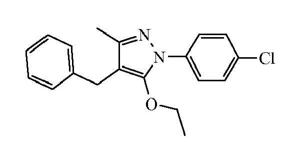 **4-benzyl-1-(4-chlorophenyl)-5-ethoxy-3-methyl-1H-pyrazole (38b):** Obtained as an oil. ¹H (CDCl₃): 1.21 (t, 3H, J = 7.0); 2.15 (s, 3H); 3.81 (s, 2H); 3.87 (q, 2H, J = 7.0); 7.15-7.71 (m, 9H). ). ¹³C (CDCl₃): 13.1; 15.2; 28.5; 70.6; 104.8; 122.9; 126.1; 128.1; 128.4; 129.0; 131.4; 137.6; 140.2; 148.9; 151.4. ¹⁵N (CDCl₃): -100 (CH₃); -180 (ArCl). HRMS: Calcd. for C₁₉H₁₉³⁵ClN₂O + H : 327.1264. Found: m/z, 327.1258.

Isomers **37c** and **38c**: The reaction was run in dichloromethane and a chromatography over silica gel (cyclohexane - ethylacetate 98/2) led to 75 % of compound **16c** followed by only traces of compound **38c**. 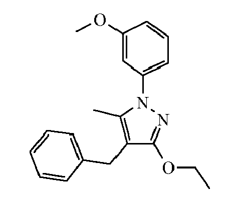 **4-benzyl-3-ethoxy-1-(3-methoxyphenyl)-5-methyl-1H-pyrazole** (**37c):** Obtained as an oil. ¹H (CDCl₃): 1.44 (t, 3H, J = 7.0); 2.23 (s, 3H); 3.79 (s, 2H); 3.86 (s, 3H); 4.38 (q, 2H, J = 7.0); 6.86-7.37 (m, 9H). ¹³C (CDCl₃) : 11.5; 15.0; 28.1; 55.4; 64.2; 104.7; 110.3; 112.3; 116.7; 125.8; 128.2; 128.3; 129.5; 137.5; 141.2; 141.3; 160.1; 162.0. ¹⁵N (CDCl₃): -186 (CH₃). HRMS: Calcd. for C₂₀H₂₂N₂O₂ + H : 323.1760. Found: m/z, 323.1751.
Isomers **39a** and **40a**: The reaction was run either in dichloromethane or in acetonitrile; a chromatography over silica gel (cyclohexane - dichloromethane 4/6) led to 73 % of compound **39a** followed by 14 % of compound **40a** in both cases. 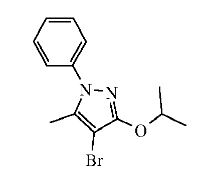 **4-bromo-3-isopropoxy-5-methyl-1-phenyl-1H-pyrazole (39a):** Obtained as an oil. ¹H (CDCl₃): 1.42 (d, 6H, J = 6.2); 2.31 (s, 3H); 5.00 (sept, 1H, J = 6.2); 7.35 - 7.48 (m, 5H). ¹³C (CDCl₃): 12.4; 22.6; 72.7; 83.3; 124.8; 127.6; 129.2; 138.7; 140.3; 159.7. ¹⁵N (CDCl₃) : -182 (CH₃, Ar). HRMS: Calcd. for C₁₃H₁₅⁷⁹BrN₂O + H : 295.0446. Found: m/z, 295.0471. 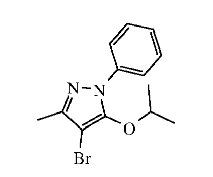 **4-bromo-5-isopropoxy-3-methyl-1-phenyl-1H-pyrazole (40a):** Obtained as an oil. ¹H (CDCl₃): 1.24 (d, 6H, J = 6.1); 2.29 (s, 3H); 4.62 (sept, 1H, J = 6.1); 7.28 (m, 1H); 7.41 (m, 2H); 7.66 (m, 1H). ¹³C (CDCl₃): 13.1; 22.3; 78.1; 82.8; 122.6; 126.7; 128.8; 138.7; 147.4; 149.5. ¹⁵N (CDCl₃) : -95 (CH₃) ; - 175 (Ar). HRMS: Calcd. for C₁₃H₁₅⁷⁹BrN₂O + H : 295.0446. Found: m/z, 295.0472.

Isomers **39b** and **40b**: The reaction was run in dichloromethane; a chromatography over silica gel (cyclohexane - dichloromethane 7/3) led to 70 % of compound **39b** followed by 19 % of compound **40b** still containing 5 % of **39b** which could be removed by a second chromatography. 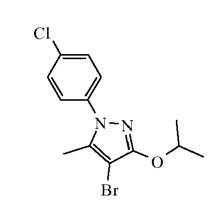 **4-bromo-1-(4-chlorophenyl)-3-isopropoxy-5-methyl-1H-pyrazole (39b):** Obtained as a white solid. ¹H (CDCl₃): 1.41 (d, 6H, J = 6.1); 2.31 (s, 3H); 4.97 (sept, 1H, J = 6.1); 7.37 (m, 2H); 7.42 (m, 2H). ¹³C (CDCl₃): 12.4; 22.5; 72.8; 83.9; 125.7; 129.6; 133.1; 138.7; 138.8; 159.9. ¹⁵N (CDCl₃): -185 (CH₃ and Ar). HRMS: Calcd. for C₁₃H₁₄³⁵Cl⁷⁹BrN₂O + H : 329.0056. Found: m/z, 329.0083. 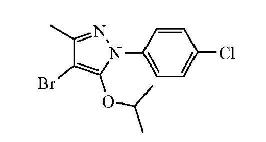 **4-bromo-1-(4-chlorophenyl)-5-isopropoxy-3-methyl-1H-pyrazole (40b):** Obtained as an oil. ¹H (CDCl₃) : 1.25 (d, 6H, J = 6.1) ; 2.26 (s, 3H); 4.68 (sept, 1H, J = 6.1); 7.41 (m, 1H); 7.63 (m, 2H); 7.66 (m, 1H). ¹³C (CDCl₃) : 13.6; 22.8; 72.8; 78.5; 123.8; 129.4; 132.5; 137.7; 148.2; 149.9. ¹⁵N (CDCl₃): -96 (CH₃) ; -178 (Ar). HRMS: Calcd. for C₁₃H₁₄³⁵Cl⁷⁹BrN₂O + H : 329.0056. Found: m/z, 329.0079.

Isomers **39d** and **40d**: The reaction was run in dichloromethane; a chromatography over silica gel (cyclohexane - dichloromethane 1/1) led to 21 % of compound **39d** and no compound **40d** could be isolated. 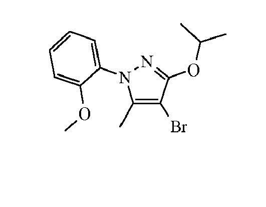 **4-bromo-1-(2-methoxyphenyl)-3-isopropoxy-5-methyl-1H-pyrazole (39d):** Obtained as a white solid. ¹H (CDCl₃): 1.39 (d, 6H, J = 6.1); 2.08 (s, 3H); 3.97 (s, 3H); 4.95 (sept, 1H, J = 6.1); 7.05 (m, 2H); 7.39 (m, 2H). ¹³C (CDCl₃): 10.9; 22.2; 55.8; 72.1; 81.1; 112.2; 121.0; 128.7; 129.2; 130.0; 140.4; 154.7; 159.2. ¹⁵N (CDCl₃) : -192 (CH₃-5). HRMS: Calcd. for C₁₄H₁₇⁷⁹BrN₂O₂ + H : 325.0552. Found: m/z, 325.0564.

Isomers **41a** and **42a**: The reaction was run in dichloromethane; a chromatography over silica gel (cyclohexane - dichloromethane 3/7) led to 70 % of compound **41a** followed by 11 % of compound **42a** still containing traces (7 %) of **41a**. 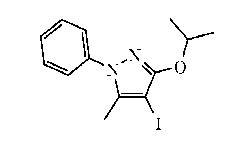 **4-iodo-3-isopropoxy-5-methyl-1-phenyl-1H-pyrazole (41a):** Obtained as an oil. ¹H (CDCl₃): 1.41 (d, 6H, J = 6.1); 2.34 (s, 3H); 4.97 (sept, 1H, J = 6.1); 7.39 (m, 5H). ¹³C (CDCl₃) : 13.5; 22.2; 50.8; 72.4; 126.6; 127.2; 129.1; 140.1; 141.3; 162.2. ¹⁵N (CDCl₃) : -182 (CH₃). HRMS: Calcd. for C₁₃H₁₅IN₂O + H : 343.0307. Found: m/z, 343.0323. 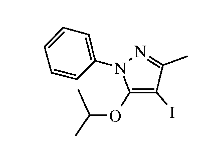 **4-iodo-5-isopropoxy-3-methyl-1-phenyl-1H-pyrazole (42a):** Obtained as an oil. ¹H (CDCl₃): 1.21 (d, 6H, J = 6.2); 2.29 (s, 3H); 4.53 (sept, 1H, J = 6.2); 7.31 (m, 1H); 7.43 (m, 2H); 7.65 (m, 2H). ¹³C (CDCl₃): 14.9; 22.3; 50.7; 78.6; 122.7; 126.8; 128.9; 138.7; 150.4; 152.6. ¹⁵N (CDCl₃): -90 (CH3); - 170 (Ar). HRMS: Calcd. for C₁₃H₁₅IN₂O + H : 343.0307. Found: m/z, 343.0321.

Isomers **41b** and **42b**: The reaction was run in dichloromethane; a chromatography over silica gel (cyclohexane - dichloromethane 7/3) led to 69 % of compound **41b** followed by 15 % of compound **42b**. 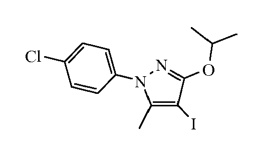 **4-iodo-1-(4-chlorophenyl)-3-isopropoxy-5-methyl-1H-pyrazole (41b):** Obtained as a solid. ¹H (CDCl₃): 1.41 (d, 6H, J = 6.2); 2.33 (s, 3H); 4.95 (sept, 1H, J = 6.2); 7.36 (m, 2H); 7.43 (m, 2H). ¹³C (CDCl₃): 13.6; 22.1; 51.4; 72.5; 125.5; 129.2; 132.8; 138.7; 141.4; 162.3. ¹⁵N (CDCl₃): -184 (CH₃ and Ar). HRMS: Calcd. for C₁₃H₁₄N₂OI³⁵Cl + H : 376.9918. Found: m/z, 376.9940. 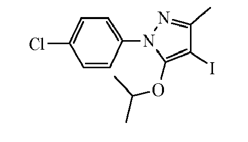 **4-iodo-1-(4-chlorophenyl)-5-isopropoxy-3-methyl-1H-pyrazole (42b):** Obtained as a solid. ¹H (CDCl₃): 1.22 (d, 6H, J = 6.2); 2.28 (s, 3H); 4.59 (sept, 1H, J = 6.2); 7.41 (m, 2H); 7.63 (m, 2H). ¹³C (CDCl₃): 14.9; 22.4; 50.7; 78.8; 123.9; 129.3; 132.2; 137.2; 150.8; 152.6. ¹⁵N (CDCl₃): -92 (CH₃) ; - 174 (Ar). HRMS: Calcd. for C₁₃H₁₄N₂OI³⁵Cl + H : 376.9918. Found: m/z, 376.9928.

Isomers **41c** and **42c**: The reaction was run in dichloromethane; a chromatography over silica gel (cyclohexane - dichloromethane 1/1) led to 63 % of compound **41c** and 13 % of compound **42c**. 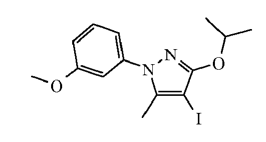 **4-iodo-1-(3-methoxyphenyl)-3-isopropoxy-5-methyl-1H-pyrazole (41c):** Obtained as an oil. ¹H (CDCl₃): 1.42 (d, 6H, J = 6.1); 2.35 (s, 3H); 3.84 (s, 3H); 4.99 (sept, 1H, J = 6.1); 6.88 (m, 1H); 6.98 (m, 2H); 7.34 (m, 1H). ¹³C (CDCl₃): 14.1; 22.6; 51.3; 55.9; 72.7; 109.4; 113.3; 117.1; 129.3; 141.5; 141.8; 160.5; 162.4. ¹⁵N (CDCl₃) : -190 (CH₃-5, Ar). HRMS: Calcd. for C₁₄H₁₇IN₂O₂ + H : 373.0413. Found: m/z, 373.0437. 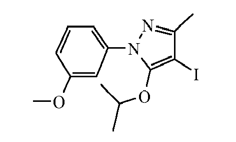 **4-iodo-1-(3-methoxyphenyl)-5-isopropoxy-3-methyl-1H-pyrazole (42c):** Obtained as an oil. ¹H (CDCl₃) : 1.22 (d, 6H, J = 6.1); 2.29 (s, 3H); 3.85 (s, 3H); 4.54 (sept, 1H, J = 6.1); 6.86 (m, 1H); 7.26 (m, 3H). ¹³C (CDCl₃) : 15.3; 22.7; 51.4; 55.8; 78.9; 108.4; 113.4; 115.2; 130.0; 140.1; 150.8; 153.0; 160.3. ¹⁵N (CDCl₃) : -95 (CH₃-5); -168 (Ar). HRMS: Calcd. for C₁₄H₁₇IN₂O₂ + H : 373.0413. Found: m/z, 373.0432.

Isomers **41d** and **42d**: The reaction was run in dichloromethane; a chromatography over silica gel (cyclohexane - dichloromethane 1/1) led to 25 % of compound **22d** and no compound **23d** could be isolated. 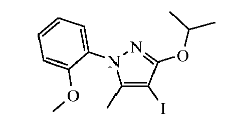 **4-iodo-1-(2-methoxyphenyl)-3-isopropoxy-5-methyl-1H-pyrazole (41d):** Obtained as a solid. ¹H (CDCl₃) : 1.39 (d, 6H, J = 6.2); 2.11 (s, 3H); 3.82 (s, 3H); 4.94 (sept, 1H, J = 6.2); 7.04 (m, 2H); 7.30 (m, 1H); 7.39 (m, 1H). ¹³C (CDCl₃): 12.4; 22.2; 48.5; 55.8; 72.1; 112.2; 120.9; 128.9; 129.2; 130.0; 143.5; 154.7; 162.1. ¹⁵N (CDCl₃) : -190 (CH₃-5). HRMS: Calcd. for C₁₄H₁₇IN₂O₂ + H : 373.0413. Found: m/z, 373.0434.

Isomers **43** and **44**: The reaction was run in dichloromethane; a chromatography over silica gel (cyclohexane - dichloromethane 4/1) led to 83 % of compound **44** followed by 2 % of compound **43**. 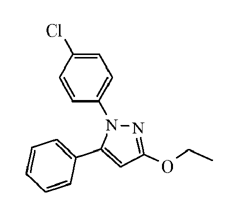 **1-(4-chlorophenyl)-3-ethoxy-5-phenyl-1H-pyrazole (43)**⁷**_{:}** Obtained as a colourless solid in a 2 % yield. ¹H (CDCl₃): 1.46 (t, 3H, J = 7.1); 4.32 (q, 2H, J = 7.1); 5.97 (s, 1H); 7.24 (m, 5H); 7.35 (m, 3H). ¹³C (CDCl₃): 14.9; 64.7; 94.3; 125.8; 128.6 (two signals); 128.7; 128.9; 130.5; 132.1; 138.7; 144.2; 163.7. ¹⁵N (CDCl₃) : -188 (H-4 and Ar). HRMS: Calcd. for C₁₇H₁₅³⁵ClN₂O + H : 299.0951. Found: m/z, 299.0969. 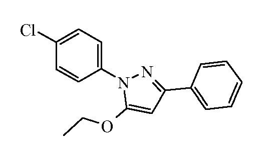 **1-(4-chlorophenyl)-5-ethoxy-3-phenyl-1H-pyrazole (44):** Obtained as a white solid in a 83 % yield. ¹H (CDCl₃): 1.51 (t, 3H, J = 7.1); 4.26 (q, 2H, J = 7.1); 6.01 (s, 1H); 7.37 (m, 1H); 7.42 (m, 4H); 7.83 (m, 4H). ¹³C (CDCl₃): 14.6; 68.2; 83.9; 122.9; 125.5; 128.2; 128.5; 128.9; 131.4; 133.3; 137.6; 150.8; 155.4. ¹⁵N (CDCl₃) : -184 (H-4 and Ar). HRMS: Calcd. for C₁₇H₁₅³⁵ClN₂O + H : 299.0951. Found: m/z, 299.0974. 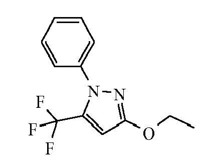 **3-ethoxy-1-phenyl-5-(trifluoromethyl)-1H-pyrazole (45):** Obtained as an oil in a 94 % yield after a chromatography over silica gel (cyclohexane - dichloromethane 7/3). ¹H (CDCl₃) : 1.48 (t, 3H, J = 7.1); 4.23 (q, 2H, J = 7.1); 5.94 (s, 1H); 7.37 (m, 1H) ; 7.45 (m, 2H); 7.73 (m, 2H). ¹³C (CDCl₃): 14.6; 68.7; 84.7; 121.0 (q, J = 260); 122.9; 127.5; 129.2; 138.0; 141.9 (q, J = 36); 154.9. HRMS: Calcd. for C₁₂H₁₁F₃N₂O + H : 257.0902. Found: m/z, 257.0928. 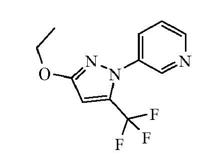 **3-(3-ethoxy-5-(trifluoromethyl)-1H-pyrazol-1-yl)pyridine (47):** Obtained as a yellow powder. ¹H (CDCl₃) : 1.53 (t, 3H, J = 7.0); 4.30 (q, 2H, J = 7.0); 5.99 (m, 1H); 7.44 (m, 1H); 8.11 (m, 1H); 8.62 (m, 1H); 9.1 (m, 1H). LC/MS: m/z = 258. 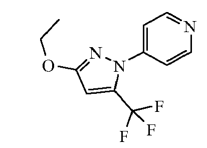 **4-(3-ethoxy-5-(trifluoromethyl)-1H-pyrazol-1-yl)pyridine (48):** For this compound, the following protocol was used: In a 5-10 mL Biotage tube, 3-ethoxy-5-trifluoropyrazole (0.3 g, 1.66 mmol), pyridine 4-boronic acid hydrate (0.22 g, 1.83 mmol), pyridine, copper acetate (0.498 g, 2.49 mmol) and 4Å molecular sieves (0.3 g) were dispersed in acetonitrile (6 mL, dried over 4Å molecular sieves). The tube was sealed heated at 120 °C for two hours. The content was adsorbed on a small amount of silica gel and purified by a chromatography over silica gel (cyclohexane / ethyl acetate 3/2) to yield the compound (0.1 g, 24 %) as a yellow powder. ¹H (CDCl₃): 1.53 (t, 3H, J = 7.0); 4.27 (q, 2H, J = 7.0); 5.92 (m, 2H); 8.05 (s(br), 4H). ¹³C (CDCl₃) : one signal missing two signal very weak and broad). 14.5; 67.0; 85.3; 121.5 (q, J = 270 Hz); 142.5 (q, J = 160); 144.2 (br); 150.4 (br); 156.0. LC/MS: m/z = 258. 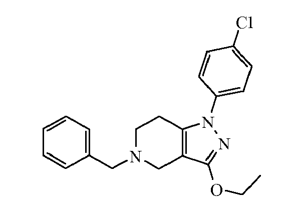 **5-benzyl-1-(4-chlorophenyl)-3-ethoxy-4,5,6,7-tetrahydro-1H-pyrazolo[4,3-c]pyridine (49):** Obtained either by the general protocol above or the protocol described just below as a yellow solid in 54 % or 63 % yield respectively after a chromatography over silica gel (dichlorométhane - éthanol 98/ 2). ¹H (CDCl₃) : 1.40 (t, 3H, J = 7.0); 2.76 (m, 2H); 2.85 (m, 2H); 3.46 (s, 2H); 3.76 (s, 2H); 4.32 (q, 2H, J = 7.0); 7.3-7.43 (m, 9H). ¹³C (CDCl₃): 14.8; 25.1; 47.9; 49.7; 62.1; 64.3; 103.4; 122.4; 127.2; 128.3; 129.0; 129.1; 130.6; 138.1; 138.3; 138.7; 159.9. HRMS: Calc for C₂₁H₂₂N₃O³⁵Cl + H: 368.1530. Exp: 368.1511.

### C-General procedure for the N-arylation of 3-alkoxypyrazoles using halogenoaryl.

In a 10-20 mL Biotage tube the 3-alkoxypyrazole (2.1 mmol), the halogeno aryl (2.3 mmol), caesium carbonate (3.24 mmol), 4Å molecular sieves (0.2 g) and salicyloxime (0.21 mmol) or [*N*,*N*'-bis-((2'-pyridine)-methylene)]-1,2-diaminocyclohexane (0.21 mmol) were dispersed in acetonitrile (15 mL, dried over 4Å molecular sieves). This was degassed using a slow stream of argon bubbling in the suspension. Copper oxide (0.11 mmol) was then added the tube was sealed and heating in a Biotage microwave oven at 180 °C for 3 hours. The resulting suspension was concentrated to dryness, adsorbed over a small amount of the relevant solid phase and purified by a chromatography on that phase as described below.

Isomers **50a** and **51a:** these isomers were obtained as oils after two chromatography (silica gel; dichloromethane / ethanol 99/1 and then neutral alumina containing 1.5 % H₂O (cyclohexane / dichloromethane 4/1 then 1/0) 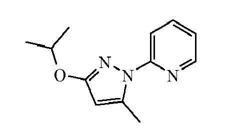 **2-(3-isopropoxy-5-methyl-1H-pyrazol-1-yl)pyridine (50a).** ¹H (CDCl₃): 1.40 (d, 6H, J = 6.1); 2.67 (s, 3H); 4.83 (sept, 1H, J = 6.1); 5.66 (s, 1H); 7.07 (m, 1H); 7.73 (m, 1H); 7.82 (m, 1H); 8.37 (m, 1H). ¹³C (CDCl₃): 15.1; 22.1; 71.4; 96.1; 114.8; ;119.7; 137.9; 142.5; 147.1; 153.8; 162.6. HRMS: Calc. for C₁₂H₁₅N₃O + H: 218.1293. Exp.: 218.1246 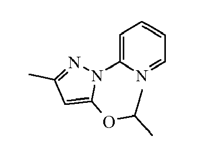 **2-(5-isopropoxy-3-methyl-1H-pyrazol-1-yl)pyridine (51a)** Obtained as an oil after two chromatography (silica gel; dichloromethane / ethanol 99/1 and then neutral alumina + 1.5 % H₂O ; Cyclohexane / dichloromethane 4/1 and 1/0). ¹H (CDCl₃): 1.42 (d, 6H, J = 6.1); 2.30 (s, 3H); 4.48 (sept, 1H, J = 6.1); 5.49 (s, 1H); 7.15 (m, 1H); 7.66 (m, 1H); 7.73 (m, 1H); 8.54 (m, 1H). ¹³C (CDCl₃): 14.6; 21.8; 71.4; 87.8; 115.8; ;120.8; 137.6; 150.0; 148.8; 150.8; 154.5. HRMS: Calc. for C₁₂H₁₅N₃O + H: 218.1293. Exp.: 218.1328

Isomers **50b** and **51b:** these isomers were obtained as oils after two chromatography (neutral alumina + 1.5 % H₂O; cyclohexane/dichloromethane 3/2 and then silica gel; dichloromethane/ethanol 99/1 to 98/2) 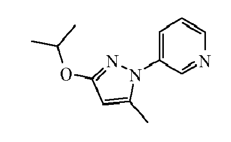 **3-(3-isopropoxy-5-methyl-1H-pyrazol-1-yl)pyridine (50b):** ¹H (CDCl₃): 1.38 (d, 6H, J = 6.1); 2.35 (s, 3H); 4.83 (sept, 1H, J = 6.1); 5.70 (s, 1H); 7.39 (m, 1H); 7.83 (m, 1H); 8.55 (m, 1H) ; 8.76 (m, 1H). ¹³C (CDCl₃): 13.0; 22.1; 71.5; 95.0; 123.6; ;131.2; 136.7; 140.5; 144.7; 147.2; 163.3. HRMS: Calc. for C₁₂H₁₅N₃O + H: 218.1293. Exp: 218.1215. 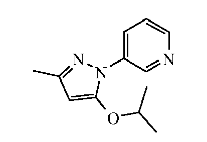 **3-(5-isopropoxy-3-methyl-1H-pyrazol-1-yl)pyridine (51b):** ¹H (CDCl₃): 1.42 (d, 6H, J = 6.1); 2.28 (s, 3H); 4.48 (sept, 1H, J = 6.1); 5.49 (s, 1H); 7.36 (m, 1H); 8.08 (m, 1H); 8.47 (m, 1H) ; 9.07 (m, 1H). ¹³C (CDCl₃): 14.6; 21.9; 76.1; 87.1; 123.5; ;128.6; 135.8; 142.5; 146.1; 150.1; 154.4. HRMS: Calc. for C₁₂H₁₅N₃O + H: 218.1293. Exp: 218.1212.

Isomers **50c** and **51c:** these isomers were obtained as oils after two chromatography (silica gel; dichloromethane / ethanol 98/2 and then neutral alumina + 1.5 % H₂O; cyclohexane / dichloromethane 1/2) 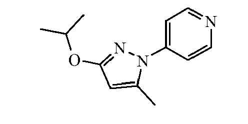 **4-(3-isopropoxy-5-methyl-1H-pyrazol-1-yl)pyridine (50c).** ¹H (CDCl₃): 1.40 (d, 6H, J = 6.1); 2.48 (s, 3H); 4.85 (sept, 1H, J = 6.1); 5.73 (s, 1H); 7.49 (m, 2H); 8.63 (s(br), 2H). ¹³C (CDCl₃): 14.1; 28.1; 71.6; 97.1; 115.9; 140.9; 146.7; 150.6; 163.2. HRMS: Calc. for C₁₂H₁₅N₃O + H: 218.1293. Exp.: 218.1230 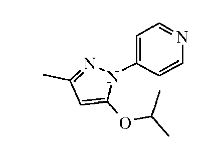 **4-(5-isopropoxy-3-methyl-1H-pyrazol-1-yl)pyridine (51c).** ¹H (CDCl₃): 1.46 (d, 6H, J = 6.1); 2.27 (s, 3H); 4.50 (sept, 1H, J = 6.1); 5.48 (s, 1H); 7.79 (m, 2H); 8.58 (s(br), 2H). ¹³C (CDCl₃): 11.6; 21.9; 76.3; 87.2; 113.8; 145.5; 150.5; 150.6; 155.3. HRMS: Calc. for C₁₂H₁₅N₃O + H: 218.1293. Exp.: 218.1200. 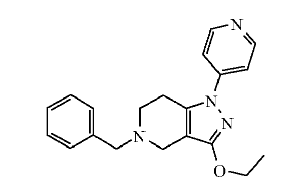 **5-benzyl-3-ethoxy-1-(pyridin-4-yl)-4,5,6,7-tetrahydro-1H-pyrazolo[4,3-c]pyridine (52):** Obtained as a solid. ¹H (CDCl₃): 1.43 (t, 3H, J = 7.0); 2.80 (m, 2H); 3.01 (m, 2H); 3.44 (s, 2H); 3.76 (s, 2H); 4.32 (q, 2H, J = 7.0);7.34 (m, 5H); 7.48 (s (1), 2H); 8.59 (s (1), 2H). ¹³C (CDCl₃) : 14.8; 26.0; 47.7; 49.7; 62.0; 64.5; 106.2; 113.5; 127.3; 128.4; 129.0; 138.0; 139.0; 146.7; 150.4; 160.8. HRMS: Calc. for C₂₀H₂₂N₄O + H: 335.1872. Exp: 335.1891. 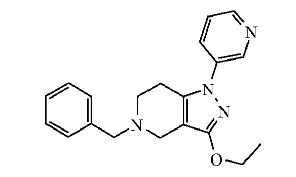 **5-benzyl-3-ethoxy-1-(pyridin-3-yl)-4,5,6,7-tetrahydro-1H-pyrazolo[4,3-c]pyridine (53):** Obtained as a solid. ¹H (CDCl₃): 1.43 (t, 3H, J = 7.0); 2.79 (m, 2H); 2.88 (m, 2H); 3.46 (s, 2H); 3.76 (s, 2H); 4.33 (q, 2H, J = 7.0); 7.34 (m, 6H); 7.84 (m, 1H); 8.46 (m, 1H); 8.79 (m, 1H). ¹³C (CDCl₃): 14.8; 25.0; 47.8; 49.7; 62.1; 64.4; 104.2; 123.6; 127.2; 128.1; 128.4; 129.0; 136.8; 138.3; 138.5; 142.3; 146.1; 160.5. HRMS: Calc. for C₂₀H₂₂N₄O + H: 335.1872. Exp: 335.1887. 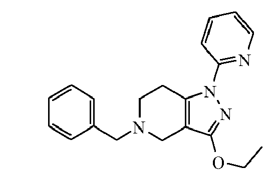 **5-benzyl-3-ethoxy-1-(pyridin-2-yl)-4,5,6,7-tetrahydro-1H-pyrazolo[4,3-c]pyridine (54):** Obtained as white crystals. ¹H (CDCl₃): 1.43 (t, 3H, J = 7.0); 2.79 (m, 2H); 2.88 (m, 2H) ; 3.46 (s, 2H); 3.76 (s, 2H); 4.33 (q, 2H, J = 7.0); 7.34 (m, 6H); 7.84 (m, 1H); 8.46 (m, 1H); 8.79 (m, 1H). ¹³C (CDCl₃): 14.8; 26.7; 47.8; 50.0; 62.0; 64.3; 104.5; 112.9; 119.1; 127.2; 128.3; 129.0; 138.0; 138.4; 140.1; 147.4; 153.6; 160.2. HRMS: Calc. for C₂₀H₂₂N₄O + H: 335.1872. Exp.: 335.1870.

### D- N1-alkylation of 3-alkoxypyrazoles.

### Methylation

Compound **10c** or **29** (4.2 mmol) was dissolved in dimethylformamide (10 mL, dried over 4Å molecular sieves). To this solution was added sodium hydride (60 % in mineral oil, 4.6 mmol) and at the end of the hydrogen evolution methyl iodide (4.6 mmol). The solution was stirred overnight at room temperature in a flask protected from moisture by a calcium chloride guard. It was then concentrated to dryness, the residue was absorbed on a small amount of silica gel and purified as described below. 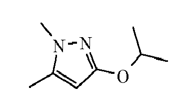 **1,5-dimethyl-3-isopropoxy-1H-pyrazole 55a:** Obtained in a 19 % yield after two consecutive chromatography over silica gel (dichloromethane and then dichloromethane - cyclohexane 7/3) as a volatile oil. *Note:* this compound is detected by the UV monitor but not by TLC, its isomer was not seen by the UV monitor although it was visible on as a minor component on ¹H NMR spectra of the crude mixture. ¹H (CDCl₃): 1.33 (d, 6H, J = 6.2); 2.19 (s, 3H); 3.62 (s, 3H); 4.66 (sept, 1H, J = 6.2); 5.41 (s, 1H). ¹³C (CDCl₃): 14.4; 22.2; 35.4; 71.3; 90.6; 139.5; 161.2. ¹⁵N (CDCl₃): -108 (CH₃,); -200 (CH₃, H-4, N-CH₃). HRMS: Calcd. for C₈H₁₄N₂O + H : 155.1184. Found: m/z, 155.1186. 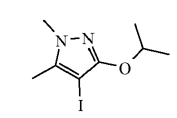 **1,5-dimethyl-4-iodo-3-isopropoxy-1H-pyrazole 55b:** Obtained in a 56 % yield as a solid still containing 2.5 % of **56b** after a chromatography over silica gel (dichloromethane - cyclohexane 1/1). ¹H (CDCl₃): 1.36 (d, 6H, J = 6.1); 2.24 (s, 3H); 3.71 (s, 3H); 4.81 (sept, 1H, J = 6.1). ¹³C (CDCl₃): 11.9; 22.2; 36.9; 46.8; 72.4; 141.1; 160.9. ¹⁵N (CDCl₃): -108 (CH₃, N-CH₃) ; -197 (N-CH₃). HRMS: Calcd. for C₁₈H₁₃IN₂O + H : 281.0151. Found: m/z, 281.0170. 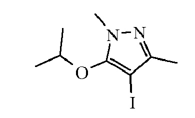 **1,3-dimethyl-4-iodo-5-isopropoxy-1H-pyrazole 56b:** Obtained in a 1.8 % yield as an oil still containing 10 % of **55b** in a second fraction of the chromatography described above. Again contrary to its isomers, this compound is barely detected by the UV monitor. ¹H (CDCl₃): 1.36 (d, 6H, J = 6.1); 2.19 (s, 3H); 3.65 (s, 3H); 4.74 (sept, 1H, J = 6.1). ¹³C (CDCl₃) : 11.9; 22.5; 34.5; 46.1; 77.8; 149.0; 152.7. ¹⁵N (CDCl₃): -90 (CH₃, N-CH₃) ; -190 (N-CH₃). HRMS: Calcd. for C₁₈H₁₃IN₂O + H : 281.0151. Found: m/z, 281.0188.

### Benzylation

Compound **10c** (0.3 g, 2.14 mmol) was dissolved in dimethylformamide (25 mL, dried over 4Å molecular sieves). To this solution was added sodium hydride (60 % in mineral oil, 0.062 g, 2.57 mmol) and at the end of the hydrogen evolution benzyl bromide (0.305 mL, 2.57 mmol). The solution was stirred overnight at room temperature in a flask protected from moisture by a calcium chloride guard. It was then concentrated to dryness, the residue was absorbed on a small amount of silica and purified by a chromatography over silica gel (cyclohexane - dichloromethane 95/5 to 0/100) to yield in order of elution, the N-1 benzylation product **57** (0.280 g, 57 %, still containing 8 % of **20**) and then its isomer **20** (0.16 g, 32 %, still containing 5 % of **57**) as described above. 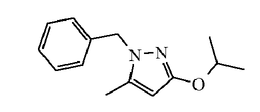 **1-benzyl-3-isopropoxy-5-methyl-1H-pyrazole (57):** ¹H (CDCl₃): 1.35 (d, 6H, J = 6.2); 2.10 (s, 3H); 4.73 (sept, 1H, J = 6.2); 5.10 (s, 2H); 5.49 (s, 1H); 7.11 (m, 2H); 7.24 (m, 1H); 7.29 (m, 2H). ¹³C (CDCl₃): 11.3; 22.2; 52.3; 71.2; 91.7; 126.6; 127.3; 128.6; 137.7; 139.7; 161.5. ¹⁵N (CDCl₃): -109 (CH₂); -191 (CH₃, CH₂, H-4). HRMS: Calcd. for C₁₄H₁₈N₂O + H : 231.1497. Found: m/z, 231.1540. 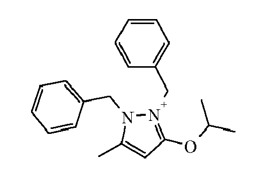 **1,2-dibenzyl-3-ethoxy-5-methyl-1H-pyrazol-2-ium (58):** In a biotage 10 ml microwave-adapted vial, compound **10c** (0.4 g, 2.85 mmol) was dissolved in acetonitrile (5 mL, dried over 4Å molecular sieves). Potassium carbonate (0.79 g, 5.7 mmol) and benzylbromide (0.68 mL, 5.7 mmol) were added and the vial was sealed. This was submitted to a microwave-based heating (140 °C for 7 mn) and the resulting suspension was diluted in dichloromethane, washed with brine, dried over sodium sulfate and concentrated to dryness. The resulting residue was purified by a chromatography over silica gel (dichloromethane - ethanol 9/1) to yield compound **58** (0.37 g, 53 %) as an oil. ¹H (CDCl₃): 1.47 (d, 6H, J = 6.1); 2.53 (s, 3H); 5.01 (sept, 1H, J = 6.1); 5.52 (s, 2H); 5.73 (s, 2H); 6.64 (s, 1H); 6.8 (m, 3H); 7.05 (m, 2H); 7.16 (m, 6H). ¹³C (CDCl₃) : (one signal overlapped); 13.6; 22.3; 49.3; 51.5; 79.9; 95.9; 126.1; 127.4; 128.9; 129.2; 129.5; 129.6; 132.5; 150.2; 157.1. ¹⁵N (CDCl₃) : -195 (2 CH₂, H-4 and CH₃).Calcd. for C₂₁H₂₅N₂O: 321.1967. Found: m/z, 321.1986.

### Results

The room temperature N-arylation of the model compound **10c** using arylboronic acid **32a-d** and copper salts²¹⁻²⁸ was studied. This turned out to be reliably reproductive and free of the necessity of the long heating time inherent to other N-arylation methods involving halogenated aryls and copper catalysts.²⁹⁻⁴¹ Interestingly, the inventors could not find a report describing a N-arylation of pyrazoles using a palladium-based catalyst as used for other NH-bearing heterocycles.⁴² Dichloromethane was first used as solvent and as long as the reaction was left in open air, N-arylation reactions were obtained in usually 48 hours. However, in order to either minimize the evaporation taking place in the course of the reaction or improve the solubility of the arylboronates used acetonitrile was also used. Few interesting results came out of this change and are further described below. The main point found out is that water is detrimental to this reaction. Thus it is necessary to use reasonably dry solvents and to add some molecular sieves if the copper salt added is hydrated. As stochiometric amount of copper salts was used, this did not seem to be necessary on a small scale as some N-arylation reactions proceeded without molecular sieves. However, on a gram scale the 4Å molecular sieves was essential in achieving a good transformation yield. Accordingly, the use of the hydrophobic dichloromethane allowed running the reaction without any particular precautions. On the other hand, the use of the hygroscopic acetonitrile required a calcium guard which has the potential of lessening a proper oxygenation of the reaction media in reaction run at room temperature.

As depicted in scheme 7, the arylation of **10c** or **10m** with the aryl boronates **32a-d** led to the separable pairs of isomers **33a-d** / **34a-d** and **35b/36b**. The N1/N2 arylation ratio of compound **6** and **10**, pointed out a tendency in favour of the N2-arylation product. Moreover, an improvement in the reaction yield was seen in the case of the preparation of compound **33c** and **34c** when we switched from dichloromethane (30 and less than 10 % yield) to boiling acetonitrile (37 and 47 % yield respectively). This effect was not seen in the case of the pair of compound **33d** and **34d** although the very low UV absorption of compound **34d** (either on TLC or with a liquid chromatography UV detector) probably lessens its isolated yield. The N-arylation of compound **10m** leads to the allyl-bearing compounds **35b** and **36b**.

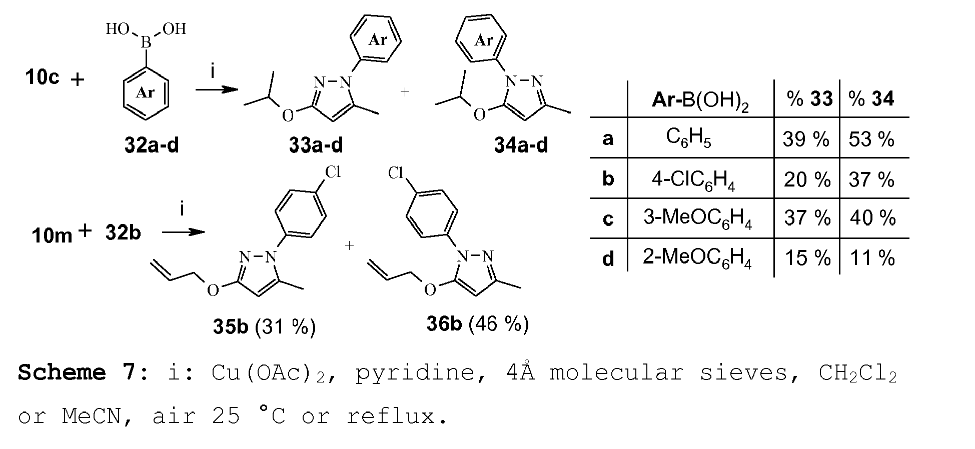

As shown in scheme 8, the arylation of the 4-benzyl derivative **10f** in dichloromethane gave a far more important **37/38** ratio in favour of the N-1 arylation products **37a-c**. For instance compound **37a** or **37c** were obtained in at least 74 % yield along with only traces of compound **19** or **38c**. On a bigger scale, less than 10% of the N-2 arylation isomer **38b** could be properly isolated. These results first underlie the influence of a substituent on carbon 4 of the pyrazole in governing the ratio of N1 or N2 arylation of 3-alkoxypyrazoles along with a somewhat smaller effect of the arylboronate considered. The ¹H-¹⁵N long distance correlation experiments on compound **37a** were of small help in its structural assignment (only one ¹⁵N NMR signal is visible). Accordingly, the corresponding minor isomer **19** was prepared unambiguously in a 37 % yield from ethyl 2-benzyl-3-oxobutanoate and phenylhydrazine hydrochloride, using the process of Example 1 as described above.

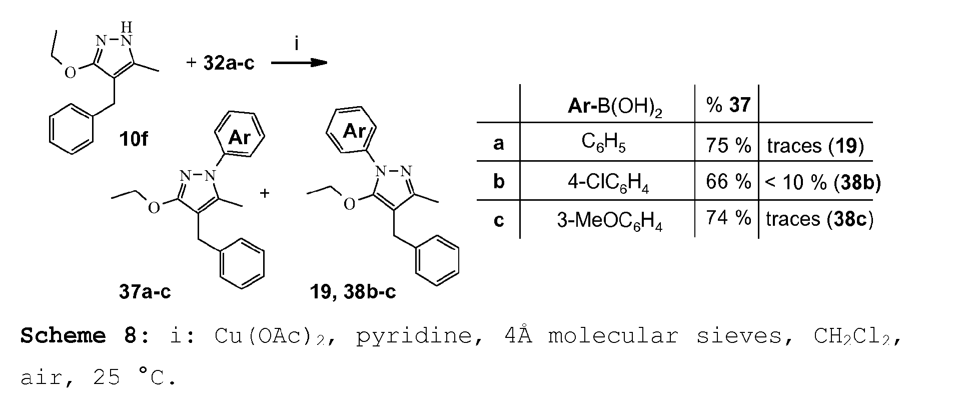

From compound **10c,** good to excellent yield of the 4-bromo or the 4-iodo derivatives **28** and **29** were obtained using simple halogenation methods (see scheme 7). The occurrence of so far unidentified side products in the course of the preparation of compound **28** forced us to purify it by chromatography over silica gel. On the other hand, the preparation of the iodinated derivative **29** only required a filtration. The N-arylation of compounds **28** or **29**, turned out to lead to a large proportion of N-1 arylation products whatever the solvent used (dichloromethane or acetonitrile). Again, yield from 2-methoxyphenylboronic acid were low and the N-2 arylation products corresponding to compound **40d** and **42d** was overlooked because of their lack of UV absorption properties as compound **34d**. As an alternative path the iodination of the N1-phenylated product **41a** also was undertook. Thus, using stronger iodination conditions,¹⁴ the 4-iodo derivative **41a** could be prepared from the N-phenyl compound **33a** in a 90 % yield. This experiment further establishes the structure asignement of compound **41a**.

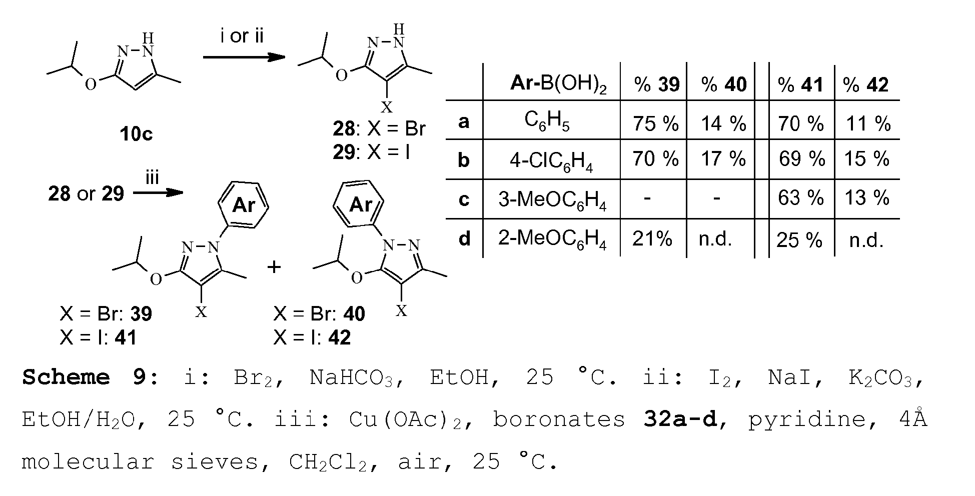

AS shown in scheme 10, further insights on the reactivity of these 3-alkoxypyrazole were obtained as the arylboronate-based arylation of the 5-phenyl-bearing compound **10f** led to 2 % of, for once, the least migrating N1-arylation product **43** along with 83 % of the N2-arylation product **44** (scheme 10). In the present case, the structural assignment of **44** had to be made, via the process in Example 4, i.e.: the acid cleavage of its ethoxy moiety. This gave the corresponding diaryl-1H-pyrazol-5(4H)-one **94** featuring the unambiguous methylene ¹H and ¹³C NMR signals.

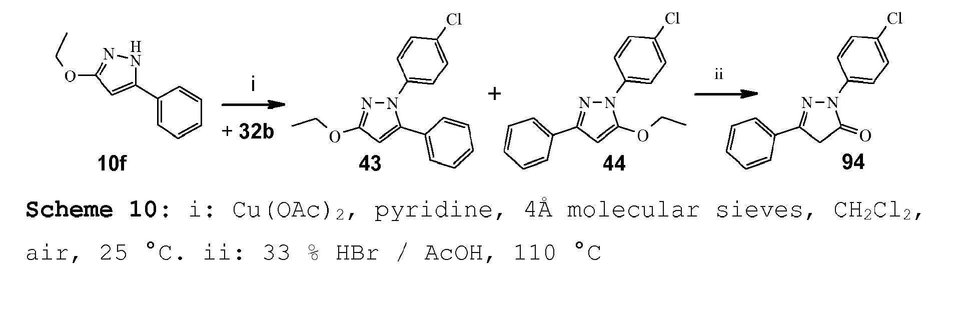

The arylation of the trifluoromethyl-bearing substrate **101** led in a 94 % yield to the N1-arylation product **49** (scheme 11). None of the alternative isomer was detected in the reaction mixture. Its structural assignment was made after the acid-assisted cleavage of the ethoxy moiety, as described in Example 4, and comparison of the analytical data of the resulting pyrazolone **95** which differ from the previously described isomer.⁴³

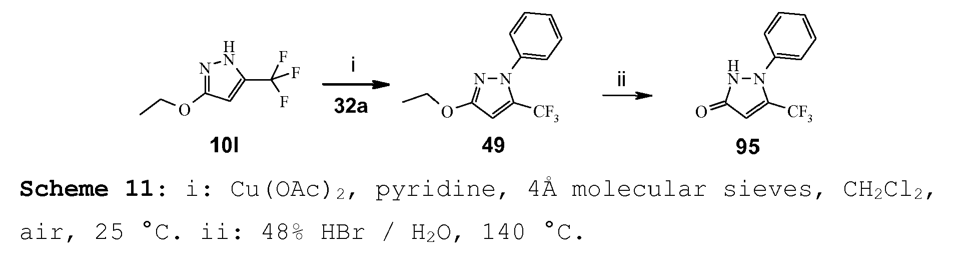

The ¹H NMR monitoring of the methylation of compound **10c,** or its iodinated derivative **29**, using sodium hydride in DMF and methyl iodide showed respectively a 10/3 or 17/3 ratio in favour of the N1-methylation product of compound **10c** or **29** (scheme 12). This ratio improvement actually follows what has been observed for the N-arylation reactions ratio of compound **10c** or **29** described above. The notable lack of UV-absorbance of the N2-methylation products **11** or **56b** made them fairly hard to isolate. Moreover the volatility of the methylation products **55a** probably explains the low isolated yield obtained. The structure assignment of these methylation products could not be achieved with the ¹⁵N-¹H correlation data as both nitrogens correlates with the methyl residues. On the other hand, 13C-1H long distance correlations allowed unequivocal structural assignments. The N-methyl residues of **55a** or **11** (see the Example 1 part) respectively correlate with the carbon bearing the methyl side chain or the one bearing the isopropyloxy side chain. The same observation was made for the pair of compounds **55a-56b**. Benzylation of **10c** also proceeded with a preference for the N1-benzylation product **57** which was obtained in a 54 % yield. As described in the experimental part, the structure assignment of compounds **57** and **20** were further established by the synthesis of **20** from isopropylacetoacetate and benzylhydrazine hydrochloride as described in the Example 1 part. Moreover, substantial amount (53 %) of the pyrazolium **58** could also be prepared when heating the benzylation reaction in a microwave-reactor.

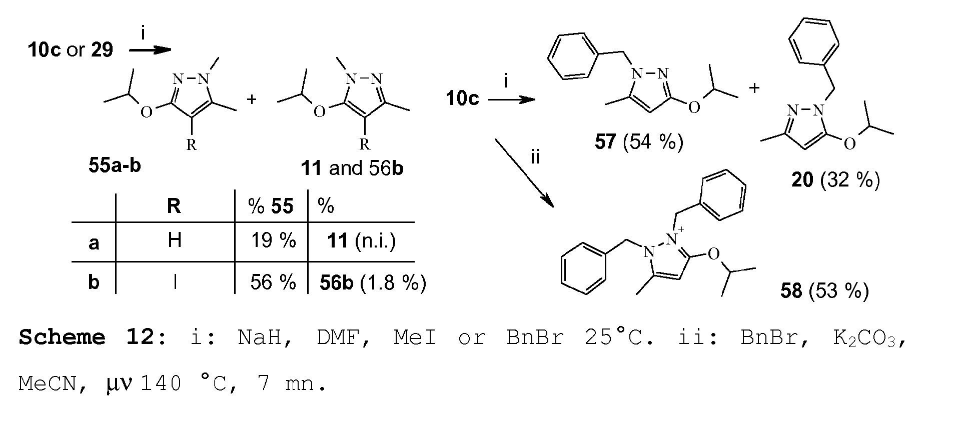

As shown in scheme 13, the 3-pyridyl derivative **47** was obtained from the condensation of **101** and pyridine-3-boronic acid 1,3-propanediol ester. More interestingly, as there are no precedents in the litterature describing the use of 4-pyridylboronic acid for this reaction, subtantial amount (24 %) of the 4-pyridyl derivative **48** was also obtained from **101** by using a micro-wave oven. This method of heating greatly shorten the otherwise very long reaction time required (at least 7 days).

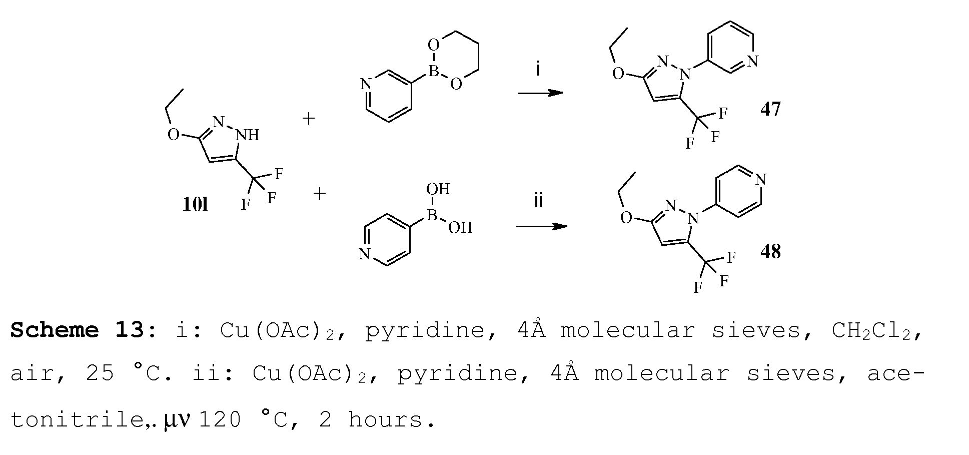

The synthesis of N-arylated pyrazoles was also undertook using the improved Ullman-based method involving halogenated aryl and copper catalysts with various ligands. ^{35,36} Interestingly, compound **49** could be prepared in similar yield from **16** by using either 4-chlorophenylboronate or 4-chlorophenyliodide (scheme 14). Indeed, either 54 % or 63 % of compound **49** were obtained along with only traces of the other isomer **18**. Structural assignment of compound **49** was made by comparison of its spectra caracteristic with the analytical data of compound **18** unambiguously prepared by the method described in the Example 1 part.

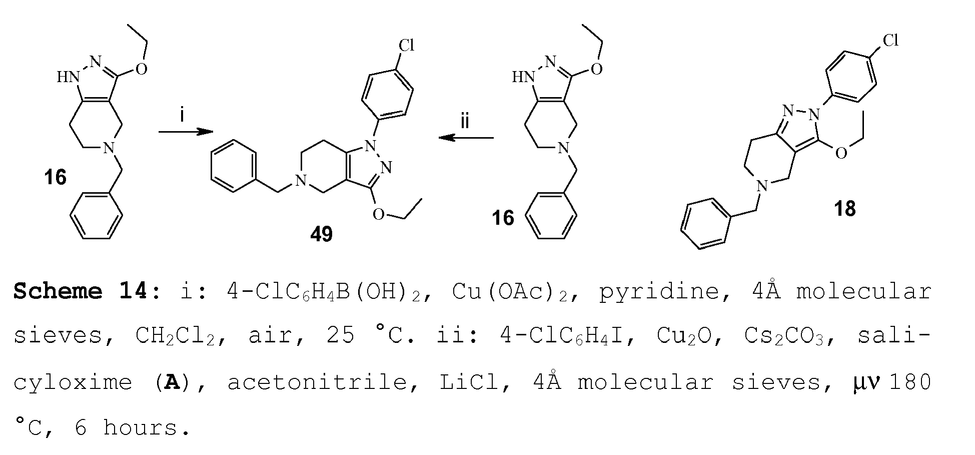

This Ullmann-based method turned out to require fairly long and high heating times even when using a microwave oven. Moreover, 4Å molecular sieves turned out to be essential for these reactions to proceed and, for the synthesis of **49**, lithium chloride had to be added in the reaction medium too. The preparation of the N-pyridyl derivatives **50a-c** and **51a-c** was also undertaken using the Ullmann-improved approaches^{37, 36, 35} using bromo-pyridines and either salicyloxime (**A**) or [*N*,*N*'-bis-((2'-pyridine)-methylene)]-1,2-diaminocyclohexane (**B**) as the copper salt ligand. Much improved reaction yields were usually observed when using the latter as an unwanted hydroxyl arylation of ligand **A** leading to the pyridyl ethers of 2-cyanophenol, much lessened the efficacy of the N-arylation reaction.

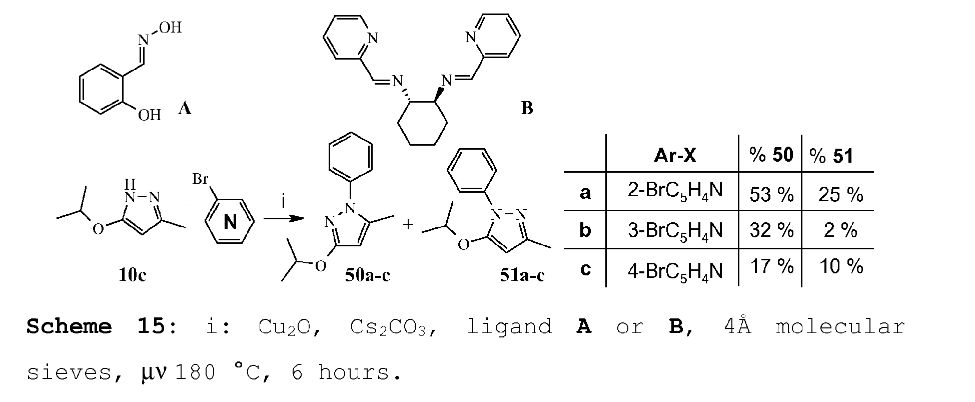

The structure assignments of compounds **50a-c** and **51a-c** were made by their isopropyl hydrolysis and examination of the spectral characteristics of the reaction products **81-86** as described below in the Example 4 part.

We also undertook the synthesis of the N-pyridyl derivatives **52-54** by N-arylation of compound **16**. The results are summed-up in scheme 16. The use of 4Å molecular sieves (M.S. 4A) was essential again. Moreover, replacing ligand **A** by ligand **B** is likely to improve the reaction yields. In theses cases, the addition of lithium chloride was detrimental to the reaction.

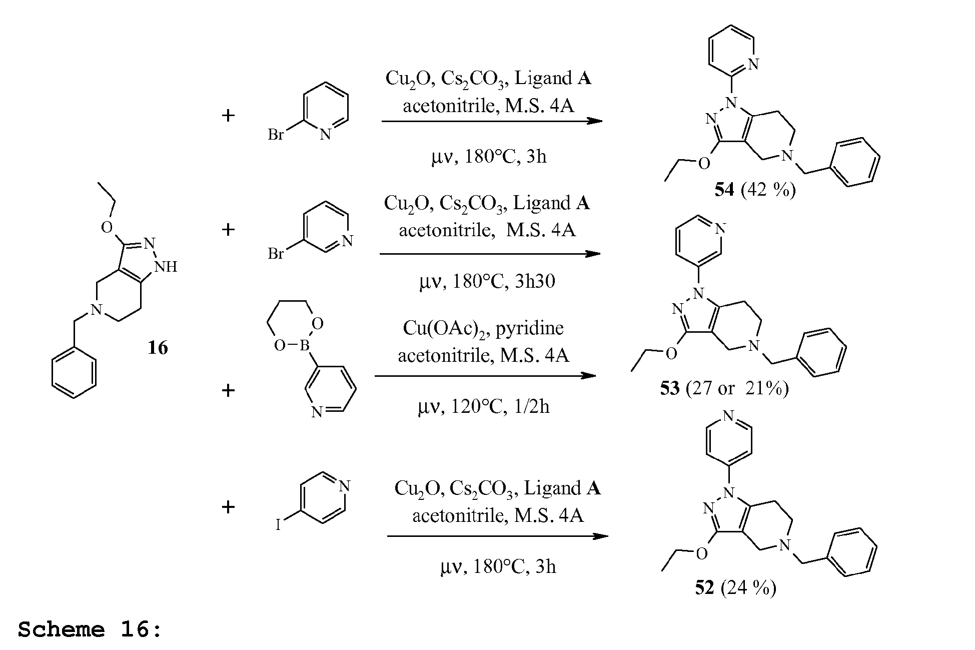

### Example 3: carbon-carbon bond formations on position C4 of 3-alkoxypyrazoles

The features of the devices used to conduct analyses of all of the compounds described in Example 3 are indicated hereinbelow:

A Biotage initiator 2 microwave oven was used for of the reactions requiring microwaves irradiations. The ¹H NMR and ¹³C NMR spectra were recorded on a Bruker Avance 400 spectrometers at 400 MHz and 100 MHz, respectively. Unless otherwise noted, CDCl₃ was the solvent used. Shifts (δ) are given in ppm with respect to the TMS signal and coupling constants (J) are given in Hertz. Column chromatography were performed either with Merck silica gel 60 (0.035 - 0.070 mm) or neutral alumina containing a suitable proportion of water, using a solvent pump operating at pressure between 2 and 7 bar (25-50 mL/mn) and an automated collecting system driven by a UV detector set to 254 nm unless stated otherwise (i.e. if ethyl acetate was used then it would be set to 280 nm). Sample deposition was always carried out by absorption of the mixture to be purified on a small amount of the solid phase followed by its deposition of the top of the column. The low resolution mass spectra were obtained on an Agilent 1100 serie LC/MSD system using an atmospheric electrospray ionisation system and the high resolution mass spectroscopy spectra (HRMS) were obtained using a Waters Micromass Q-Tof with an electrospray ion source.

### A-transient N-protection of some 4-halogenopyrazoles

Prior to these studies, the N-protection of 4-iodopyrazoles **29** and **30** was undertaken using a methanesulfonyl group. This led to the pairs of compounds **59a-b and 60a-b** as described below. 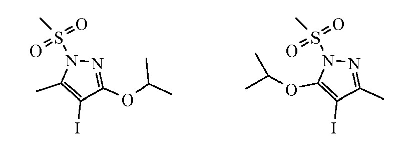 **4-iodo-5-isopropoxy-3-methyl-1-(methylsulfonyl)-1H-pyrazole** and **4-iodo-3-isopropoxy-5-methyl-1-(methylsulfonyl)-1H-pyrazole (59a-b):** Compound **29**(2.31 g, 8.68 mmol) was dissolved in ethyl acetate (90 mL), triethylamine (1.9 mL, 13.8 mmol; dried over 4Å molecular sieves) was added followed by mesylchloride (1 mL, 13.0 mmol). The solution was protected from moisture by a calcium chloride guard and stirred for two hours at room temperature. This was washed with 2 N potassium carbonate, 0.5 N hydrochloric acid and brine, dried over sodium sulfate and concentrated to dryness to yield the 1/4 mixture of compounds **59a-b** as a dark oil (2.84 g, 95 %). ¹H (CDCl₃) : 1.40 (d, 4/5 of 6H, J = 6.1); 1.42 (d, 1/5 of 6H, J = 6.1); 2.28 (s, 1/5 of 3H); 2.54 (s, 4/5 of 3H); 3.23 (s, 4/5 of 3H); 3.24 (s, 1/5 of 3H); 5.02 (m, 1H). 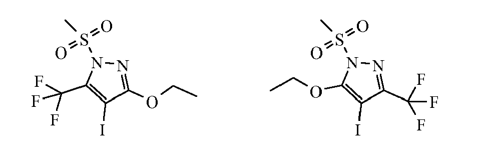 **4-iodo-3-ethoxy-1-(methylsulfonyl)-5-(trifluoromethyl)-1H-pyrazole** and **4-iodo-5-ethoxy-1-(methylsulfonyl)-3-(trifluoromethyl)-1H-pyrazole (60a-b):** From compound **30**, the same protocol described above led to a 7/5 mixture of compounds **60a-b** as a colorless oil. ¹H (CDCl₃): 1.44 (t, 5/12 of 3H, J = 7.1); 1.53 (t, 7/12 of 3H, J = 7.1); 3.03 (s, 5/12 of 3H); 3.42 (s, 7/12 of 3H); 4.33 (q, 5/12 of 3H, J = 7.1); 4.46 (q, 7/12 of 3H, J = 7.1).

### B-Suzuki-based aryl-aryl coupling methods

**Palladium-catalysed C-arylation of compound 29 preparation of 61a and 61b.** In biotage 5 mL tube a mixture of pyrazole **29** (1.1 mmol), the relevant boronic acid (1.45 mmol), cesium carbonate (2.8 mmol), lithium chloride (2.24 mmol) in dioxane (5 mL) and water (5 mL) were degassed with a slow stream of argon for ten minutes. Following this, [1,1'-bis(diphenylphosphino)ferrocene] dichloropalladium complexed with dichloromethane (0.044 mmol) was added, the tube was sealed and heated at 120 °C for 30 minutes in the microwave oven. After cooling to room temperature, the reaction mixture was diluted in water and extracted with ethyl acetate. The organic phase was dried over sodium sulfate and concentrated to dryness. The residue was purified by chromatography on silica gel as described below. 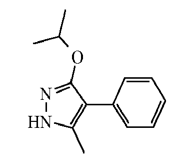 **3-isopropoxy-5-methyl-4-phenyl-1H-pyrazole (61a):** Obtained as an oil that slowly solidified in a 50 % yield after a chromatography over silica gel (cyclohexane - dichloromethane 2/8). ¹H (CDCl₃): 1.47 (d, 6H, J = 6.1); 2.38 (s, 3H); 5.00 (sept, 1H, J = 6.1); 7.30 (m, 1H); 7.44 (m, 2H); 7.57 (m, 2H). ¹³C (CDCl₃): 11.9; 22.8; 72.2; 105.4; 126.0; 128.6; 128.7; 133.1; 138.1; 160.5. HRMS: Calcd. for C₁₃H₁₆N₂O + H : 217.1341. Found: m/z, 217.1348. 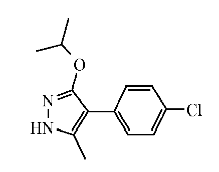 **4-(4-chlorophenyl)-3-isopropoxy-5-methyl-1H-pyrazole (61b):** Obtained as a solid in a 59 % yield after a chromatography over silica gel (dichloromethane - ethanol 99/1). ¹H (CDCl₃) : 1.27 (d, 6H, J = 6.1); 2.20 (s, 3H); 4.79 (sept, 1H, J = 6.1); 7.23 (m, 2H); 7.29 (m, 2H); 9.0 (s(br), 1H). ¹³C (CDCl₃): 11.7; 22.4; 71.9; 104.1; 128.5; 129.3; 131.1; 131.3; 137.6; 160.1. HRMS: Calcd. for C₁₃H₁₅³⁵ClN₂O + H : 251.0951. Found: m/z, 251.0978.

**Palladium-catalysed C-arylation of the mixture of compounds 59a-b, preparation of 61c-e:** in biotage 10-20 mL tube a mixture of compounds **59a-b** (1.1 mmol), the relevant boronic acid (1.45 mmol), cesium carbonate (2.8 mmol), lithium chloride (2.24 mmol) in propanol (5 mL) and water (5 mL) were degassed with a slow stream of argon for ten minutes. Following this, [1,1'-bis(diphenylphosphino)ferrocene] dichloropalladium complexed with dichloromethane (0.044 mmol) was added, the tube was sealed and heated at 120 °C for 60 minutes in the micro-wave oven. After cooling to room temperature, potassium hydroxide (5 mmol) was added and the tube heated in an oil bath at 90 °C for two hours. This was diluted in water, extracted with ethyl acetate, the organic layer was washed with brine and dried over sodium sulfate and concentrated to dryness. The residue was purified by chromatography on silica gel as described below. 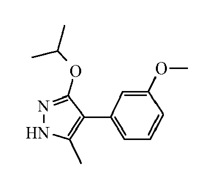 **3-isopropoxy-4-(3-methoxyphenyl)-5-methyl-1H-pyrazole (61c):** Obtained as an oil in a 32 % yield after a chromatography over neutral alumina (1.5 % water; cyclohexane - ethyl acetate 7/3). ¹H (CDCl₃) : 1.39 (d, 6H, J = 6.1); 2.37 (s, 3H); 3.85 (s, 3H); 4.93 (sept, 1H, J = 6.1); 6.80 (m, 1H); 7.08 (m, 1H); 7.12 (m, 1H); 7.30 (m, 1H). ¹³C (CDCl₃) : 11.7; 22.2; 55.1; 71.6; 105.0; 111.2; 113.7; 120.5; 129.2; 133.9; 137.5; 159.5; 160.2. HRMS: Calcd. for C₁₄H₁₈N₂O₂ + H 247.1406. Found: m/z, 247.1387. 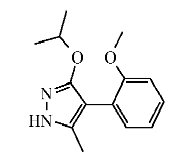 **3-isopropoxy-4-(2-methoxyphenyl)-5-methyl-1H-pyrazole (61d):** Obtained as an oil in a 53 % yield after a chromatography over neutral alumina (1.5 % water; cyclohexane - ethyl acetate 7/3) followed by a second chromatographie over silica gel (dichloromethane / ethanol 98.5 / 1.5). ¹H (CDCl₃): 1.35 (d, 6H, J = 6.1); 2.20 (s, 3H); 3.83 (s, 3H); 4.86 (sept, 1H, J = 6.1); 7.01 (m, 2H); 7.28 (m, 2H). ¹³C (CDCl₃) : 11.5; 22.2; 55.3; 102.2; 110.0; 120.4; 120.8; 128.2; 132.0; 139.2; 156.9; 160.5. HRMS: Calcd. for C₁₄H₁₈N₂O₂ + H : 247.1406. Found: m/z, 247.1398. 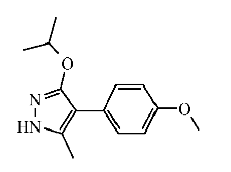 **3-isopropoxy-4-(4-methoxyphenyl)-5-methyl-1H-pyrazole (61e):** Obtained as yellow powder in a 82 % yield after a chromatography over over silica gel (cyclohexane / ethyl acetate 7 / 3). ¹H (CDCl₃): 1.40 (d, 6H, J = 6.1); 2.37 (s, 3H); 3.87 (s, 3H); 4.93 (sept, 1H, J = 6.1); 6.98 (m, 2H); 7.43 (m, 2H). m/z (LC/MS) = 247.

### Preparation of 4-subtituted phenyl 3-alkoxypyrazoles using the Negishi coupling reaction.

In a biotage 20 mL tube, the orgonozinc bromide (0.5 M in THF; 2.52 mmol) and THF (1 mL) were degassed with a slow stream of argon for ten minutes. The pyrazole iodide (0.84 mol) and [1,1'-bis(diphenylphosphino)ferrocene] dichloropalladium complexed with dichloromethane (0.042 mmol) were then added, and the tube was degassed again with a slow stream of argon for 5 min prior to capping. The reaction vessel was then heated at 85 °C for 4 hours in an oil bath. After cooling to room temperature, water and a 1N solution of potassium sodium tartrate (3 mL) were added. The aqueous layer was extracted with ethyl acetate. The combined organic layers were dried over sodium sulphate, filtered and concentrated to dryness. The residue was purified by chromatography on silica gel (dichloromethane - ethanol 99/1). 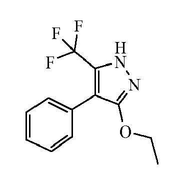 **4-phenyl-3-isopropoxy-5-(trifluoromethyl)-1H-pyrazole (61f):** Obtained as a white powder after a second chromatography over neutral alumina containing 1.5 % water (dichloromethane / ethanol 95-5). ¹H (CDCl₃): 1.40 (t, 3H, J = 7.1); 4.31 (q, 2H, J = 7.1); 7.34 (m, 1H); 7.40 (m, 2H); 7.50 (m, 2H). ¹³C (CDCl₃): 14.7; 65.9; 107.3; 120.1 (q, J = 270 Hz); 127.5; 128.3; 128.8; 129.2; 131.5 (1); 159.7. m/z (LC/MS) = 257.

**Palladium-catalysed C-arylation of compound 55b or 41a.** In a 60 mL round-bottomed thick glass tube fitted with a PTFE-faced screw-cap, a mixture of the considered pyrazole (1.14 mmol), the relevant boronic acid (2.27 mmol), cesium carbonate (2.27 mmol) in dioxane (4 mL) and water (1 mL) was degassed with a slow stream of argon for ten minutes. Following this, [1,1'-bis(diphenylphosphino)ferrocene] dichloropalladium complexed with dichloromethane (0.057 mmol) was added, the tube was closed tightly and heated at 90 °C overnight. After cooling to room temperature, the reaction mixture was diluted in water and extracted with ethyl acetate. The organic phase was dried over sodium sulfate and concentrated to dryness. The residue was purified by chromatography on silica gel as described below. 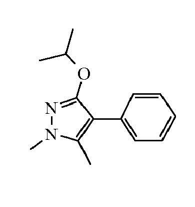 **3-isopropoxy-1,5-dimethyl-4-phenyl-1H-pyrazole (62a):** This compound was obtained as an oil in a 61 % yield after a chromatography over neutral alumina (1.5 % water; dichloromethane - cyclohexane 1/4). ¹H (CDCl₃): 1.36 (d, 6H, J = 6.1); 2.31 (s, 3H); 3.71 (s, 3H); 4.88 (sept, 1H, J = 6.1); 7.23 (m, 1H) ; 7. 41 (m, 4H). ¹³C (CDCl₃) : 10.7; 22.3; 35.7; 71.4; 105.8; 125.5; 128.2; 128.6; 133.0; 136.6; 158.6. HRMS: Calcd. for C₁₄H₁₈N₂O + H : 231.1497. Found: m/z, 231.1528. 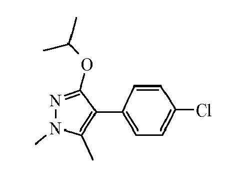 **4-(4-chlorophenyl)-3-isopropoxy-1,5-dimethyl-1H-pyrazole (62b):** This compound was obtained as a solid in a 67 % yield after a chromatography over neutral alumina (1.5 % water; dichloromethane - cyclohexane 1/9). ¹H (CDCl₃) : 1.35 (d, 6H, J = 6.1); 2.29 (s, 3H); 3.70 (s, 3H); 4.88 (sept, 1H, J = 6.1); 7.34 (m, 4H).. ¹³C (CDCl₃): 10.7; 22.2; 35.7; 71.4; 104.7; 128.3; 129.7; 131.1; 131.5; 136.6; 158.4. HRMS: Calcd. for C₁₄H₁₇³⁵ClN₂O + H : 265.1108 Found: m/z, 231.1528. 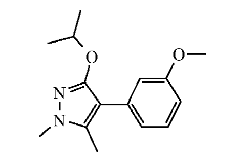 **3-isopropoxy-4-(3-methoxyphenyl)-1,5-dimethyl-1H-pyrazole (62c):** This compound was obtained as an oil in a 43 % yield after a chromatography over silica gel (cyclohexane - ethyl acetate 97/3). ¹H (CDCl₃): 1.38 (d, 6H, J = 6.1); 2.31 (s, 3H); 3.69 (s, 3H); 3.84 (s, 3H); 4.91 (sept, 1H, J = 6.1); 6.80 (m, 1H); 7. 05 (m, 2H); 7.30 (m, 1H). ¹³C (CDCl₃) : 10.8; 22.2; 35.6; 55.1; 71.4; 105.6; 111.1; 114.2; 129.4; 134.3; 136.8; 158.6; 159.5. HRMS: Calcd. for C₁₅H₂₀N₂O₂ + H : 261.1603. Found: m/z, 261.1553. 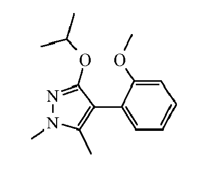 **3-isopropoxy-4-(2-methoxyphenyl)-1,5-dimethyl-1H-pyrazole (62d):** This compound was obtained as an oil in a 41 % yield after a chromatography over neutral alumina (1.5 % water; dichloromethane - cyclohexane 1/9). ¹H (CDCl₃): 1.34 (d, 6H, J = 6.1); 2.13 (s, 3H); 3.71 (s, 3H); 3.83 (s, 3H); 4.85 (sept, 1H, J = 6.1); 6.98 (m, 2H); 7. 28 (m, 2H). ¹³C (CDCl₃): 10.9; 22.3; 35.7; 55.3; 71.1; 102.1; 110.9; 120.4; 121.4; 127.8; 132.1; 138.1; 156.9; 158.7. HRMS: Calcd. for C₁₅H₂₀N₂O₂ + H : 261.1603. Found: m/z, 261.1531. 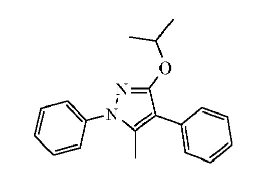 **3-isopropoxy-5-methyl-1,4-diphenyl-1H-pyrazole (63a):** This compound was obtained as an oil in a 90 % yield after a chromatography over neutral alumina (1.5 % water; dichloromethane - cyclohexane 1/4). ¹H (CDCl₃): 1.42 (d, 6H, J = 6.1); 2.39 (s, 3H); 5.06 (sept, 1H, J = 6.1); 7.27 (m, 1H); 7.34 (m, 1H); 7.47 (m, 8H). ¹³C (CDCl₃): 13.0; 22.7; 71.8; 108.5; 125.2; 126.2; 127.2; 128.6; 129.1; 129.4; 132.9; 132.2; 140.4; 160.6. HRMS: Calcd. for C₁₉H₂₀N₂O + H : 293.1654. Found: m/z, 293.1672. 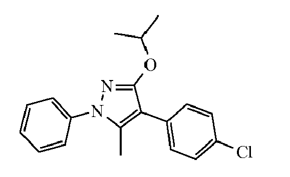 **4-(4-chlorophenyl)-3-isopropoxy-5-methyl-1-phenyl-1H-pyrazole (63b):** This compound was obtained as a solid in a 79 % yield after a chromatography over silica gel (dichloromethane - cyclohexane 2/8). ¹H (CDCl₃): 1.43 (d, 6H, J = 6.1); 2.38 (s, 3H); 5.08 (sept, 1H, J = 6.1); 7.38 (m, 3H); 7.50 (m, 6H). ¹³C (CDCl₃): 12.6; 22.3; 71.5; 107.0; 124.8; 126.9; 128.4; 129.0; 129.9; 131.0; 131.6; 136.7; 139.9; 160.0. HRMS: Calcd. for C₁₉H₁₉N₂³⁵ClO + H : 293.1654. Found: m/z, 293.1672. 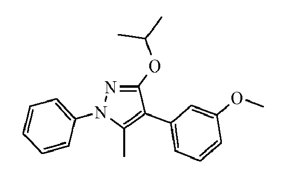 **3-isopropoxy-4-(3-methoxyphenyl)-5-methyl-1-phenyl-1H-pyrazole (63c):** This compound was obtained as an oil in a 80 % yield after a chromatography over neutral alumina (dichloromethane - cyclohexane 1/9). ¹H (CDCl₃) : 1.45 (d, 6H, J = 6.1); 2.43 (s, 3H); 3.89 (s, 3H); 5.11 (sept, 1H, J = 6.1); 6.88 (m, 1H); 7.16 (m, 2H); 7.36 (m, 2H); 7.50 (m, 4H). ¹³C (CDCl₃): 12.7; 22.3; 55.1; 71.3; 107.9; 111.6; 114.4; 121.1; 125.0; 126.8; 128.7; 128.9; 133.8; 136.9; 140.0; 159.6; 160.2. m/z (LC/MS) = 323. 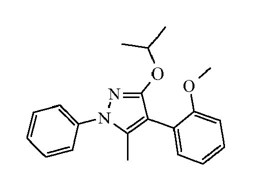 **3-isopropoxy-4-(2-methoxyphenyl)-5-methyl-1-phenyl-1H-pyrazole (63d):** This compound was obtained as an oil in a 71 % yield after a chromatography over neutral alumina (dichloromethane - cyclohexane 2/8). ¹H (CDCl₃): 1.47 (d, 6H, J = 6.1); 2.29 (s, 3H); 3.90 (s, 3H); 5.12 (sept, 1H, J = 6.1); 7.08 (m, 2H); 7.35 (m, 2H); 7.47 (m, 3H); 7.61 (m, 2H). ¹³C (CDCl₃): 12.7; 22.3; 55.4; 71.2; 104.9; 111.2; 120.5; 121.1; 121.4; 124.4; 126.5; 128.2; 128.7; 132.3; 138.2; 140.1; 157.1; 160.5. m/z (LC/MS) = 323. 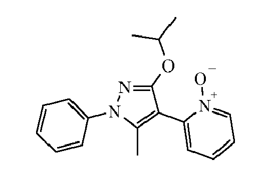 **2-(3-isopropoxy-5-methyl-1-phenyl-1H-pyrazol-4-yl)pyridine 1-oxide (64):** In a 5 ml biotage vial, 4-bromo-3-isopropoxy-5-methyl-1-phenyl-1H-pyrazole **(39a)** (0.28 g, 0.95 mmol), pyridine N-oxide (0.35 g, 3.75 mmol), di-tert-butylmethyl phosphonium tetrafluoroborate (0.035 g, 0.14 mmol), potassium carbonate (0.26 g, 1.87 mmol) and palladium acetate (10.5 mg, 0.047 mmol) were dispersed in toluene (5 mL). This was degassed by a slow stream of argon for 10 minutes, sealed and heated in the microwave oven for 40 minutes at 170 °C. The resulting mixture was adsorbed over a small amount of silica and purified by a chromatography over silica gel (dichloromethane - ethanol 98/2) to yield compound **64** (0.18 g, 61 %) as a solid. ¹H (CDCl₃): 1.34 (d, 6H, J = 6.1); 2.36 (s, 3H); 5.01 (sept, 1H, J = 6.1); 7.11-7.34 (m, 3H); 7.42-7.53 (m, 5H); 8.30 (m, 1H). ¹³C (CDCl₃): 14.2; 22.2; 71.8; 99.8; 123.4; 124.8; 125.0; 127.3; 128.8; 128.9; 139.5; 139.9; 141.3; 142.9; 159.9. HRMS: Calcd. for C₁₈H₁₉N₃O + H : 310.1555. Found: m/z, 310.1570. 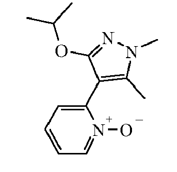 **2-(3-isopropoxy-5-methyl-1-methyl-1H-pyrazol-4-yl)pyridine 1-oxide (65):** From 4-bromo-3-isopropoxy-1,5-dimethyl-1H-pyrazole, by using the protocol used for the preparation of **64**, compound **65** was obtained as a solid in a 9 % yield. ¹H (CDCl₃): 1.28 (d, 6H, J = 6.4); 2.30 (s, 3H); 3.60 (s, 3H); 4.84 (sept, 1H, J = 6.4); 7.10 (m, 1H); 7.20 (m, 1H); 7.43 (m, 1H); 8.24 (m, 1H). LC/MS: m/z = 248 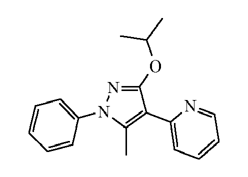 **2-(3-isopropoxy-5-methyl-1-phenyl-1H-pyrazol-4-yl)pyridine (66):** Compound **64** (0.15 g, 0.48 mmol), ammonium formate (0.3 g, 4.85 mmol), 10 % palladium over charcoal (5 mg, 0.05 mmol) were heated to reflux in methanol (10 mL) for 20 hours. The crude residue obtained after concentration to dryness was purified by a chromatography over silica gel (dichloromethane) to yield compound **66** (0.1 g, 71 %). ¹H (CDCl₃) : 1.45 (d, 6H, J = 6.1); 2.65 (s, 3H); 5.13 (sept, 1H, J = 6.1); 7.07 (m, 1H); 7.36 (m, 1H); 7.49 (m, 4H); 7.66 (m, 1H); 7.90 (m, 1H); 8.62 (m, 1H). ¹³C (CDCl₃): 13.2; 22.4; 71.6; 106.7; 119.9; 122.7; 125.3; 127.2; 129.0; 135.8; 139.7; 139.9; 148.8; 153.2; 160.5. HRMS: Calcd. for C₁₈H₁₉N₃ + H : 294.1606. Found: m/z, 294.1624.

**Preparation of 63a and 67 by the copper-catalysed N-arylation of 61a.** Compound **61a** (0.16 g, 0.74 mmol) phenyl boronic acid (0.1 g, 0.81 mmol), pyridine (0.12 mL, 1.48 mmol; dried over 4Å molecular sieves), copper (II) acetate hydrate (0.22 g, 1.11 mmol) and 4Å molecular sieves (0.3 g) were stirred in open air for 48 hours in dichloromethane (50 mL). The resulting suspension was absorbed on a small amount of silica gel and purified by a chromatography over silica gel (dichloromethane - cyclohexane 5/5 to 7/3) to yield the 3-isopropoxy-5-methyl-1,4-diphenyl-1H-pyrazole (**63a**) (0.18 g, 83 % as described above) and then compound **67** (0.01 g, 4 %). 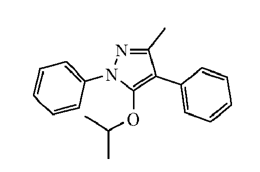 **5-isopropoxy-3-methyl-1,4-diphenyl-1H-pyrazole (67):** ¹H (CDCl₃): 1.03 (d, 6H, J = 6.1); 2.39 (s, 3H); 4.06 (sept, 1H, J = 6.1); 7.30 (m, 2H); 7.45 (m, 4H); 7.51 (m, 2H); 7.78 (m, 2H). ¹³C (CDCl₃): 13.3; 22.6; 77.8; 108.6; 123.1; 127.0; 128.6; 128.8; 129.1; 129.3; 133.1; 139.3; 147.4; 149.9. HRMS: Calcd. for C₁₉H₂₀N₂O + H : 293.1654. Found: m/z, 293.1665. 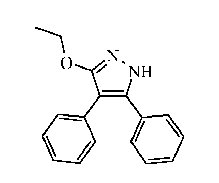 **3-ethoxy-4,5-diphenyl-1H-pyrazole (68):** In a 10 mL biotage glass tube compound **31** (0.29 g, 9.2 mmol), phenyl boronic acid (0.14 g, 1.20 mmol), cesium carbonate (0.75 g, 2.30 mmol), lithium chloride (0.078 g, 1.84 mmol) in propanol (2.5 mL) and water (2.5 mL) was degassed with a slow stream of argon for ten minutes. Following this, [1,1'-bis(diphenylphosphino)ferrocene] dichloropalladium complexed with dichloromethane (0.038g, 0.046 mmol) was added, the tube was sealed and heated at 120 °C for 30 minutes in the micro-wave oven. After cooling to room temperature, the reaction mixture was diluted in water and extracted with dichloromethane. The organic phase was dried over sodium sulfate and concentrated to dryness. The residue was purified by chromatography over alumina containing 1.5 % water (dichloromethane and then dichloromethane - ethanol 99/1) to yield, in this order, compound **68** (0.05 g, 20 %) and then the reduced compound **10g** (0.09 g, 52 %). ¹H (CDCl₃): 1.42 (t, 3H, J = 7.0); 4.34 (q, 2H, J = 7.0); 7.20 (m, 1H) ; 7.32 (m, 2H); 7.37 (m, 7H); 9.67 (s(1), 1H). ¹³C (CDCl₃): 15.0; 64.1; 104.6; 126.1; 127.8; 128.2; 128.7; 128.9; 129.1; 130.3; 131.6; 141.2; 161.6. HRMS: Calcd. for C₁₇H₁₆N₂O+ H : 265.1341. Found: m/z, 265.1292.

**Palladium-catalysed C-pyridylation of 3-alkoxy-4-iodopyrazoles.** In biotage 5 mL tube a mixture of the mixture of pyrazoles **59a-b** or the relevant N-substituted pyrazole (1.1 mmol), the relevant pyridylboronic acid (1.45 mmol), cesium carbonate (2.8 mmol), lithium chloride (2.2 mmol) in propanol (5 mL) and water (5 mL) was degassed with a slow stream of argon for ten minutes. Following this, [1,1'-bis(diphenylphosphino)ferrocene] dichloropalladium complexed with dichloromethane (0.044 mmol) was added, the tube was sealed and heated at 90 °C for 90 minutes in the microwave oven. After cooling to room temperature, potassium hydroxide (5 mmol) was added and the tube heated in an oil bath at 90 °C for two hours. This was diluted in brine, extracted with ethyl acetate, the organic layer was washed with brine and dried over sodium sulfate and concentrated to dryness. The residue was purified by chromatography over silica gel. 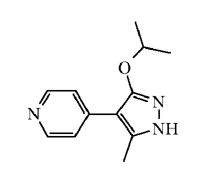 **4-(3-isopropoxy-5-methyl-1H-pyrazol-4-yl)pyridine (69)** Obtained as a beige powder. ¹H (CDCl₃): 1.44 (d, 6H, J = 6.1); 2.48 (s, 3H); 5.01 (sept, 1H, J = 6.1); 7.50 (m, 2H); 8.60 (m, 2H); 9.4 (s(br), 1H). LC/MS : m/z = 218 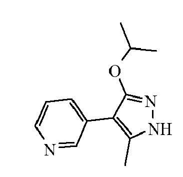 **3-(3-isopropoxy-5-methyl-1H-pyrazol-4-yl)pyridine (70)** Obtained as an oil. ¹H (CDCl₃): 1.41 (d, 6H, J = 6.1); 2.43 (s, 3H); 4.98 (sept, 1H, J = 6.1); 7.30 (m, 1H); 7.84 (m, 1H); 8.49 (m, 1H); 8.77 (m, 1H); 8.94 (s(br), 1H). LC/MS: *m*/*z* = 218. 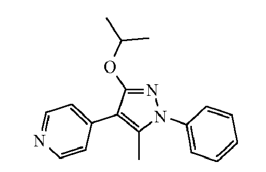 **4-(3-isopropoxy-5-methyl-1-phenyl-1H-pyrazol-4-yl)pyridine (71)** Obtained as a brown powder. ¹H (CDCl₃): 1.41 (d, 6H, J = 6.1); 2.42 (s, 3H); 5.07 (sept, 1H, J = 6.1); 7.44 (m, 7H); 8.59 (m, 2H). LC/MS: m/z = 294. 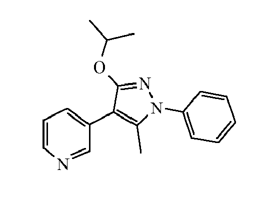 **3-(3-isopropoxy-5-methyl-1-phenyl-1H-pyrazol-4-yl)pyridine (72)** Obtained as a yellow solid. ¹H (CDCl₃): 1.44 (d, 6H, J = 6.1); 2.42 (s, 3H); 5.09 (sept, 1H, J = 6.1); 7.40 (m, 1H); 7.50 (m, 5H); 7.88 (m, 1H); 8.51 (m, 1H); 8.79 (m, 1H). LC/MS: m/z = 294.

### C- Neghishi-based carbon-carbon bond formation methods Preparation of 4-subtituted benzyl or phenethyl 3-alkoxypyrazoles using the Negishi coupling reaction.

In a biotage 20 mL tube, the orgonozinc bromide (0.5 M in THF; 2.52 mmol) and THF (1 mL) were degassed with a slow stream of argon for ten minutes. The pyrazole iodide (0.84 mol) and [1,1'-bis(diphenylphosphino)ferrocene] dichloropalladium complexed with dichloromethane (0.042 mmol) were then added, and the tube was degassed again with a slow stream of argon for 5 min prior to capping. The reaction vessel was then heated at 85 °C for 4 hours in an oil bath. After cooling to room temperature, water and a 1N solution of potassium sodium tartrate (3 mL) were added. The aqueous layer was extracted with ethyl acetate. The combined organic layers were dried over sodium sulphate, filtered and concentrated to dryness. The residue was purified by chromatography on silica gel (dichloromethane - ethanol 99/1). 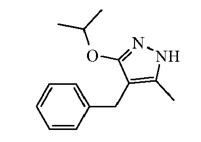 **4-benzyl-3-isopropoxy-5-methyl-1*H*-pyrazole (73a):** Obtained as an oil. ¹H (CDCl₃): 1.34 (d, 6H, J = 6.1); 2.10 (s, 3H); 3.70 (s, 2H); 4.85 (sept, 1H, J = 6.1); 7.23 (m, 5H). ¹³C (CDCl₃): 10.3; 22.2; 27.7; 71.4; 102.8; 125.6; 128.2; 128.3; 137.8; 141.4; 161.5. Calcd. for C₁₄H₁₈N₂O + H : 231.1497. Found: m/z, 231.1417. 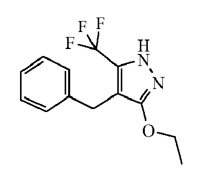 **4-benzyl-3-isopropoxy-5-(trifluoromethyl)-1H-pyrazole (73b):** Obtained as a white solid after a second chromatography over neutral alumina containing 1.5 % water (dichloromethane / ethanol 95-5). ¹H (CDCl₃): 1.38 (t, 3H, J = 7.1); 3.83 (s, 2H); 4.26 (q, 2H, J = 7.1); 7.25 (m, 5H). ¹³C (CDCl₃) : 14.7; 27.1; 65.5; 105.5; 122.1 (q, J = 270 Hz); 126.2; 128.3; 130.9; 131.3; 139.5; 161.2. m/z (LC/MS) = 271. 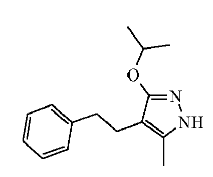 **3-isopropoxy-5-methyl-4-phenethyl-1H-pyrazole (74):** Obtained as an oil. ¹H (CDCl₃): 1.35 (d, 6H, J = 6.1); 1.92 (s, 3H); 2.59 (t, 2H, J = 7.7); 2.79 (t, 2H, J = 7.7); 4.85 (sept, 1H, J = 6.1); 7.15 (m, 2H); 7.21 (m, 1H) ; 7.27 (m, 2H). ¹³C (CDCl₃): 9.9; 22.3; 23.9; 35.9; 72.2; 103.0; 125.8; 128.2; 128.6; 138.6; 142.0; 160.4. m/z (LC/MS) = 245. 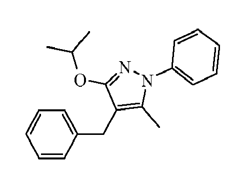 **4-benzyl-3-isopropoxy-5-methyl-1-phenyl-1*H*-pyrazole (75):** Obtained as an oil. ¹H (CDCl₃) : 1.37 (d, 6H, J = 6.1); 2.19 (s, 3H); 3.74 (s, 2H); 5.00 (sept, 1H, J = 6.1); 7.20 (m, 1H); 7.29 (m, 5H); 7.42 (m; 4H). ¹³C (CDCl₃): 11.4; 22.3; 28.1; 71.3; 105.3; 124.4; 125.7; 126.3; 128.2; 128.3; 137.3; 140.1; 141.3; 161.3. Calcd. for C₂₀H₂₂N₂O + H : 307.1810. Found: m/z, 307.1806.

### Results

The use of tetrakis(triphenylphosphine) palladium as a catalyst for the Suzuki reaction pointed out a slow reaction, even from iodine-bearing pyrazole **29**. On the other hand, the use of a reported⁴⁴ method, involving [1,1'-bis(diphenylphosphino)ferrocene] dichloropalladium as a catalyst and cesium carbonate as a base in a water/dioxanne solution, led again⁴⁵ to a remarkably short reaction time. How-vever, yields although rather average, also turned out to sometime vary widely. Interestingly, the addition of lithium chloride has been reported to improve a few palladium-based aryl coupling reaction involving halogenated heterocycles with an NH component.⁴⁶⁻⁴⁹ This turned out to be also true in some cases, as this salt was essential in avoiding this variability in the preparation of compound **61a.** Moreover, the reaction time was shortened even further by the use of a micro-wave oven. From the brominated compound **28,** lesser yields of C-arylation were obtained under these reaction conditions. From the electron-rich 3-methoxyphenylboronic acid, very low yield of the coupling product **61c** were obtained along with a host of unidentified product which made fairly hard a proper purification of **61c.** From the hindered 2-methoxyphenylboronic acid, only traces of the coupling product **61d** were observed by LC/MS monitoring of the reaction mixture. Little or no improvements were observed switching from dioxane to propanol as a solvent for this reaction. We then resorted to the palladium-catalysed coupling of the mixture of N-mesylated isomers **59a-b** with 2, 3 or 4-methoxyphenylboronic acid. This was followed *in situ* by the basic hydrolysis of the mesylate group using potassium hydroxide. This approach allowed the far easier preparation of compound **61c-e** in respectively 32 %, 55 % or 82 % overall yields. Little arylation was seen from the trifluromethyl homologues **60a-b**, but the target C4-phenyl derivative **61f** could be prepared in a low 4% yield using the Negishi coupling reaction between compound **30** and phenylzincbromide.

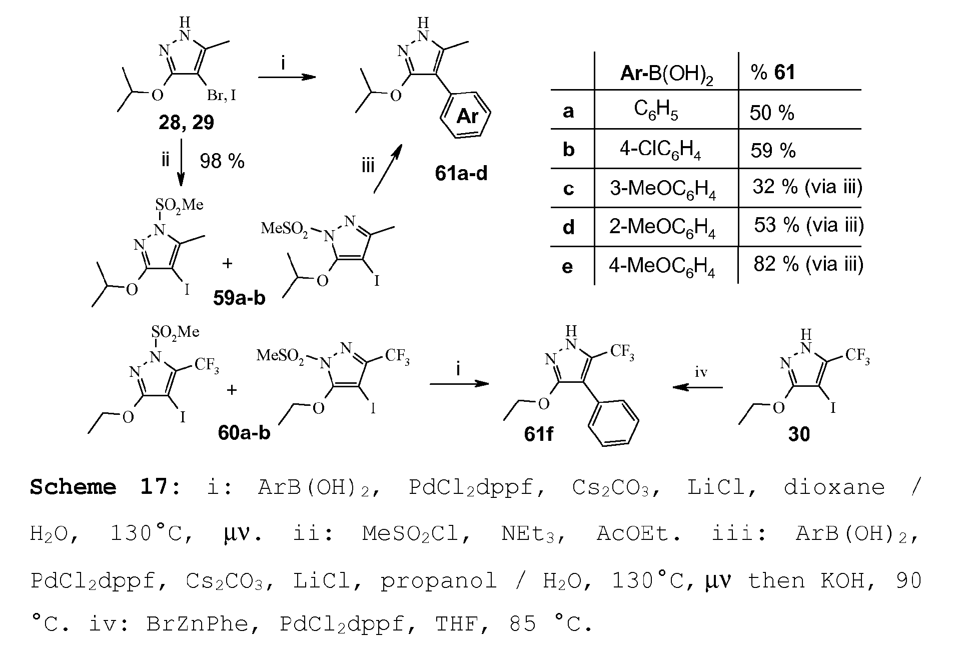

Somehow logically, from the N-methylated or N-phenyl iodo-bearing derivatives **41a** and **55b,** much less problems were encountered. Indeed, without the recourse to the addition of lithium chloride, nor, in early cases, a microwave oven, consistent (an better) yields of the arylation products **62a-d** or **63a-d** were obtained from their aryl-aryl coupling with various arylboronates.

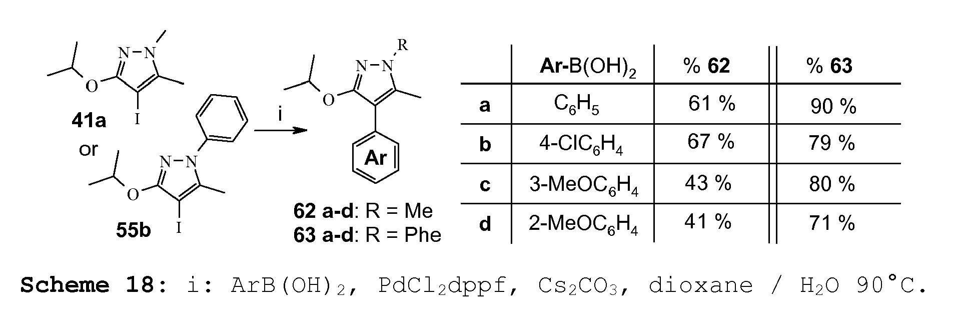

In order to increase the chemical diversity attainable from these pyrazole, the recently reported⁵⁰ coupling of pyridine N-oxide to the halogenated pyrazoles **39a** and **41a** was also applied. Unexpectedly, from the iodo-bearing N-phenyl derivative **41a,** using microwave-based heating, none of the expected arylation product was obtained. A slow reduction of the iodine atom was the sole transformation of **41a** we could monitor by LC/MS. On the other hand, from the bromo-bearing homolog **39a,** the aryl coupling reaction proceeded well as the 2-pyridyl-N-oxide bearing substrate **64** was isolated in a **61** % yield. Unfortunately, much less of C4-arylation product **65** could be obtained from the N-methyl analogue of **39a** and none from the NH-bearing compounds **28** or **29** (with or without lithium chloride added). Compound **64** was further catalytically reduced to the corresponding 2-pyridyl derivative **66** using ammonium formate as a source of hydrogen in boiling ethanol. On the other hand, from the relevant pyridylboronates, the two isomeric -pyridyl derivatives **69** and **70** were prepared by the Suzuki aryl-aryl coupling procedure with the mixture of the mesyl-protected pyrazoles **59a-b.**

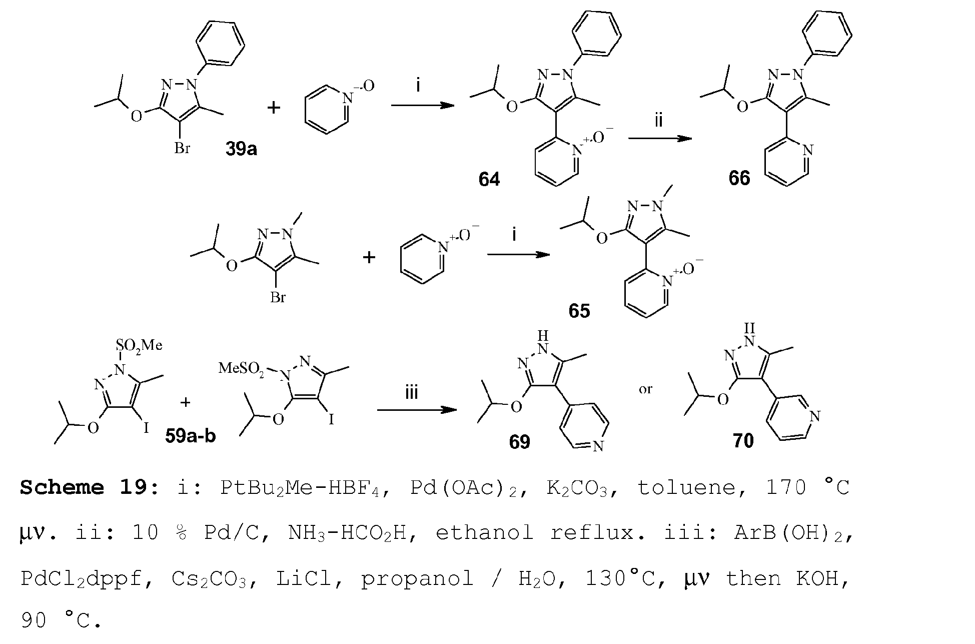

The N-arylation of compound **61a,** by the copper-mediated method previously used, was also studied. From this compound, the previously prepared N-1 arylation product **63a** could be isolated in 83 % yield along with traces amount (4 %) of the N-2 arylation product **67.** Arylation reaction of the more hindered 4-iodo-5-phenyl derivative **31** led to the bis-arylated compound **68** although in only a 20 % yield along with large amount (50 %) of the reduced material **10f.**

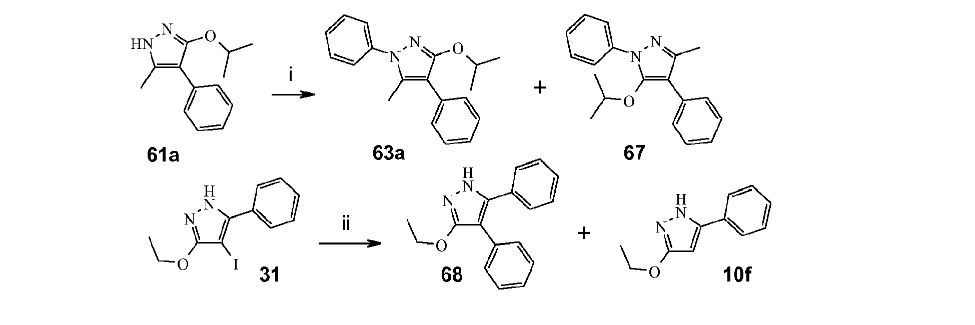

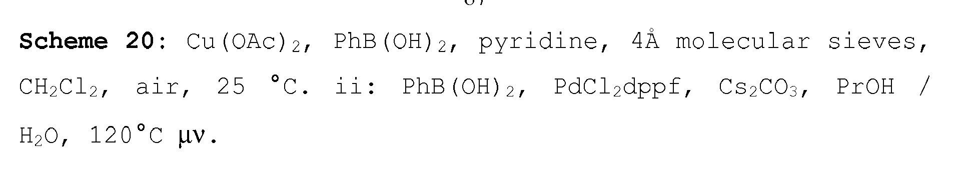

Aside from Suzuki-based arylation reaction, the study of Negishi-based carbon-carbon bond formation from the 4-iodobearing pyrazoles **29** or **41a** and various organozinc bromides was also undertook. The scheme 21 depicts some examples which illustrate a fairly large array of possibilities. Interestingly the presence of an NH moiety of compound **29** or compound **30** does not seem to hinder these coupling reactions leading to compounds **73ab** or **74.**

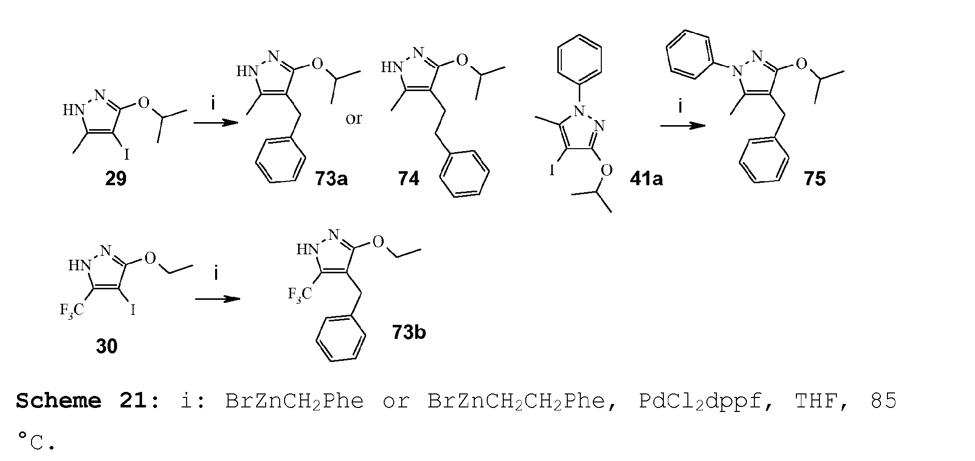

### Example 4 oxygen deprotections of 3-alkoxypyrazoles.

The features of the devices used to conduct analyses of all of the compounds described in Example 4 are indicated hereinbelow:

The ¹H NMR and ¹³C NMR spectra were recorded on a Bruker Avance 400 spectrometers at 400 MHz and 100 MHz, respectively. Unless otherwise noted, CDCl₃ was the solvent used. Shifts (δ) are given in ppm with respect to the TMS signal and coupling constants (J) are given in Hertz. Column chromatography were performed either with Merck silica gel 60 (0.035 - 0.070 mm) or neutral alumina containing a suitable proportion of water, using a solvent pump operating at pressure between 2 and 7 bar (25-50 mL/mn) and an automated collecting system driven by a UV detector set to 254 nm unless stated otherwise (i.e. if ethylacetate was used then it would be set to 280 nm). Sample deposition was always carried out by absorption of the mixture to be purified on a small amount of the solid phase followed by its deposition of the top of the column. The low resolution mass spectra were obtained on an Agilent 1100 serie LC/MSD system using an atmospheric electrospray ionisation system and the high resolution mass spectroscopy spectra (HRMS) were obtained using a Waters Micromass Q-Tof with an electrospray ion source.

### A-deprotection of 3-alkoxypyrazoles using hydrogen bromide in acetic acid.

The considered alkoxypyrazole (1 mmol) was placed in a 60 mL round-bottomed thick glass tube fitted with a PTFE-faced screw-cap. This was degassed with argon and then 33 % hydrogen bromide in acetic acid (1.5 mL) was added. The tube was tightly closed and was heated at 140 °C for 3 hours. The resulting solution was cooled, opened and worked up as described below. 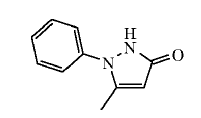 **5-methyl-1-phenyl-1H-pyrazol-3(2H)-one (76a)**⁵¹ : This compound was obtained in a 74 % yield as a white powder after a chromatography over silica gel (dichloromethane - ethanol 98/2) of the extracted residue. ¹H (CDCl₃) : 2.27 (s, 3H) ; 5.61 (s, 1H) ; 7.40 (m, 3H) ; 7.48 (m, 2H). ¹³C (CDCl₃): 13.0; 93.8; 125.1; 127.3; 129.6; 139.2; 141.1; 163.2. HRMS: Calcd. for C₁₀H₁₀N₂O + H : 175.0871. Found: m/z, 175.0894. 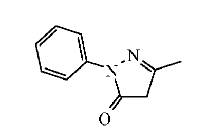 **3-methyl-1-phenyl-1H-pyrazol-5(4H)-one (76b):** This compound was obtained in a 76 % yield as a white powder after a chromatography over silica gel (dichloromethane - ethanol 98/2) of the extracted residue. Its analytical data are identical with a commercially available sample. Note: spectra in deuterated chloroform show the existence of a unique methylene signal whereas spectra in deuterated dimethylsulfoxide show the existence of a mixture of the two possible bonds distribution. 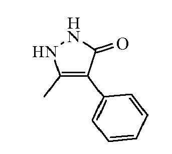 **5-methyl-4-phenyl-1H-pyrazol-3(2H)-one (77a):⁵²** Obtained as white solid in a 43 % yield after the extraction. ¹H (DMSO*d₆*): 2.27 (s, 3H, CH₃); 7.13 (m, 1H); 7.33 (m, 2H); 7.46 (m, 2H); 10.4 (s(br), 1H). ¹³C (DMSO-*d₆*): 11.5; 102.3; 124.7; 127.3; 128.1; 133.5; 136.5; 158.8. m/z (LC/MS) = 175. 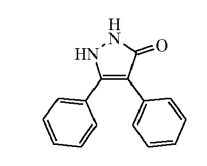 **4,5-diphenyl-1H-pyrazol-3(2H)-one (77b):** Obtained as a white solid in a 73 % yield after a chromatography over silica gel (dichloromethane - ethanol 98/2) of the concentrated residue. ¹H (DMSO-*d₆*): 7.15 (m, 1H); 7.34 (m, 9H) ; 10. 00 (s(1), 1H); 11.95 (s(1), 1H). ¹³C (DMSO-*d₆*): 102.6; 125.5; 127.4; 128.0 (two signals ?); 128.6; 128.8; 130.6; 132.7; 139.6; 159.0. m/z (LC/MS) = 237. 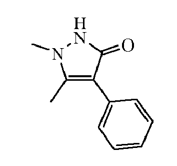 **1,5-dimethyl-4-phenyl-1H-pyrazol-3(2H)-one (78)**⁵³: Obtained as white solid in a 76 % yield after the extraction of the aqueous phase which had been saturated with sodium chloride. ¹H (DMSO-*d₆*): 2.25 (s, 3H, CH₃); 3.58 (s, 3H); 7.17 (m, 1H); 7.34 (m, 4H); 9.71 (s(br), 1H). ¹³C (DMSO-*d₆*): 11.0; 35.8; 103.9; 125.4; 128.3; 128.6; 133.8; 136.5; 157.6. HRMS: Calcd. for C₁₁H₁₂N₂O + H : 189.1028. Found: m/z, 189.1054. 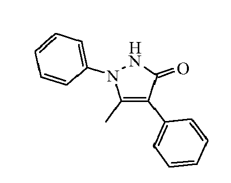 **5-methyl-1,4-diphenyl-1H-pyrazol-3(2H)-one (79):** Obtained as a white solid in a 92 % yield after precipitation of the reaction mixture with an excess of water followed by a filtration. ¹H (DMSO-*d₆*): 2.35 (s, 3H, CH₃) ; 7.25 (m, 1H); 7.36 (m, 2H); 7.42 (m, 2H); 7.48 (m, 5H); 10.32 (s(br), 1H). ¹³C (DMSO-*d₆*): 13.0; 107.0; 124.4; 126.1; 127.0; 128.7; 128.8; 128.5; 132.9; 136.7; 139.9; 159.8. HRMS: Calcd. for C₁₆H₁₄N₂O + H : 251.1184. Found: m/z, 251.1199. 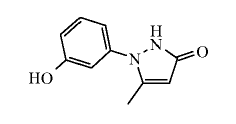 **1-(3-hydroxyphenyl)-5-methyl-1H-pyrazol-3(2H)-one (80):** This compound was obtained in a 64 % yield as a white powder after a chromatography over silica gel (dichloromethane - ethanol 98/2 to 95/5) of the concentrated residue. ¹H (DMSO-*d₆*): 2.25 (s, 3H, CH₃); 5.55 (s, 1H); 6.70 (m, 1H); 6.86 (m, 2H); 7.22 (m, 1H); 9.68 (s(br), 1H); 9.84 (s(br), 1H). ¹³C (DMSO-*d₆*): 12.8; 93.7; 110.4; 113.1; 113.7; 129.6; 139.4; 140.7; 157.7; 161.1. HRMS: Calcd. for C₁₀H₁₀N₂O₂ + H : 191.0821. Found: m/z, 191.0841. 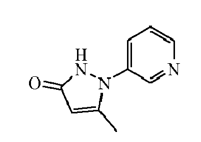 **5-methyl-1-(pyridin-3-yl)-1H-pyrazol-3(2H)-one (81):** Obtained as a solid after a chromatography over silica gel (dichloromethane / ethanol 95/5). ¹H (CDCl₃): 2.35 (s, 3H); 5.70 (s, 2H); 7.47 (m, 1H); 7.82 (m, 1H); 8.60 (m, 1H); 8.93 (s, 1H). ¹³C (CDCl₃)_{:} 12.7; 94.8; 123.8; ;131.0; 135.8; 141.2; 145.3; 147.4; 163.3. HRMS Calc. for C₉H₉N₃O + H : 176.0824. Exp: 176.0764. 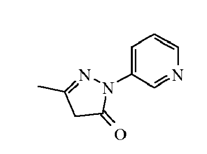 **3-methyl-1-(pyridin-3-yl)-1H-pyrazol-5(4H)-one (82):** Obtained as a solid after a chromatography over silica gel (dichloromethane / ethanol 95/5). ¹H (CDCl₃): 2.23 (s, 3H); 3.46 (s, 2H); 7.34 (m, 1H); 8.29 (m, 1H); 8.45 (m, 1H); 9.20 (s, 1H). ¹³_{C} (CDCl₃)_{:} 17.1; 42.7; 123.7; 125.6; 134.9; 139.9; 145.3; 157.3; 170.8. HRMS: Calc. for C₉H₉N₃O + H : 176.0824. Exp: 176.0788. 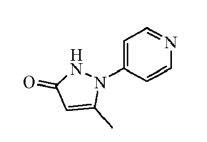 **5-methyl-1-(pyridin-4-yl)-1H-pyrazol-3(2H)-one (83):** Obtained as a solid after a chromatography over silica gel (dichloromethane / ethanol 94/6). ¹H (CDCl₃): 2.54 (s, 3H); 5.78 (s, 1H) ; 7.43 (m, 2H); 8.71 (m, 2H) . ¹³C (CDCl₃): ¹³C (DMSO*d6*): 13.5; 96.9; 115.5 (br); 141.2; 146.1; 150.5; 162.2. HRMS : Calc. for C₉H₉N₃O + H : 176.0824. Exp. : 176.0866. 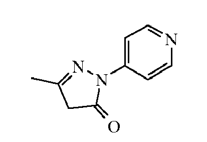 **3-methyl-1- (pyridin-4-yl) -1H-pyrazol-5 (4H) -one (84):** Obtained as a solid after a chromatography over silica gel (dichloromethane / ethanol 94/6) followed by washing it in boiling tetrachloromethane. ¹H (CDCl₃): 2.26 (s, 3H); 3.51 (s, 2H); 7.99 (m, 2H); 8.60 (m, 2H). ¹³C (CDCl₃): 14.5; 89.8; 113.0; 145.4; 150.6. HRMS: Calc. for C₉H₉N₃O + H: 176.0824. Exp: 176.0771. 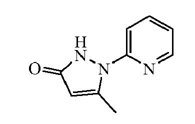 **5-methyl-1-(pyridin-2-yl)-1H-pyrazol-3(2H)-one (85):** Obtained as a solid after a chromatography over silica gel (dichloromethane / ethanol 9/1). ¹H (CDCl₃): 2.62 (s, 3H); 5.70 (s, 1H); 7.17 (m, 1H); 7.61 (m, 1H); 8.84 (m, 1H); 8.43 (m, 1H). ¹³C (CDCl₃): 14.7; 95.9; 115.3; 120.6; 138.6; ;143.4; 147.6; 152.5; 162.8. HRMS: Calc. for C₉H₉N₃O + H : 176.0824. Exp: 176.0864 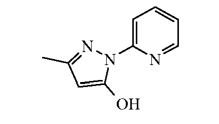 **3-Methyl-1-(pyridin-2-yl)-1H-pyrazol-5-ol (86)⁴³**: This compound was obtained in a better yield if the heating was shorten to one hour and the temperature kept to 100 °C. Obtained as a solid after a chromatography over silica gel (dichloromethane / ethanol 9/1). ¹H (CDCl₃): 2.27 (s, 3H); 5.44 (s, 1H); 7.12-7.18 (m, 1H); 7.80-7.86 (m, 1H); 7.89-7.97 (m, 1H); 8.20-8.30 (m, 1H). ¹³C (CDCl₃): 14.5; 88.7; 111.9; 119.5, 139.8; 145.1; 151.5; 154.1; 157.3. HRMS: Calc. for C₉H₉N₃O + H : 176.0824. Exp: 176.0790. 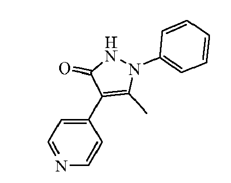 **5-methyl-1-phenyl-4-(pyridin-4-yl)-1H-pyrazol-3(2H)-one (87)** Obtained as a brown powder. ¹H (DMSOd6): 2.58 (s, 3H); 7.56 (m, 5H); 8.13 (m, 2H); 8.76 (m, 2H); 11.8 (s (br), 1H). LC/MS: m/z = 252. 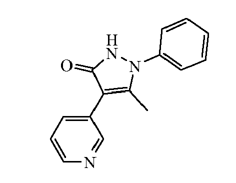 **5-methyl-1-phenyl-4-(pyridin-3-yl)-1H-pyrazol-3(2H)-one (88)** Obtained as an off white powder. ¹H (DMSOd6): 2.37 (s, 3H); 7.44 (m, 6H); 7.87 (m, 1H); 8.45 (m, 1H); 8.69 (m, 1H); 10.59 (s(br), 1H). LC/MS: m/z = 252. 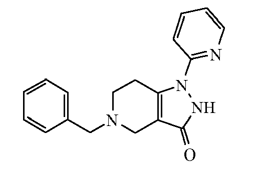 **5-benzyl-1-(pyridin-2-yl)-4,5,6,7-tetrahydro-1H-pyrazolo[4,3-c]pyridin-3(2H)-one (89):** Obtained as a pale yellow powder ¹H (DMSOd6): 2.75 (s, 2H, CH₂₋7); 3.15 (m, 2H, CH₂-6); 3.17 (s, 2H, CH₂-Bn); 3.72 (s, 2H, CH₂-4); 7.12-7.16 (m, 1H, Ar-Py); 7.26-7.30 (m, 1H, Ar-Bn); 7.33-7.38 (m, 5H, Ar-Bn); 7.62 (m, 1H, Ar-Py); 7.83-7.87 (m, 1H, Ar-Py); 8.32-8.33 (m, 1H, Ar-Py); 10.32 (s, 1H, NH). ¹³C (DMSOd6): 26.9; 47.5; 50.2; 61.5; 104.7; 112.6; 119.9; 127.5; 128.7; 129.2; 139.2; 139.5; 148.0; 153.3; 158.9. HRMS: calc for C₁₈H₁₉N₄O + H: 307.1559 Exp. 307.1545. 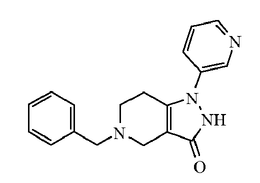 **5-benzyl-1-(pyridin-3-yl)-4,5,6,7-tetrahydro-1H-pyrazolo[4,3-c]pyridin-3(2H)-one (90):** Obtained as a beige powder ¹H (CDCl₃): 2.69 (t, 2H, CH₂-7); 2.88 (t, 2H, CH₂-6); 3.25 (s, 2H, CH₂-4); 3.69 (s, 2H, CH₂-Bn); 7.25-7.37 (m, 5H, Ar-Bn); 7.43-7-46 (m, 1H, Ar-Py); 7.86-7.88 (m, 1H, Ar-Py); 8.40 (m, 1H, Ar-Py) ; 8.75 (s, 1H, Ar-Py) ; 10.21 (s, 1H, NH). ¹³C (CDCl₃): 24; 47.2; 49.5; 61.0; 103.3; 123.9; 127.0; 127.3; 128.2; 128.7; 136.4; 138.3; 138.5; 141.5; 145.6; 158.6. HRMS: calc for C₁₈H₁₉N₄O + H: 307.1559 Exp. 307.1527. 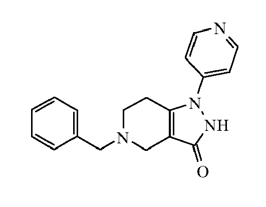 **5-benzyl-1-(pyridin-4-yl)-4,5,6,7-tetrahydro-1H-pyrazolo[4,3-c]pyridin-3(2H)-one (91):** Obtained as a beige powder ¹H (CDCl₃): 2.83 (m, 2H); 2. 99 (m, 2H); 3.49 (s, 2H) ; 3.79 (s, 2H); 7.31 (m, 7H); 8.66 (m, 2H). ¹³C (CDCl₃): 25.8; 47.6; 49.6; 62.1; 106.1; 114.4; 127.4; 128.5; 129.2; 137.8; 139.6; 146.2; 150.4; 160.6. HRMS: calc for C₁₈H₁₈N₄O + H: 307.1559. Exp: 307.1550. 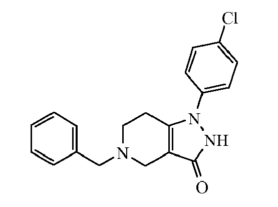 **5-benzyl-1-(4-chlorophenyl)-4,5,6,7-tetrahydro-1H-pyrazolo[4,3-c]pyridin-3(2H)-one hydrobromide salt (92):** In a 60 mL round-bottomed thick glass tube fitted with a PTFE-faced screw-cap, a mixture of the considered alkoxypyrazole (1 mmol) was degassed with argon. Following this, 33 % hydrogen bromide in acetic acid (1.5 mL) was added; the tube was tightly closed and was heated at 140 °C for 2 hours. The resulting solution was cooled, diluted in water, filtered, washed with water and dried under vacuum to yield compound 92 as a white powder (64 %). ¹H (DMSOd6): 3.23 (m, 2H); 3.40 (m, 1H) ; 3.73 (m, 1H) ; 4.02 (m, 2H); 4.52 (m, 2H); 7.50 (m, 7H); 7.65 (m, 2H); 10.35 (s, 1H). ¹³C (DMSOd6): 21.9; 46.2; 48.9; 58.0; 97.6; 123.1; 129.4; 129.8; 130.1; 130.2; 130.5; 131.7; 135.8; 138.5; 158.4. HRMS: Calc for C₁₉H₁₈³⁵ClN₃O + H: 340.1272. Exp: 340.1207. 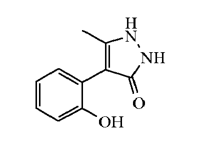 **4-(2-hydroxyphenyl)-5-methyl-1H-pyrazol-3(2H)-one (93):** This compound was obtained after evaporation of the reaction mixture, adsorption over a small amount of silica gel and chromatography over silica gel (dichloromethane / ethanol 95/5 to 91 /9) as a white powder. ¹H (DMSOd6): 2.17 (s, 3H); 6.79 (m, 2H); 7.05 (m, 1H); 7.15 (m, 1H); 11.0 (s(1); 3H). ¹³C (DMSOd6): 11.7; 101.4; 116.7; 118.7; 120.0; 126.9; 129.9; 139.8; 153.0; 160.7. HRMS: C₁₀H₁₀N₂O₂ + H: Cald: 191.0821; found 191.0708. 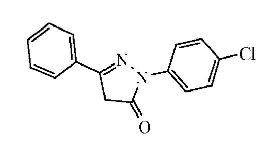 **1-(4-chlorophenyl)-3-phenyl-1H-pyrazol-5(4H)-one (94):** Compound **44** (0.032 g, 0.1 mmol) and 33 % hydrogen bromide in acetic acid (1 mL) were heated in a sealed flask at 110 °C for 36 hours. The solution was dispersed in water and the precipitate extracted with dichloromethane. The organic phase was washed with water, dried over sodium sulfate and concentrated to dryness to yield compound **94** (0.025 g, 86 %) as a solid. ¹H (CDCl₃): 3.86 (s, 2H); 7.40 (m, 2H); 7.48 (m, 2H); 7.78 (m, 2H); 7.99 (m, 2H). ¹³C (CDCl₃): 39.6; 120.0; 126.0; 128.9; 129.0; 130.4; 130.7; 130.9; 154.9; 170.1. HRMS: Calcd. for C₁₅H₁₁³⁵ClN₂O + H : 271.0638. Found: m/z, 271.0657.

### B-Deprotection of 49 using 48 % hydrobromic acid.

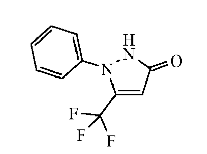

**1-phenyl-5-(trifluoromethyl)-1H-pyrazol-3(2H)-one (95):** Compound 49 (0.5 g, 1.95 mmol) and 48 % hydrogen bromide in water (1 mL) were heated in a sealed flask at 140 °C for 4 hours. The solution was dispersed in water and the precipitate extracted with ethyl acetate. The organic phase was washed with water, dried over sodium sulfate and concentrated to dryness. The residue was further purified by a chromatography over silica gel (cyclohexane - dichloromethane 1/1) to yield compound **95** (0.38 g, 85 %) as a solid. ¹H (DMSO-*d₆*): 5.93 (s, 1H); 7.38 (m, 1H); 7.51 (m, 2H); 7.71 (m, 2H). ¹³C (DMSO-*d₆*): 86.1; 121.0 (q, J = 260); 122.7; 127.7; 129.6; 138.2; 140.9 (q, J = 37); 154.1. HRMS: Calcd. for C₁₀H₇F₃N₂O + H : 229.0589. Found: m/z, 229.0606.

**C-Deprotection using hydrochloric acid.** The considered alkoxypyrazoles was refluxed for 4 hours in 37 % hydrochloric acid. This was concentrated to dryness and the residue purified as described below. 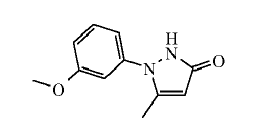 **1-(3-methoxyphenyl)-5-methyl-1H-pyrazol-3(2H)-one (96):** Obtained as a colourless solid in 43 % after a chromatography over silica gel (cyclohexane - ethyl acetate 9-1). ¹H (CDCl₃): 2.30 (s, 3H, CH₃) ; 3.88 (s, 3H); 5.61 (s, 1H); 6.90 (dd, 1H, J = 2.4 and 0.8); 6.98 (m, 2H); 7.36 (t, 1H, J = 8.3). ¹³C (CDCl₃): 13.1; 55.9; 93.9; 110.6; 113.7; 117.0; 130.1; 140.3; 141.2; 160.5; 163.2. HRMS: Calcd. for C₁₁H₁₂N₂O₂ + H: 205.0977. Found: m/z, 205.0999. 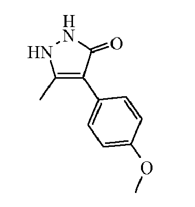 **4-(4-methoxyphenyl)-5-methyl-1H-pyrazol-3(2H)-one (97):** Obtained as a colourless powder in 59 % after a chromatography over silica gel (dichloromethane - methanol 95-5 to 9/1) . ¹H (DMSO-*d₆*): 2.23 (s, 3H, CH₃); 3.75 (s, 3H); 6.92 (m, 2H); 7.36 (m, 2H). *m*/*z* (LC/MS) 205.

### D-deprotection using boron tribromide in dichloromethane

In a moisture-protected setting, compound **33a** (0.36 g; 1.66 mmol) dissolved in dichloromethane (20 mL). At 0°C, the 1M solution of boron tribromide in dichloromethane (3.3 mL; 3.33 mmol) was added and the solution was refluxed 18 hours. The resulting solution was quenched by the addition of ice, extracted with dichloromethane; the organic phase was dried over sodium sulfate and concentrated to dryness. The resulting residue was purified by a chromatography over silica gel (dichloromethane) to yield compound **76a** as described above (0.08 g; 28 %).

### Results

By using hydrogen bromide in acetic acid at 140°C, many pyrazol-3-ones were obtained. Amongst the salient features of these reaction, a quite high temperature was most often required and more importantly the exclusion of oxygen, especially in the case of compounds featuring electron-rich components. With this method, compounds **76a** and **76b** were obtained from compounds **33a** and **34a** in 74 % and 76 % yield respectively. The methylene signal of compound **76b** being a characteristic of these isomers in that specific tautomeric form. From **61a** and **68** the deprotected compound **77a** and **77b** were obtained in 45 % and 73 % yields respectively. Moreover, the N-substituted compounds **78** and **79** were obtained from **62a** and **63a** in 76 % and 92 % yields respectively. The use of boron tribromide, although in boiling dichloromethane, was also effective in cleaving the isopropyl ether of **33a.** However, compound **76a** was isolated in a lesser 28% yield.

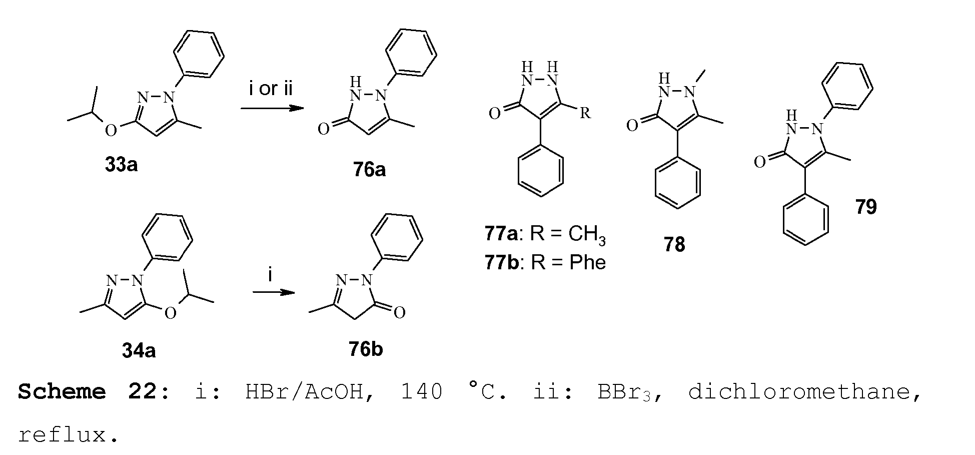

This hydrolysis turned out to be fairly useful in the structure assignment of some of the product of the reactions described above. For instance, as previously mentioned, the methylene signals of **76b, 94,** or **82** and **84** were instrumental in determining the structures of the parent alkyl ethers. In the case of compound **86,** an internal hydrogen bond led to the stabilization of the conformation depicted in scheme 23. However, this compound has been previously prepared via the condensation between 2-pyridylhydrazine and ethylacetoacetate in boiling acetic acid.⁴³ From the methoxy-bearing derivative **33c,** the use of hydrogen bromide in acetic acid led to the full cleavage of the ether residues and gave compound **93** in a 64 % yield. On the other hand, a partial selectivity of deprotection of compound **33c** could be achieved in boiling hydrochloric acid as the main reaction product (43 % yield) was the methoxylated pyrazole **96.** The same phenomenon was observed in the preparation of compound **97.**

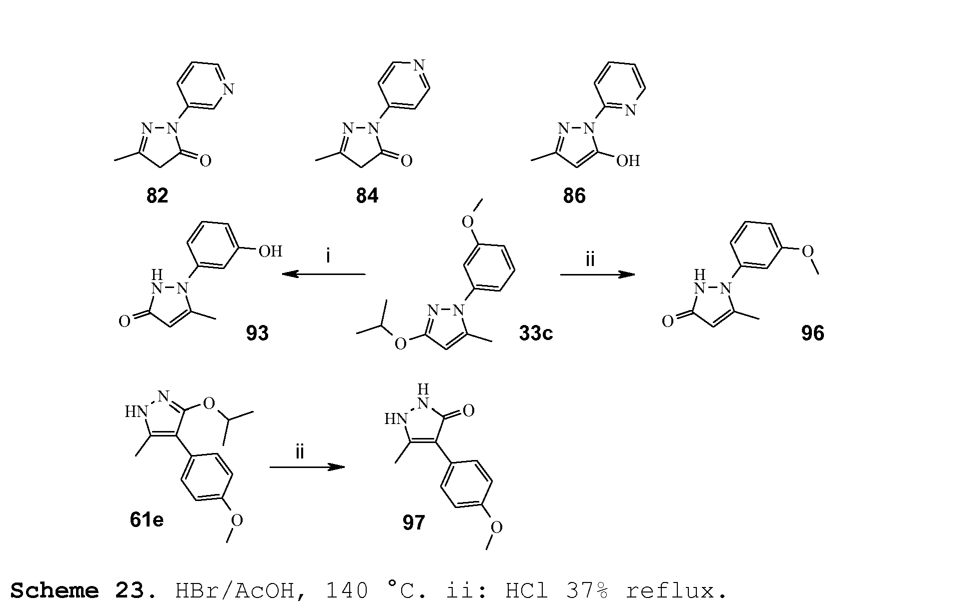

### Example 5: comparison of different molar ratios of hydrazine monochloride in the process of Example 1

According to the process described in **Example 1,** isopropylacetoacetate **9c** (0.01 mmol) and hydrazine monohydrochloride (0.008, 0.0095, 0.01, 0.011 or 0.013 mmol) were refluxed in isopropanol (60 mL) for 8 hours.

### Results

| | | | | | |
|---|---|---|---|---|---|
| **NH₂NH₂ , HCl** | | | 1 | 1.1 | 1.3 |
| | 0.8 eq | 0.95 eq | | | |
| | | | eq | eq | eq |
| | **34 %** | **48 %** | **51** | **50 %** | **50 %** |
| **Yield** | (∼ 29 % starting product) | (< 3% impurities) | **%** | | |

### Example 6: comparison of different salts of hydrazine in the process of Example 1

According to the process described in **Example 1,** isopropylacetoacetate **9c** (0.01 mmol) and hydrazine monohydrochloride, hydrazine dihydrochloride, hydrazine sulfate (0.0105 mmol) were refluxed in isopropanol (60 mL) for 8 hours.

### Results

| | | | | |
|---|---|---|---|---|
| **Hydrazine** | | | NH₂NH₂, | NH₂NH₂, |
| | NH₂NH₂, ½ H₂SO₄ | NH₂NH₂, H₂SO₄ | | |
| **(1.05 eq)** | | | HCl | 2HCl |
| | **< 30 %** | **30 %** | **55 %** | **49 %** |
| **Yield** | (inappropriate reaction) | (+ 35 % starting product) | | |

### REFERENCES

1. Kimata, A.; Nakagawa, H.; Ohyama, R.; Fukuuchi, T.; Ohta, S.; Doh-ura, K.; Suzuki, T.; Miyata, N. J. Med. Chem. 2007, 50, 5053-5056.
2. Miyata, N.; Suzuki, T.; Nakagawa, H.; Ohta, S.; Doh-ura, K.; Fukuuchi, T. Patent WO 2008 53764 *see* CheM. Abstr. 148: 517711x
3. Kees, K.L.; Fitzgerald, J.J.; Steiner, K.E.; Mattes, J.F.; Mihan, B.; Tosi, T.; Mondoro, D.; McCaleb, M.L. J. Med. Chem. 1996, 39, 3920-3928.
4. Himly, M.; Jahn-Schmid, B.; Pittertschatscher, K.; Bohle, B.; Grubmayr, K.; Ferreira, F.; Ebner, H.; Ebner, C. J. Allergy Clin. Immunol. 2003, 111, 882-888.
5. Kees, K.L.; Caggiano, T.J.; Steiner, K.E.; Fitzgerald, J.J.; Kates, M.J.; Christos, T.E.; Kulishoff, J.M.; Moore, R.D.; McCaleb, M.L. J. Med. Chem. 1995, 38, 617-628.
6. Otterness, I.G. Clin. Exp. Immunol. 1981, 46, 332-339.
7. Braibante, M.E.F.; Braibante, H.T.S.; de Carvalho Tavares, L.; Rohte, S.F.; Costa, C.C.; Morel, A.F.; Stüker, C.Z.; Burrow, R.A. Synthesis 2007, 2485-2490.
8. Ishimaru, T. Yakugaku Zasshi 1957, 77, 800-802. See Chem. Abstr. 1957: 17893.
9. Li, L.; Chen, X.; Cutler, S.T. Patent WO 2001 082930 *see* Chem. Abstr. 2001 135: 344479
10. Backer, H.J.; Meijer, W. Rec. Trav. Chim. 1926, 45, 428-432.
11. Ohta, T.; Fujisawa, H.; Nakai, Y.; Furukawa, I. Bull. Soc. Chim. Jpn. 2000, 73, 1861-1864.
12. Khalil, A.K.; Hassan, M.A.; Mohamed, M.M.; El-Sayed, A.M. Phosphorus, Sulfur, and Silicon Relat. Elem. 2005, 180, 479-496.
13. Arakawa, K.; Miyasaka, T.; Ochi, H. Chem. Pharm. Bull. 1974, 22, 207-213.
14. Rodriguez-Franco, M.I.; Dorronsoro, I.; Hernández-Higueras, A.I.; Antequera, G. Tetrahedron Lett. 2001, 42, 863-865.
15. Martins, M.A.P.; Cunico, W.; Brondani, S.; Peres, R.L.; Zimmermann, N.; Rosa, F.A.; Fiss, G.F.; Zanatta, N.; Bonacorso, H.G. Synthesis 2006, 1485-1493.
16. Pyne, S.G.; Spellmeyer, D.C.; Chen, S.; Fuchs, P. L. J. Am. Chem. Soc. 1982, 104, 5728-5740.
17. Maquestiau, A.; Van Haverbeke, Y.; Bruyere, A. Bull. Soc. Chim. Belg. 1973, 82, 747-754.
18. Claramunt, R.M.; Sanz, D.; Lopez, C.; Jimenez, J.A.; Jimeno, M.L.; Elguero, J.; Fruchier, A. Magn. Res. Chem. 1997, 35, 35-75.
19. Boehme, W.R. Org. Synth. Coll. Vol. IV 1963, 590-593.
20. Schultz, A.G.; Hagmann, W.K. J. Org. Chem. 1978, 43, 4231-4233.
21. Rossiter, S.; Woo, C.K.; Hartzoulakis, B.; Wishart, G.; Stanyer, L.; Labadie, J.W.; Selwood, D.L. J. Comb. Chem. 2004, 6, 385-390.
22. Chan, D.M.T.; Monaco, K.L.; Wang, R.-P.; Winters, M.P. Tetrahedron Lett. 1998, 39, 2933-2936.
23. Lam, P.Y.S.; Clark, C.G.; Saubern, S.; Adams, J.; Winters, M.P.; Chan, D.M.T.; Combs, A. Tetrahedron Lett. 1998, 39, 2941-2944.
24. Lam, P.Y.S.; Vincent, G.; Clark, C.G.; Deudon, S.; Jadhav, P.K. Tetrahedron Lett. 2001, 42, 3415-3418.
25. Collman, J.P.; Zhong, M.; Zhang, C.; Costanzo, S. J. Org. Chem. 2001, 66, 7892-7897.
26. Lan, J.B.; Chen, L.; Yu, X.Q.; You, J.S.; Xie, R.G. Chem. Commun. 2004, 188-189.
27. Strouse, J.J.; Jeselnik, M.; Tapaha, F.; Jonsson, C.B.; Parker, W.B.; Arterburn, J.B. Tetrahedron Lett. 2005, 46, 5699-5702.
28. Chan, D.M.T.; Lam, P.Y.S. Recent advances in copper-promoted C-heteroatom bond cross-coupling reactions with boronic acids and derivatives. In Boronic acids; Wiley-VCH Ed., 2005; pp. 205-240.
29. Khan, M.A.; Polya, J.B. J. Chem. Soc. (C) 1970, 85-91.
30. Kiyomori, J.F.; Marcoux, J.F.; Buchwald, S. L. Tetrahedron Lett. 1999, 40, 2657-2660.
31. Klapars, A.; Antilla, J. C.; Huang, X.; Buchwald, S. L. J. Am. Chem. Soc. 2001, 123, 7727-7729.
32. Kunz, K.; Scholz, U.; Ganzer, D. Synlett 2003, 2428-2439.
33. Ley, S.V.; Thomas, A.W. Angew. Chem., Int. Ed. 2003, 42, 5400-5449.
34. Antilla, J. C.; Baskin, J.M.; Barders, T.E.; Buchwald, S. L. J. Org. Chem. 2004, 69, 5578-5587.
35. Cristau, H.J.; Cellier, P.P.; Spindler, J.F.; Taillefer, M. Eur. J. Org. Chem. 2004, 695-709.
36. Cristau, H.J.; Cellier, P.P.; Spindler, J.F.; Taillefer, M. Chem. Eur. J. 2004, 10, 5607-5622.
37. Cristau, H.J.; Cellier, P.P.; Hamada, S.; Spindler, J.F.; Taillefer, M. Org. Lett. 2004, 6, 913-916.
38. Kuil, M.; Bedekam, E.K.; Visser, G.M.; van den Hoog-band, A.; Terpstra, J.W.; Kamer, P.C.J.; van Leewen, P.W.N.M.; van Strijdonck, G.P.F. Tetrahedron Lett. 2005, 46, 2405-2409.
39. Alcade, E.; Dinarès, I.; Rodriguez, S.; Garcia de Miguel, C. Eur. J. Org. Chem. 2005, 1637-1643.
40. Xu, L.; Zhu, D.; Wu, F.; Wang, R.; Wan, B. Tetrahedron 2005, 61, 6553-6560.
41. Correa, A.; Bolm, C. Adv. Synth. Catal. 2007, 349, 2673-2676.
42. Old, D.W.; Harris, M.C.; Buchwald, S.L. Org. Lett. 2000, 2, 1403-1406.
43. Nakagawa, H.; Ohyama, R.; Kimata, A.; Suzuki, T.; Miyata, N. Bioorg. Med. Chem. 2006, 16, 5939-5942.
44. Jurcak, J.G.; Barrague, M.; Gillespy, T.A.; Edwards, M.L.; Musick, K.Y.; Weintraub, P.M.; Du, Y.; Dharanipragada, R.M.; Parkar, A.A. Patent WO 2005 026175 *see* Chem. Abstr. 2005 142: 316835
45. Guillou, S.; Janin, Y.L. J. Heterocyclic. Chem. 2008, in print.
46. Chu, L.; Fisher, M.H.; Goulet, M.T.; Wyvratt, M.J. Tetrahedron Lett. 1997, 38, 3871-3874.
47. Drysdale, M.J.; Dymock, B.W.; Barril-Alonso, X.; Workman, P.; Pearl, L.H.; Prodromou, C.; MacDonald, E. Patent WO 03 55,860 *see* Chem. Abstr. 2003 139: 101122k
48. Beaulieu, P.L.; Gillard, J; Bykowski, D.; Brochu, C.; Dansereau, N.; Duceppe, J.S.; Haché, B.; Jakalian, A.; Lagacé, L.; LaPlante, S.; McKercher, G.; Moreau, E.; Perreault, S.; Stammers, T.; Thauvette, L.; Warrington, J.; Kukolj, G. Bioorg. Med. Chem. Lett. 2006, 16, 4987-4993.
49. Bernal, P.; Tamariz, J. Helv. Chim. Acta 2007, 90, 1449-1454.
50. Campeau, L.C.; Rousseaux, S.; Fagnou, K. J. Am. Chem. Soc. 2005, 127, 18020-18021.
51. Elguero, J.; Jacquier, R.; Tarrago, G. Bull. Soc. Chim. Fr. 1967, 3780-3794.
52. Buchi, J.; Ursprung, R.; Lauener, G. Helv. Chim. Acta 1949, 32, 984-992.
53. Adembri, G.; Ponticel, F.; Pedeschi, P. J. Heterocyclic. Chem. 1972, 9, 1219-1225.
54. Stolz, F. J. Prakt. Chem. 1897, 55, 145-171.
55. Mayer, K. Ber. Dtsch. Chem. Ges. 1903, 36, 717-718.
56. Michaelis, A. Justus Liebigs Ann. Chem. 1905, 338, 267-321.
57. Elguero, J.; Jacquier, R.; Tarrago, G. Bull. Soc. Chim. Fr. 1967, 3780-3794.
58. Harries, C.; Loth, G. Ber. Dtsch. Chem. Ges. 1896, 29, 513-520.
59. Bellamy, A.J.; Innes, D.I.; Hillson, P.J. J. Chem. Soc. Perkin. Trans. 2 1982, 1599-1604.
60. Vogelbacher, UJ.; Keil, M.; Klintz, R.; Wahl, J.; Wingert, H.; Konig, H.; Rack, M.; Gotz, R.; Teles, J.H. Patent WO 98027062 *see* Chem. Abstr. 1998 129: 81726
61. Lecher, H.Z.; Parker, R.P.; Conn, R.C. J. Am. Chem. Soc. 1944, 66, 1959-1963.
62. Ainsworth, D.P.; Suschitzky, H. J. Chem. Soc. (C) 1967, 1003-1006.
63. Yamamoto, Y.; Shirakawa, N.; Takano, S.; Kawai, Y. Patent US 5663365 *see* Chem. Abstr. 1997 127: 262675
64. Effenberger, F.; Hartmann, W. Angew. Chem. 1964, 76, 188.
65. Effenberger, F.; Hartmann, W. Chem. Ber. 1969, 102, 3260-3267.
66. Nakamura, N.; Kishida, Y.; Ishida, N. Chem. Pharm. Bull. 1971, 19, 1389-1394.
67. Venturello, C. J. Chem. Soc. Perkin. Trans. I 1978, 681-685.
68. Venturello, C.; D'Aloisio, R. Synthesis 1979, 283-287.
69. Almerico, A.M.; Boulton, A.J. J. Chem. Soc. Chem. Commun. 1985, 204-205.
70. Varvounis, G.; Fiamegos, Y.; Pilidis, G. In Adv. Heterocyclic Chem.; Katritzky, A. R. Ed.; Academic Press, 2001; Vol. 80; pp. 74.
71. Stanovik, E.; Svete, J. In Science of Synthesis; Neier, R. Ed.; Thieme, 2002; Vol. 12; pp. 15.
72. Knorr, L. Ber. Dtsch. Chem. Ges. 1895, 28, 706.
73. Stolz, F. Ber. Dtsch. Chem. Ges. 1895, 28, 635.
74. Wolff, L. Ber. Dtsch. Chem. Ges. 1904, 37, 2827.
75. Backer, H. J.; Meijer, W. Rec. Trav. Chim. 1926, 45, 428.
76. Maquestiau, A.; Van Haverbeke, Y.; Bruyere, A. Bull. Soc. Chim. Belg. 1973, 82, 747.
77. Arakawa, K.; Miyasaka, T.; Ochi, H. Chem. Pharm. Bull. 1974, 22, 207.
78. Puar, M. S.; Funke, P. T.; Cohen, A. I. J. Pharm. Sci . 1978, 67, 850.
79. Pilgram, K. J. Heterocyclic. Chem. 1980, 17, 1413.
80. Zefirov, N. S.; Kozhushkov, S. I.; Kuznetsova, T. S. Khim. Geterotsikl. Soedin. 1983, 19, 801.
81. Krohn, K.; Stenns, C. Arch. Pharm. 1989, 322, 351.
82. Claramunt, R. M.; Sanz, D.; Lopez, C.; Jimenez, J. A. ; Jimeno, M. L.; Elguero, J.; Fruchier, A. Magn. Res. Chem. 1997, 35, 35.
83. Batterjee, S. M. J. Text. Assoc. 2001, 62, 145.
84. Martins, M. A. P.; Pereira, C. M. P.; Zimmermann, N. E. K.; Moura, S.; Sinhorin, A. P.; Cunico, W.; Zanatta, N.; Bonacorso, H. G.; Flores, A. C. F. Synthesis 2003, 2353.
85. Bevk, D.; Svete, J.; Stanovnik, B. J. Heterocyclic. Chem. 2005, 42, 1413.

## Claims

1. A process for the preparation of alkoxypyrazoles of formula (I) and, when R₇ = H, of formula (I') 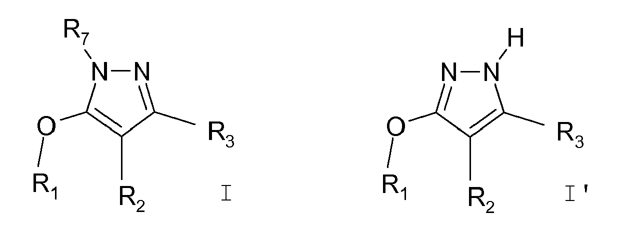 in which
R₁ stands for a radical selected from the group consisting of C₁-C₆ alkyl, C₂-C₆ alkenyl, C₂-C₆ alkynyl and (C₆-C₁₀)aryl(C₁-C₆)alkyl, each optionally substituted by R₄;
R₂ stands for a radical selected from the group consisting of hydrogen, halogen, C₁-C₆ alkyl, C₂-C₆ alkenyl, C₂-C₆ alkynyl, C₃-C₈ cycloalkyl, C₃-C₈ heterocycloalkyl, C₆-C₁₀ aryl, C₆-C₁₀ heteroaryl, C₆-C₁₀ aryloxy, (C₆-C₁₀) aryl (C₁-C₆) alkyl, (C₆-C₁₀) heteroaryl (C₁-C₆) alkyl, pyridyl, NR_{α}R_{β}, each optionally substituted by R₄;
R_{α} and R_{β}, identical or different, stand for a radical selected from the group consisting of H, C₁-C₆ alkyl or C₆-C₁₀ aryl;
R₃ stands for a radical selected from the group consisting of C₁-C₆ alkyl, C₂-C₆ alkenyl, C₂-C₆ alkynyl, C₁-C₆ polyhalogenoalkyl, C₃-C₈ cycloalkyl, C₃-C₈ heterocycloalkyl, C₆-C₁₀ aryl, C₆-C₁₀ heteroaryl, (C₆-C₁₀)aryl(C₁-C₆)alkyl, (C₆-C₁₀)heteroaryl(C₁-C₆)alkyl, -COOR₅, each optionally substituted by R₄;
or R₂ and R₃ form together an C₄-C₆ alkylene radical, an C₄-C₆ alkenylene, an C₄-C₆ heteroalkylene radical or an C₄-C₆ heteroalkenylene radical, each optionally substituted by R₆;
R₄ is a radical selected from the group consisting of halogen, hydroxyl, C₁-C₆ alkyl, C₁-C₆ alkylthio, C₁-C₆ alkoxy, and trialkylsilyl;
R₅ is a radical selected from the group consisting of hydrogen, C₁-C₆ alkyl, C₂-C₆ alkenyl and C₂-C₆ alkynyl;
R₆ is a radical selected from the group consisting of C₁-C₆ alkyl, C₂-C₆ alkenyl, C₂-C₆ alkynyl, C₆-C₁₀ aryl and (C₆-C₁₀ )aryl(C₁-C₆)alkyl;
R₇ stands for H, C₁-C₆ alkyl, C₆-C₁₀ aryl, (C₆-C₁₀) aryl (C₁-C₆) alkyl, C₆-C₁₀ heteroaryl or (C₆-C₁₀) heteroaryl (C₁-C₆)alkyl,each optionally substituted by a halogen;
comprising the successive following steps:
(a) reacting an alkylacetoacetate of formula (II) or one of its salts 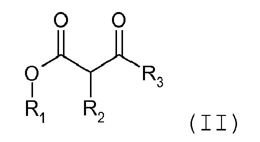 in which R₁, R₂ and R₃ are as defined for compound of formula (I)
with 1 to 1.5 molar equivalents of R₇-NH-NH₂, xHX, in which R₇ is as defined for compound of formula (I), x is 1 or 2 and X is Cl or Br;
in an alkylalcohol R₈OH as a solvent, in which R₈ is a radical selected from the group consisting of C₁-C₆ alkyl, C₂-C₆ alkenyl, C₂-C₆ alkynyl and (C₆-C₁₀)aryl(C₁-C₆)alkyl, (C₆-C₁₀)heteroaryl(C₁-C₆)alkyl;
(b) recovering the 3-alkoxypyrazole of formula (I).

2. The process according to claim 1, wherein the alkylacetoacetate of formula (II) is reacted with 1.05 molar equivalents of R₇-NH-NH₂, xHCl, in which R₇ and x are as defined in claim 1.

3. The process according to claims 1 or 2, wherein R₇ is a hydrogen, phenyl optionally substituted by a halogen, benzyl or a methyl group.

4. The process according to any one of the preceding claims, wherein x is 1.

5. The process according to any one of claims 1 to 3, wherein a salt of the alkylacetoacetate of formula (II) is reacted with R₇-NH-NH₂, 2HCl.

6. The process according to claim 5, wherein the salt of the alkylacetoacetate of formula (II) is a sodium salt.

7. The process according to any one of the preceding claims, wherein R₈ is R₁.

8. The process according to any one of the preceding claims, wherein the reaction in step (a) is run at the boiling point of the solvent.

9. The process according to any one of the preceding claims, wherein R₈ is an ethyl group.

10. The process according to any one of the preceding claims, wherein R₁ stands for a radical selected from the group consisting of C₁-C₆ alkyl optionally substituted by a C₁-C₆ alkoxy or trialkylsilyl group, and (C₆-C₁₀) aryl (C₁-C₆) alkyl.

11. The process according to any one of the preceding claims, wherein R₂ stands for a radical selected from the group consisting of hydrogen, F, Cl, C₁-C₆ alkyl, C₆-C₁₀ aryloxy, NRαRβ wherein Rα=Me and Rβ=Phenyl, and (C₆-C₁₀) aryl (C₁-C₆) alkyl.

12. The process according to any one of the preceding claims, wherein R₃ stands for a radical selected from the group consisting of C₁-C₆ alkyl, -CF₃, benzyl, C₆-C₁₀ aryl, -COOR₅, in which R₅ is a hydrogen or C₁-C₆ alkyl.

13. The process according to any one of the preceding claims, wherein R₂ and R₃ form together a C₄-C₆ alkylene radical or a C₄-C₆ heteroalkenylene radical, each eventually substituted by a benzyl group.

14. Alkoxypyrazoles of formula I or I' obtainable by the process according any one of the preceding claims, except compounds of formula (I') wherein simultaneously
R₂ is H, R₃ is methyl, R₁ is ethyl, methyl or benzyl;
R₁ is ethyl, R₂ is H, R₃ is phenyl, COOH or CF₃;
R₁ is ethyl, R₂ is ethyl, R₃ is methyl;
R₁ is ethyl, R₂ is phenyl, R₃ is phenyl;
R₁ is benzyl, R₂ is H, R₃ is methyl.

15. Alkoxypyrazoles of formula (III) or (IV) 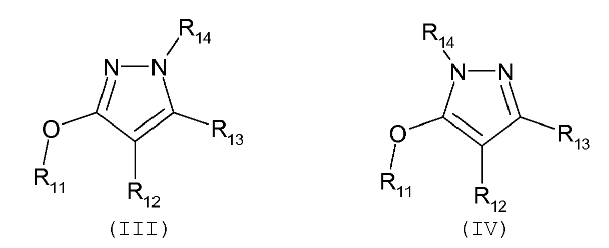 in which
R₁₁ stands for a radical selected from the group consisting of methyl, ethyl, 2-propyl, propene, benzyl, methoxyethyl, 2-(1-trimethylsilyl)ethyl,
R₁₂ stands for a radical selected from the group consisting of a hydrogen, halogen, a methyl, ethyl, benzyl, phenyl optionally substituted by Cl or methoxy, pyridyl, pyridyl N-oxide, phenoxy optionally substituted by one or more methyl, N,N-methylphenylamino, 2-phenethyl and is preferably an halogen and more preferably an iodine or a bromine atom,
R₁₃ stands for a radical selected from the group consisting of methyl, CF₃, phenyl, 4-chlorobenzyl, -COOR₅, in which R₅ is a radical selected from the group consisting of C₁-C₆ alkyl, C₂-C₆ alkenyl, and C₂-C₆ alkynyl and is preferably ethyl,
or R₁₂ and R₁₃ form together an C₄-C₆ alkylene radical, an C₄-C₆ alkenylene, an C₄-C₆ heteroalkylene radical or an C₄-C₆ heteroalkenylene radical, each optionally substituted by R₆ as defined above and is preferably selected among butylene, a -CH₂-NBn-CH₂-CH₂- group, a -CH₂-CH₂-NBn-CH₂- group,
R₁₄ stands for a radical selected from the group consisting of hydrogen, methyl, benzyl, mesyl, -CH₃SO₂, tosyl, phenyl optionally substituted by Cl or a methoxy group, pyridyl,
except compounds of formula (III) wherein R₁₄ is H and-simultaneously
R₁₂ is H, R₁₃ is methyl, R₁₁ is ethyl, methyl or benzyl;
R₁₁ is ethyl, R₁₂ is H, R₁₃ is phenyl, COOH or CF₃;
R₁₁ is ethyl, R₁₂ is ethyl, R₁₃ is methyl;
R₁₁ is ethyl, R₁₂ is phenyl, R₁₃ is phenyl;
R₁₁ is benzyl, R₁₂ is H, R₁₃ is methyl.

16. Alkoxypyrazoles according to claim 15, of formula (III) wherein R₁₄ is H or formula (IV) wherein R₁₄ stands for a radical selected from the group consisting of hydrogen, methyl, benzyl, phenyl optionally substituted by Cl,
and,
R₁₁ stands for a radical selected from the group consisting of methyl, ethyl, 2-propyl, propene, benzyl, 2-(1-trimethylsilyl)ethyl,
R₁₂ stands for a radical selected from the group consisting of a hydrogen, F, Cl, a methyl, ethyl, benzyl, R₁₃ stands for a radical selected from the group consisting of methyl, CF₃, phenyl, -COOR₅, in which R₅ is ethyl,
or R₁₂ and R₁₃ form together an C₄-C₆ alkylene radical, an C₄-C₆ heteroalkylene radical each optionally substituted by R₆ as defined in claim 1 and is preferably selected among butylene, a -CH₂-NBn-CH₂-CH₂- group, a -CH₂-CH₂-NBn-CH₂- group,
except compounds of formula (III) wherein R₁₄ is H and-simultaneously
R₁₂ is H, R₁₃ is methyl, R₁₁ is ethyl, methyl or benzyl;
R₁₁ is ethyl, R₁₂ is H, R₁₃ is phenyl, COOH or CF₃;
R₁₁ is ethyl, R₁₂ is ethyl, R₁₃ is methyl;
R₁₁ is ethyl, R₁₂ is phenyl, R₁₃ is phenyl;
R₁₁ is benzyl, R₁₂ is H, R₁₃ is methyl.

17. Alkoxypyrazoles according to claim 15, of formula (III) in which,
R₁₁ is ethyl, R₁₂ is benzyl, R₁₃ is methyl, R₁₄ is phenyl or 4-ClC₆H₄;
R₁₁ is 2-propyl, R₁₂ is H, R₁₃ is methyl, R₁₄ is benzyl, phenyl, 4-ClC₆H₄, 2-or 3- MeOC₆H₄;
R₁₁ is 2-propyl, R₁₂ is I, R₁₃ is methyl, R₁₄ is phenyl, 4-ClC₆H₄, 2- or-3- MeOC₆ H₄;
R₁₁ is 2-propyl, R₁₂ is Br, R₁₃ is methyl, R₁₄ is H, phenyl, 4-ClC₆H4. 2- or 3-MeOC₆ H₄;
R₁₁ is 2-propyl, R₁₃ is methyl, R₁₄ is methyl, and R₁₂ is phenyl, 4-ClC₆ H₄, or 2- or 3-MeOC₆ H₄
R₁₁ is 2-propyl, R₁₃ is methyl, R₁₄ is phenyl, R₁₂ is phenyl, 4-ClC₆ H₄, 2- or 3-MeOC₆H₄, 2-pyridyl or 2-pyridyl N-Oxide,
R₁₁ is 2-propyl, R₁₃ is methyl, R₁₄ is H, R₁₂ is H, I, C₆H₅, 4 ClC₆H₄, 2- or 3- MeOC₆H₄, 3- or 4-pyridyl, or CH₂-C₆H₅,
R₁₁ is 2-propyl, R₁₃ is methyl, R₁₄ is methyl, and R₁₂ is H, I, or 2-pyridyl N-Oxide,
R₁₁ is 2-propyl, R₁₂ is H, R₁₃ is methyl, R₁₄ is 2- 3-or-4-pyridyl;
R₁₁ is 2-propyl, R₁₃ is methyl, R₁₄ is phenyl, R₁₂ is CH₂C₆H₅ or 3-or 4-pyridyl,
R₁₁ is ethyl, R₁₃ is methyl, R₁₄ is H, R₁₂ is F, Cl, benzyl, C₆H₅, 3,5(CH₃)C₆H₅ O,
R₁₁ is ethyl, R₁₂ is H, R₁₃ is CF₃, R₁₄ is phenyl, 2-, 3- or 4-pyridyl,
R₁₁ is ethyl, R₁₂ is methyl or H, R₁₃ is -COOEt, R₁₄ is H;
R₁₁ is 1-propene, R₁₂ is H, R₁₃ is methyl, R₁₄ is H, 4-ClC₆ H₄,
R₁₁ is ethyl, R₁₂ is H, R₁₃ is phenyl, R₁₄ is 4-ClC₆ H₄;
R₁₁ is ethyl, R₁₂ is H, R₁₃ is CH₂-4-ClC₆ H₄, R₁₄ is H;
R₁₁ is methoxyethyl, R₁₂ is H, R₁₃ is CH₃, R₁₄ is H;
R₁₁ is ethyl, R₁₂ is benzyl, R₁₃ is methyl, R₁₄ is 4-MeOC₆H₄;
R₁₁ is 2- (1-trimethylsilyl) ethyl, R₁₂ is H, R₁₃ is methyl, R₁₄ is H;
R₁₁ is ethyl, R₁₂ is N, N-methylphenylamino, R₁₃ is methyl, R₁₄ is H;
R₁₁ is ethyl, R₁₂ is I, R₁₃ is -CF₃ or phenyl, R₁₄ is H;
R₁₁ is 2-propyl, R₁₂ is I, R₁₃ is methyl, R₁₄ is -CH₃SO₂;
R₁₁ is ethyl, R₁₂ is I, R₁₃ is -CF₃, R₁₄ is -CH₃SO₂;
R₁₁ is ethyl, R₁₂ is phenyl or benzyl, R₁₃ is -CF₃, R₁₄ is H;
R₁₁ is 2-propyl, R₁₂ is 2-phenethyl, R₁₃ is methyl, R₁₄ is H;
R₁₁ is ethyl, R₁₂ and R₁₃ form together a -CH₂-CH₂-NBn-CH₂-group, R₁₄ is H, 2-, 3- or 4-pyridyl or phenyl,
R₁₁ is ethyl, R₁₂ and R₁₃ form together a -CH₂-NBn-CH₂-CH₂-group, R₁₄ is 2-, 3 or 4-pyridyl, 4-ClC₆H₅ or H,
R₁₁ is ethyl; R₁₂ and R₁₃ form together a -CH₂-NH-CH₂-CH₂-group, R₁₄ is H, phenyl, 2-, 3- or 4- pyridyl.

18. Alkoxypyrazoles according to claim 15, of formula (IV) in which,
R₁₁ is 2-propyl, R₁₂ is H, R₁₃ is methyl, R₁₄ is methyl, benzyl, phenyl, 4-ClC₆H₄, 2- or 3-MeOC₆H₄, 2-, 3- or 4-pyridyl,
R₁₁ is 2-propyl, R₁₂ is I, R₁₃ is methyl, R₁₄ is methyl, phenyl, 4-ClC₆H₄, 3-MeOC₆H₄,
R₁₁ is 2-propyl, R₁₂ is Br, R₁₃ is methyl, R₁₄ is phenyl or 4-ClC₆H₄,
R₁₁ is 2-propyl, R₁₂ is phenyl, R₁₃ is methyl, R₁₄ is phenyl,
R₁₁ is ethyl, R₁₂ is H, R₁₃ is phenyl, R₁₄ is 4-ClC₆H₄,
R₁₁ is ethyl, R₁₂ and R₁₃ form together a -CH₂-NBn-CH₂-CH₂-group, R₁₄ is 4-ClC₆H₅,
R₁₁ is 1-propene, R₁₂ is H, R₁₃ is methyl, R₁₄ is 4-ClC₆H₄;
R₁₁ is ethyl, R₁₂ is benzyl, R₁₃ is methyl, R₁₄ is phenyl or 4-ClC₆H₄;
R₁₁ is 2-propyl, R₁₂ is I, R₁₃ is methyl, R₁₄ is -CH₃SO₂;
R₁₁ is ethyl, R₁₂ is I, R₁₃ is -CF₃, R₁₄ is -CH₃SO₂.
